# EUROPEAN PATENT APPLICATION

(11) **EP 2 813 492 A1**
(43) Date of publication of application: **17.12.2014**
(21) Application number: 13746367.5
(22) Date of filing: 05.02.2013
(51) Int. Cl.: C07D 307/14, A01N 43/08, A01N 43/16, A01P 7/04, C07D 309/04

(54) **AMIDE COMPOUND AND USE THEREOF FOR PEST CONTROL**

(30) Priority: 10.02.2012 JP 2012027001
(71) Applicant: Sumitomo Chemical Co., Ltd, Chuo-ku, Tokyo 104-8260 (JP)
(72) Inventor: OHSHITA, Jun, Takarazuka-shi Hyogo 665-8555 (JP); AWANO, Tomotsugu, Takarazuka-shi Hyogo 665-8555 (JP); IHARA, Hideki, Osaka-shi Osaka 555-0021 (JP)
(74) Representative: Nieuwenhuys, William Francis
(86) International application number: PCT/JP2013/053165
(87) International publication number: WO 2013/118905

(57) **Abstract**

An amide compound represented by formula (I) has an excellent pest control effect. (In the formula, Y represents a 3-7 membered saturated heterocyclic ring which contains, as ring-forming component(s), one or more atoms or groups that are selected from the group consisting of an oxygen atom and -S(O)ₜ-, the saturated heterocyclic ring may have one to three atoms or groups selected from group D and t represents 0 or the like; X represents a C₁-C₈ chain hydrocarbon group having one group that is selected from group A; W represents -CR⁸- or the like; r represents 1 or the like; R¹, R², R³, R⁴, R⁵, R⁶, R⁷ and R⁸ may be the same or different and each represents a hydrogen atom or the like; and n represents 1 or the like.)

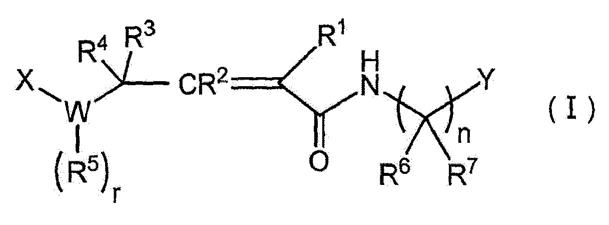

## Description

### TECHNICAL FIELD

The present invention relates to an amide compound and a use thereof for pest control.

### BACKGROUND ART

Conventionally, many pest control agents have been developed for controlling pests, and put to practical use. In addition, a certain type of amide compound is described in The Journal of Organic Chemistry, 1945, 10, pages 236 to 242.

### SUMMARY OF THE INVENTION

### PROBLEMS TO BE SOLVED BY THE INVENTION

The present invention provides a novel compound having an excellent control effect on pests.

More specifically, the present invention provides a compound represented by the following formula (I) having an excellent control effect on pests.

### MEANS FOR SOLVING THE PROBLEMS

The present invention is as described below.
[1] An amide compound represented by formula (I) 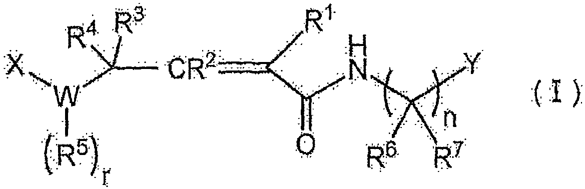 wherein
   Y represents a 3 to 7-membered saturated heterocyclic ring which contains, as ring-forming component(s), one or more atoms or groups that are selected from the group consisting of an oxygen atom and -S(O)ₜ-, the saturated heterocyclic ring may have one to three atoms or groups selected from group D,
   t represents 0, 1 or 2,
   X represents a C1 to C8 chain hydrocarbon group having one group that is selected from group A,
   W represents -CR⁸-, an oxygen atom or -S(O)ᵤ-,
   when W is -CR⁸-, r represents 1,
   when W is an oxygen atom or -S(O)ᵤ-, r represents 0, u represents 0, 1 or 2,
   R⁸ represents a C1 to C4 alkyl group optionally having one or more halogen atoms, a halogen atom or a hydrogen atom,
   R¹ and R² are the same or different and represent a C1 to C4 alkyl group optionally having one or more halogen atoms, a C1 to C4 alkoxy group optionally having one or more halogen atoms, a halogen atom or a hydrogen atom,
   R⁴ represents a C1 to C4 alkyl group optionally having one or more halogen atoms, a halogen atom or a hydrogen atom,
   R³ and R⁵ are the same or different and represent a C1 to C4 alkyl group optionally having one or more halogen atoms, a halogen atom or a hydrogen atom, or R³ and R⁵ are taken together to represent -(CR⁹R¹⁰)ᵥ-, -NR¹¹-, an oxygen atom or a sulfur atom,
   R⁹ and R¹⁰ are the same or different and represent a C1 to C4 alkyl group optionally having one or more halogen atoms, a halogen atom or a hydrogen atom,
   R¹¹ represents a C1 to C4 alkyl group optionally having one or more halogen atoms or a hydrogen atom,
   v represents 1, 2, 3 or 4,
   R⁶ and R⁷ are the same or different and represent a C1 to C4 alkyl group optionally having one or more halogen atoms or a hydrogen atom,
   n represents 0, 1 or 2,
      Group D: A group consisting of C1 to C4 alkyl groups optionally having one or more halogen atoms, C1 to C4 alkoxy groups optionally having one or more halogen atoms and halogen atoms. Group A: A group consisting of a phenyl group, a naphthyl group, a pyridyl group, a quinolyl group, a furyl group, a thienyl group, a benzofuranyl group and a benzothienyl group. Here, all the above-described groups in the group A may have one or more atoms or groups selected from group B.
      Group B: A group consisting of C1 to C4 alkyl groups optionally having one or more halogen atoms, C1 to C4 alkoxy groups optionally having one or more halogen atoms, C1 to C4 alkylthio groups optionally having one or more halogen atoms, C1 to C4 alkylsulfinyl groups optionally having one or more halogen atoms, C1 to C4 alkylsulfonyl groups optionally having one or more halogen atoms, C1 to C4 alkoxycarbonyl groups optionally having one or more halogen atoms, vinyl groups optionally having one or more atoms or groups selected from group E, ethynyl groups optionally having an atom or group selected from group E, a cyano group, a nitro group, a carboxyl group, a hydroxyl group, -CONR¹²R¹³ group and halogen atoms.
      R¹² and R¹³ are the same or different and represent a C1 to C4 alkyl group optionally having one or more halogen atoms or a hydrogen atom,
      Group E: A group consisting of C1 to C4 alkyl groups optionally having one or more halogen atoms and halogen atoms. (hereinafter, described as the compound of the present invention).
[2] The amide compound according to [1], wherein Y is represented by the following formula (II-a) or (II-b) 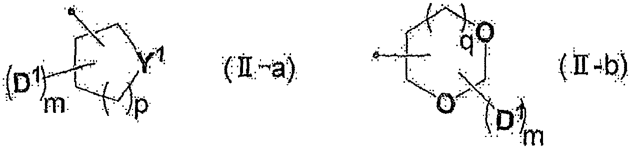 wherein Y¹ represents an oxygen atom or a sulfur atom, D¹ represents an atom or a group selected from group D, m represents 0 or 1, p represents 0, 1 or 2, and q is 0 or 1.
[3] The amide compound according to [2], wherein p is 1, or q is 0.
[4] The amide compound according to [2], wherein p is 2, or q is 1.
[5] The amide compound according to any of [2] to [4], wherein Y¹ is an oxygen atom.
[6] The amide compound according to [2], wherein Y is a group represented by the formula (II-a), and Y¹ is an oxygen atom.
[7] The amide compound according to [2], wherein Y is a group represented by the formula (II-b).
[8] The amide compound according to any of [1] to [7], wherein W is -CR⁸-, r is 1, R¹ is a halogen atom or a hydrogen atom, R² is a C1 to C4 alkyl group optionally having one or more halogen atoms or a hydrogen atom,
   R⁹ is a C1 to C4 alkyl group optionally having one or more halogen atoms, a halogen atom or a hydrogen atom,
   R³ and R⁵ are the same or different and represent a C1 to C4 alkyl group optionally having one or more halogen atoms, a halogen atom or a hydrogen atom, or R³ and R⁵ are taken together to be -(CR⁹R¹⁰)ᵥ-, v is 1, R⁹ and R¹⁰ are the same or different and are a C1 to C4 alkyl group optionally having one or more halogen atoms, a halogen atom or a hydrogen atom,
   R⁸ is a hydrogen atom, and
   R⁶ and R⁷ are the same or different and are a C1 to C4 alkyl group optionally having one or more halogen atoms or a hydrogen atom.
[9] The amide compound according to any of [1] to [8], wherein X is a C1 to C5 alkyl group having one group selected from group A.
[10] The amide compound according to any of [1] to [8], wherein X is a C1 to C5 alkyl group having one group selected from group A, one group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, and all the above-described groups in the group A may have one or more atoms or groups selected from group B.
[11] A pest control agent containing the amide compound as defined in any of [1] to [10] and an inert carrier.
[12] A method for controlling pests including the step of applying an effective amount of the amide compound as defined in any of [1] to [10] to a pest or a place where a pest inhabits.

### MODE FOR CARRYING OUT THE INVENTION

The compound of the present invention may include an isomer derived from an asymmetric carbon atom, and an isomer derived from a double bond, and the present invention includes each isomer having a pest control activity and an isomeric mixture at an arbitrary ratio.

In the compound of the present invention, "position 2 of the carbonyl group" means a position of the carbon atom represented by "2" in the following formula.

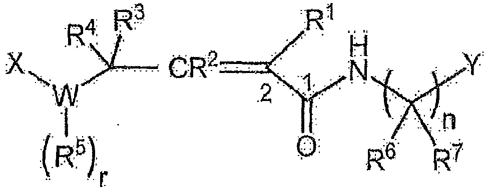

In the present invention, examples of the "a 3 to 7-membered saturated heterocyclic ring which contains, as ring-forming component (s), one or more atoms or groups that are selected from the group consisting of an oxygen atom and -S(O)ₜ-(herein, the saturated heterocyclic ring may have one to three atoms or groups selected from group D.)" include 3 to 7-membered saturated heterocyclic rings which contain, as ring-forming component(s), one or more oxygen atoms and optionally having one to three atoms or groups selected from group D, such as an oxetan-2-yl group, an oxetan-3-yl group, a tetrahydrofuran-2-yl group, a tetrahydrofuran-3-yl group, a 1,3-dioxolan-2-yl group, a 1,3-dioxolan-4-yl group, a tetrahydropyran-2-yl group, a tetrahydropyran-3-yl group, a tetrahydropyran-4-yl group, a 1,3-dioxan-4-yl group, a 1,3-dioxan-5-yl group, a 1, 4-dioxan-2-yl group, an oxepan-2-yl group, an oxepan-3-yl group, an oxepan-4-yl group, a 1,3-dioxepan-4-yl group, a 1,3-dioxepan-5-yl group, a 1,4-dioxepan-2-yl group, a 1,4-dioxepan-5-yl group and a 1,4-dioxepan-6-yl group; 3 to 7-membered saturated heterocyclic rings which contain, as ring-forming component (s), one or more sulfur atoms and optionally having one to three atoms or groups selected from group D, such as a thietan-2-yl group, a thietan-3-yl group, a tetrahydrothiophen-2-yl group, a tetrahydrothiophen-3-yl group, a 1,3-dithiolan-4-yl group, a tetrahydrothiopyran-2-yl group, a tetrahydrothiopyran-3-yl group, a tetrahydrothiopyran-4-yl group, a 1,3-dithian-4-yl group, a 1,3-dithian-5-yl group, a 1,4-dithian-2-yl group, a thiepan-2-yl group, a thiepan-3-yl group, a thiepan-4-yl group, a 1,3-dithiepan-4-yl group, a 1,3-dithiepan-5-yl group, a 1,4-dithiepan-2-yl group, a 1,4-dithiepan-5-yl group and a 1,4-dithiepan-6-yl group; 3 to 7-membered saturated heterocyclic rings which contain, as ring-forming component (s), one or more -SO- and optionally having one to theree atoms or groups selected from group D, such as an 1-oxo-thietan-2-yl group, an 1-oxo-thietan-3-yl group, an 1-oxo-tetrahydrothiophen-2-yl group, an 1-oxo-tetrahydrothiophen-3-yl group, an 1-oxo-tetrahydrothiopyran-2-yl group, an 1-oxo-tetrahydrothiopyran-3-yl group, an 1-oxo-tetrahydrothiopyran-4-yl group, an 1-oxo-thiepan-2-yl group, an 1-oxo-thiepan-3-yl group and an 1-oxo-thiepan-4-yl group; 3 to 7-membered saturated heterocyclic rings which contain, as ring-forming component(s), one or more -SO₂- and optionally having one to three atoms or groups that are selected from group D, such as a 1,1-dioxo-thietan-2-yl group, a 1,1-dioxo-thietan-3-yl group, a 1,1-dioxo-tetrahydrothiophen-2-yl group, a 1,1-dioxo-tetrahydrothiophen-3-yl group, a 1,1-dioxo-tetrahydrothiopyran-2-yl group, a 1,1-dioxo-tetrahydrothiopyran-3-yl group, a 1,1-dioxo-tetrahydrothiopyran-4-yl group, a 1,1-dioxo-thiepan-2-yl group, a 1,1-dioxo-thiepan-3-yl group, and a 1,1-dioxo-thiepan-4-yl group; and 3 to 7-membered saturated heterocyclic rings which contain, as ring-forming component(s), one or more oxygen atoms and one or more -S(O)ₜ-and optionally having one to three atoms or groups selected from group D, such as an 1,3-oxathietan-2-yl group, an 1,3-oxathiolan-2-yl group, an 1,3-oxathiolan-4-yl group, an 1,3-oxathiolan-5-yl group, an 1,3-oxathian-2-yl group, an 1,3-oxathiolan-4-yl group, a 1,3-oxathiolan-5-yl group, an 1,4-oxathian-2-yl group and an 1,4-oxathian-3-yl group.

Examples of the term "halogen atom" in the present invention include a fluorine atom, a chlorine atom, a bromine atom, and an iodine atom.

Examples of the phrase "a C1 to C4 alkyl group optionally having one or more halogen atoms" in the present invention include a methyl group, an ethyl group, a propyl group, an iso-isopropyl group, a butyl group, an iso-isobutyl group, a sec-butyl group, a tert-butyl group, a chloromethyl group, a difluoromethyl group, a trifluoromethyl group, a trichloromethyl group, a 2,2,2-trifluoroethyl group, a 1,1,2,2-tetrafluoroethyl group and a 1,1,2,2,2-pentafluoroethyl group.

Examples of the phrase "a C1 to C4 alkoxy groups optionally having one or more halogen atoms" in the present invention include a methoxy group, an ethoxy group, a propoxy group, an iso-isopropoxy group, a butoxy group, a difluoromethoxy group, a trifluoromethoxy group, a trichloromethoxy group, a 2,2,2-trifluoroethoxy group, a 1,1,2,2-tetrafluoroethoxy group and a 1,1,2,2,2-pentafluoroethoxy group.

Examples of the phrase "a C1 to C4 alkylthio group optionally having one or more halogen atoms" in the present invention include a methylthio group, an ethylthio group, a propylthio group, an iso-isopropylthio group, a butylthio group, a difluoromethylthio group, a trifluoromethylthio group, a trichloromethylthio group, a 2,2,2-trifluoroethylthio group, a 1,1,2,2-tetrafluoroethylthio group and a 1,1,2,2,2-pentafluoroethylthio group.

Examples of the phrase "a C1 to C4 alkylsulfinyl group optionally having one or more halogen atoms" in the present invention include a methylsulfinyl group, an ethylsulfinyl group, a propylsulfinyl group, an iso-isopropylsulfinyl group, a butylsulfinyl group, a difluoromethylsulfinyl group, a trifluoromethylsulfinyl group, a trichlorornethylsulfinyl group, a 2,2,2-trifluoroethylsulfinyl group, a 1,1,2,2-tetrafluoroethylsulfinyl group and a 1,1,2,2,2-pentafluoroethylsulfinyl group.

Examples of the phrase "a C1 to C4 alkylsulfonyl group optionally having one or more halogen atoms" in the present invention include a methylsulfonyl group, an ethylsulfonyl group, a propylsulfonyl group, an iso-isopropylsulfonyl group, a butylsulfonyl group, a difluoromethylsulfonyl group, a trifluoromethylsulfonyl group, a trichloromethylsulfonyl group, a 2,2,2-trifluoroethylsulfonyl group, a 1,1,2,2-tetrafluoroethylsulfonyl group and a 1,1,2,2,2-pentafluoroethylsulfonyl group.

Examples of the phrase "a C1 to C4 alkoxycarbonyl group optionally having one or more halogen atoms" in the present invention include a methoxycarbonyl group, an ethoxycarbonyl group, a propoxycarbonyl group, an isopropoxycarbonyl group, a butoxycarbonyl group, a tert-butoxycarbonyl group, a difluoromethoxycarbonyl group, a trifluoromethoxycarbonyl group, a trichloromethoxycarbonyl group, a 2,2,2-trifluoroethoxycarbonyl group, a 1,1,2,2-tetrafluoroethoxycarbonyl group and a 1,1,2,2,2-pentafluoroethoxycarbonyl group.

Examples of the phrase "a vinyl group optionally having one or more atoms or groups selected from group E" include a vinyl group, a 1-propenyl group, a 1-butenyl group, a 1-pentenyl group, a 1-hexenyl group, a 2-fluorovinyl group, a 2,2-difluorovinyl group, a 2,2-dichlorovinyl group, a 2-fluoro-1-propenyl group and a 2,3,3,3-tetrafluoro-1-propenyl group.

Examples of the phrase "an ethynyl group optionally having an atom or group selected from group E" include an ethynyl group, a 1-propynyl group, a 1-butynyl group, a 1-pentynyl group, a 1-hexynyl group, a 2-fluoroethynyl group, a 3,3-difluoro-1-propynyl group, a 3,3,3-trifluoro-1-propynyl group, a 4,4,4-trifluoro-1-butynyl group and a 3,3,4,4,4-pentafluoro-1-butynyl group.

Examples of the phrase "a group consisting of a phenyl group, a naphthyl group, a pyridyl group, a quinolyl group, a furyl group, a thienyl group, a benzofuranyl group and a benzothienyl group (herein, all the above-described groups in the group A may have one or more atoms or groups selected from group B)" include "phenyl groups optionally having one or more atoms or groups selected from group B" such as a phenyl group, a 2-cyanophenyl group, a 3-cyanophenyl group, a 4-cyanophenyl group, a 2-nitrophenyl group, a 3-nitrophenyl group, a 4-nitrophenyl group, a 2-carboxylphenyl group, a 3-carboxylphenyl group, a 4-carboxylphenyl group, a 2-hydroxylphenyl group, a 3-hydroxylphenyl group, a 4-hydroxylphenyl group, a 2-(N-methylcarboamide)phenyl group, a 3-(N-methylcarboamide)phenyl group, a 4-(N-methylcarboamide)phenyl group, a 2-(N,N-dimethylcarboamide)phenyl group, a 3-(N,N-dimethylcarboamide)phenyl group, a 4-(N,N-dimethylcarboamide)phenyl group, a 2-methylphenyl group, a 3-methylphenyl group, a 4-methylphenyl group, a 3,4-dimethylphenyl group, an 2-ethylphenyl group, an 3-ethylphenyl group, an 4-ethylphenyl group, a 2-trifluoromethylphenyl group, a 3-trifluoromethylphenyl group, a 4-trifluoromethylphenyl group, a 3,4-ditrifluoromethylphenyl group, a 2-methoxyphenyl group, a 3-methoxyphenyl group, a 4-methoxyphenyl group, a 2-trifluoromethoxyphenyl group, a 3-trifluoromethoxyphenyl group, a 4-trifluoromethoxyphenyl group, a 2-methylthiophenyl group, a 3-methylthiophenyl group, a 4-methylthiophenyl group, a 2-methylsulfinylphenyl group, a 3-methylsulfinylphenyl group, a 4-methylsulfinylphenyl group, a 2-methylsulfonylphenyl group, a 3-methylsulfonylphenyl group, a 4-methylsulfonylphenyl group, a 2-methoxycarbonylphenyl group, a 3-methoxycarbonylphenyl group, a 4-methoxycarbonylphenyl group, a 2-vinylphenyl group, a 3-vinylphenyl group, a 4-vinylphenyl group, a 2-(2',2'-difluorovinyl)phenyl group, a 3-(2',2'-difluorovinyl)phenyl group, a 4-(2',2'-difluorovinyl)phenyl group, an 2-ethynylphenyl group, an 3-ethynylphenyl group, an 4-ethynylphenyl group, a 2-(2'-fluoroethynyl)phenyl group, a 3-(2'-fluoroethynyl)phenyl group, a 4-(2'-fluoroethynyl)phenyl group, a 2-fluorophenyl group, a 3-fluorophenyl group, a 4-fluorophenyl group, a 3,4-difluorophenyl group, a pentafluorophenyl group, a 2-chlorophenyl group, a 3-chlorophenyl group, a 4-chlorophenyl group and a 3,4-dichlorophenyl group; "1-naphthyl groups optionally having one or more atoms or groups selected from group B" such as a 1-naphthyl group, a 2-cyano-1-naphthyl group, a 3-cyano-1-naphthyl group, a 4-cyano-1-naphthyl group, a 2-nitro-1-naphthyl group, a 3-nitro-1-naphthyl group, a 4-nitro-1-naphthyl group, a 2-carboxyl-1-naphthyl group, a 3-carboxyl-1-naphthyl group, a 4-carboxyl-1-naphthyl group, a 2-hydroxyl-1-naphthyl group, a 3-hydroxyl-1-naphthyl group, a 4-hydroxyl-1-naphthyl group, a 2-methyl-1-naphthyl group, a 3-methyl-1-naphthyl group, a 4-methyl-1-naphthyl group, a 2-trifluoromethyl-l-naphthyl group, a 3-trifluoromethyl-1-naphthyl group, a 4-trifluoromethyl-1-naphthyl group, a 2-methoxy-1-naphthyl group, a 3-methoxy-1-naphthyl group, a 4-methoxy-1-naphthyl group, a 2-trifluoromethoxy-l-naphthyl group, a 3-trifluoromethoxy-1-naphthyl group, a 4-trifluoromethoxy-1-naphthyl group, a 2-methoxycarbonyl-1-naphthyl group, a 3-methoxycarbonyl-1-naphthyl group, a 4-methoxycarbonyl-1-naphthyl group, a 2-vinyl-1-naphthyl group, a 3-vinyl-1-naphthyl group, a 4-vinyl-1-naphthyl group, a 2-(2',2'-difluorovinyl)-1-naphthyl group, a 3-(2',2'-difluorovinyl)-1-naphthyl group, a 4-(2',2'-difluorovinyl)-1-naphthyl group, an 2-ethynyl-1-naphthyl group, an 3-ethynyl-1-naphthyl group, an 4-ethynyl-1-naphthyl group, a 2-(2'-fluoroethynyl)-1-naphthyl group, a 3-(2'-fluoroethynyl)-1-naphthyl group, a 4-(2'-fluoroethynyl)-1-naphthyl group, a 2-fluoro-1-naphthyl group, a 3-fluoro-1-naphthyl group, a 4-fluoro-1-naphthyl group, a 2-chloro-1-naphthyl group, a 3-chloro-1-naphthyl group and a 4-chloro-1-naphthyl group; "2-naphthyl groups optionally having one or more atoms or groups selected from group B" such as a 2-naphthyl group, a 1-cyano-2-naphthyl group, a 3-cyano-2-naphthyl group, a 4-cyano-2-naphthyl group, a 1-nitro-2-naphthyl group, a 3-nitro-2-naphthyl group, a 4-nitro-2-naphthyl group, a 1-carboxyl-2-naphthyl group, a 3-carboxyl-2-naphthyl group, a 4-carboxyl-2-naphthyl group, a 1-hydroxyl-2-naphthyl group, a 3-hydroxyl-2-naphthyl group, a 4-hydroxyl-2-naphthyl group, a 1-methyl-2-naphthyl group, a 3-methyl-2-naphthyl group, a 4-methyl-2-naphthyl group, a 1-trifluoromethyl-2-naphthyl group, a 3-trifluoromethyl-2-naphthyl group, a 4-trifluoromethyl-2-naphthyl group, a 1-methoxy-2-naphthyl group, a 3-methoxy-2-naphthyl group, a 4-methoxy-2-naphthyl group, a 1-trifluoromethoxy-2-naphthyl group, a 3-trifluoromethoxy-2-naphthyl group, a 4-trifluoromethoxy-2-naphthyl group, a 1-methoxycarbonyl-2-naphthyl group, a 3-methoxycarbonyl-2-naphthyl group, a 4-methoxycarbonyl-2-naphthyl group, a 1-vinyl-2-naphthyl group, a 3-vinyl-2-naphthyl group, a 4-vinyl-2-naphthyl group, a 1-(2',2'-difluorovinyl)-2-naphthyl group, a 3-(2',2'-difluorovinyl)vinyl-2-naphthyl group, a 4-(2',2'-difluorovinyl)vinyl-2-naphthyl group, an 1-ethynyl-2-naphthyl group, an 3-ethynyl-2-naphthyl group, an 4-ethynyl-2-naphthyl group, a 1-(2'-fluoroethynyl)-2-naphthyl group, a 3-(2'-fluoroethynyl)-2-naphthyl group, a 4-(2'-fluoroethynyl)-2-naphthyl group, a 1-fluoro-2-naphthyl group, a 3-fluoro-2-naphthyl group, a 4-fluoro-2-naphthyl group, a 1-chloro-2-naphthyl group, a 3-chloro-2-naphthyl group and a 4-chloro-2-naphthyl group; "2-pyridyl groups optionally having one or more atoms or groups selected from group B" such as a 2-pyridyl group, a 3-cyano-2-pyridyl group, a 4-cyano-2-pyridyl group, a 5-cyano-2-pyridyl group, a 6-cyano-2-pyridyl group, a 3-nitro-2-pyridyl group, a 4-nitro-2-pyridyl group, a 5-nitro-2-pyridyl group, a 6-nitro-2-pyridyl group, a 3-carboxyl-2-pyridyl group, a 4-carboxyl-2-pyridyl group, a 5-carboxyl-2-pyridyl group, a 6-carboxyl-2-pyridyl group, a 3-hydroxyl-2-pyridyl group, a 4-hydroxyl-2-pyridyl group, a 5-hydroxyl-2-pyridyl group, a 6-hydroxyl-2-pyridyl group, a 3-methyl-2-pyridyl group, a 4-methyl-2-pyridyl group, a 5-methyl-2-pyridyl group, a 6-methyl-2-pyridyl group, a 3-trifluoromethyl-2-pyridyl group, a 4-trifluoromethyl-2-pyridyl group, a 5-trifluoromethyl-2-pyridyl group, a 6-trifluoromethyl-2-pyridyl group, a 3-methoxy-2-pyridyl group, a 4-methoxy-2-pyridyl group, a 5-methoxy-2-pyridyl group, a 6-methoxy-2-pyridyl group, a 3-trifluoromethoxy-2-pyridyl group, a 4-trifluoromethoxy-2-pyridyl group, a 5-trifluoromethoxy-2-pyridyl group, a 6-trifluoromethoxy-2-pyridyl group, a 3-methoxycarbonyl-2-pyridyl group, a 4-methoxycarbonyl-2-pyridyl group, a 5-methoxycarbonyl-2-pyridyl group, a 6-methoxycarbonyl-2-pyridyl group, a 3-vinyl-2-pyridyl group, a 4-vinyl-2-pyridyl group, a 5-vinyl-2-pyridyl group, a 6-vinyl-2-pyridyl group, a 3-(2',2'-difluorovinyl)-2-pyridyl group, a 4-(2',2'-difluorovinyl)vinyl-2-pyridyl group, a 5-(2',2'-difluorovinyl)vinyl-2-pyridyl group, a 6-(2',2'-difluorovinyl)vinyl-2-pyridyl group, a 3-ethynyl-2-pyridyl group, an 4-ethynyl-2-pyridyl group, an 5-ethynyl-2-pyridyl group, an 6-ethynyl-2-pyridyl group, a 3-(2'-fluoroethynyl)-2-pyridyl group, a 4-(2'-fluoroethynyl)-2-pyridyl group, a 5-(2'-fluoroethynyl)-2-pyridyl group, a 6-(2'-fluoroethynyl)-2-pyridyl group, a 3-fluoro-2-pyridyl group, a 4-fluoro-2-pyridyl group, a 5-fluoro-2-pyridyl group, a 6-fluoro-2-pyridyl group, a 3-chloro-2-pyridyl group, a 4-chloro-2-pyridyl group, a 5-chloro-2-pyridyl group and a 6-chloro-2-pyridyl group; "3-pyridyl groups optionally having one or more atoms or groups selected from group B" such as a 3-pyridyl group, a 2-cyano-3-pyridyl group, a 4-cyano-3-pyridyl group, a 5-cyano-3-pyridyl group, a 6-cyano-3-pyridyl group, a 2-nitro-3-pyridyl group, a 4-nitro-3-pyridyl group, a 5-nitro-3-pyridyl group, a 6-nitro-3-pyridyl group, a 2-carboxyl-3-pyridyl group, a 4-carboxyl-3-pyridyl group, a 5-carboxyl-3-pyridyl group, a 6-carboxyl-3-pyridyl group, a 2-hydroxyl-3-pyridyl group, a 4-hydroxyl-3-pyridyl group, a 5-hydroxyl-3-pyridyl group, a 6-hydroxyl-3-pyridyl group, a 2-methyl-3-pyridyl group, a 4-methyl-3-pyridyl group, a 5-methyl-3-pyridyl group, a 6-methyl-3-pyridyl group, a 2-trifluoromethyl-3-pyridyl group, a 4-trifluoromethyl-3-pyridyl group, a 5-trifluoromethyl-3-pyridyl group, a 6-trifluoromethyl-3-pyridyl group, a 2-methoxy-3-pyridyl group, a 4-methoxy-3-pyridyl group, a 5-methoxy-3-pyridyl group, a 6-methoxy-3-pyridyl group, a 2-trifluoromethoxy-3-pyridyl group, a 4-trifluoromethoxy-3-pyridyl group, a 5-trifluoromethoxy-3-pyridyl group, a 6-trifluoromethoxy-3-pyridyl group, a 2-methoxycarbonyl-3-pyridyl group, a 4-methoxycarbonyl-3-pyridyl group, a 5-methoxycarbonyl-3-pyridyl group, a 6-methoxycarbonyl-3-pyridyl group, a 2-vinyl-3-pyridyl group, a 4-vinyl-3-pyridyl group, a 5-vinyl-3-pyridyl group, a 6-vinyl-3-pyridyl group, a 2-(2',2'-difluorovinyl)-3-pyridyl group, a 4-(2',2'-difluorovinyl)vinyl-3-pyridyl group, a 5-(2',2'-difluorovinyl)vinyl-3-pyridyl group, a 6-(2',2'-difluorovinyl)vinyl-3-pyridyl group, an 2-ethynyl-3-pyridyl group, an 4-ethynyl-3-pyridyl group, an 5-ethynyl-3-pyridyl group, an 6-ethynyl-3-pyridyl group, a 2-(2'-fluoroethynyl)-3-pyridyl group, a 4-(2'-fluoroethynyl)-3-pyridyl group, a 5-(2'-fluoroethynyl)-3-pyridyl group, a 6-(2'-fluoroethynyl)-3-pyridyl group, a 2-fluoro-3-pyridyl group, a 4-fluoro-3-pyridyl group, a 5-fluoro-3-pyridyl group, a 6-fluoro-3-pyridyl group, a 2-chloro-3-pyridyl group, a 4-chloro-3-pyridyl group, a 5-chloro-3-pyridyl group and a 6-chloro-3-pyridyl group; "4-pyridyl groups optionally having one or more atoms or groups selected from group B" such as a 4-pyridyl group, a 2-cyano-4-pyridyl group, a 3-cyano-4-pyridyl group, a 5-cyano-4-pyridyl group, a 6-cyano-4-pyridyl group, a 2-nitro-4-pyridyl group, a 3-nitro-4-pyridyl group, a 5-nitro-4-pyridyl group, a 6-nitro-4-pyridyl group, a 2-carboxyl-4-pyridyl group, a 3-carboxyl-4-pyridyl group, a 5-carboxyl-4-pyridyl group, a 6-carboxyl-4-pyridyl group, a 2-hydroxyl-4-pyridyl group, a 3-hydroxyl-4-pyridyl group, a 5-hydroxyl-4-pyridyl group, a 6-hydroxyl-4-pyridyl group, a 2-methyl-4-pyridyl group, a 3-methyl-4-pyridyl group, a 5-methyl-4-pyridyl group, a 6-methyl-4-pyridyl group, a 2-trifluoromethyl-4-pyridyl group, a 3-trifluoromethyl-4-pyridyl group, a 5-trifluoromethyl-4-pyridyl group, a 6-trifluoromethyl-4-pyridyl group, a 2-methoxy-4-pyridyl group, a 3-methoxy-4-pyridyl group, a 5-methoxy-4-pyridyl group, a 6-methoxy-4-pyridyl group, a 2-trifluoromethoxy-4-pyridyl group, a 3-trifluoromethoxy-4-pyridyl group, a 5-trifluoromethoxy-4-pyridyl group, a 6-trifluoromethoxy-4-pyridyl group, a 2-methoxycarbonyl-4-pyridyl group, a 3-methoxycarbonyl-4-pyridyl group, a 5-methoxycarbonyl-4-pyridyl group, a 6-methoxycarbonyl-4-pyridyl group, a 2-vinyl-4-pyridyl group, a 3-vinyl-4-pyridyl group, a 5-vinyl-4-pyridyl group, a 6-vinyl-4-pyridyl group, a 2-(2',2'-difluorovinyl)-4-pyridyl group, a 3-(2',2'-difluorovinyl)vinyl-4-pyridyl group, a 5-(2',2'-difluorovinyl)vinyl-4-pyridyl group, a 6-(2',2'-difluorovinyl)vinyl-4-pyridyl group, an 2-ethynyl-4-pyridyl group, an 3-ethynyl-4-pyridyl group, an 5-ethynyl-4-pyridyl group, an 6-ethynyl-4-pyridyl group, a 2-(2'-fluoroethynyl)-4-pyridyl group, a 3-(2'-fluoroethynyl)-4-pyridyl group, a 5-(2'-fluoroethynyl)-4-pyridyl group, a 6-(2'-fluoroethynyl)-4-pyridyl group, a 2-fluoro-4-pyridyl group, a 3-fluoro-4-pyridyl group, a 5-fluoro-4-pyridyl group, a 6-fluoro-4-pyridyl group, a 2-chloro-4-pyridyl group, a 3-chloro-4-pyridyl group, a 5-chloro-4-pyridyl group and a 6-chloro-4-pyridyl group; "2-quinolyl groups optionally having one or more atoms or groups selected from group B" such as a 2-quinolyl group, a 5-cyano-2-quinolyl group, a 6-cyano-2-quinolyl group, a 7-cyano-2-quinolyl group, a 8-cyano-2-quinolyl group, a 5-nitro-2-quinolyl group, a 6-nitro-2-quinolyl group, a 7-nitro-2-quinolyl group, a 7-nitro-2-quinolyl group, a 5-carboxyl-2-quinolyl group, a 6-carboxyl-2-quinolyl group, a 7-carboxyl-2-quinolyl group, a 8-carboxyl-2-quinolyl group, a 5-hydroxyl-2-quinolyl group, a 6-hydroxyl-2-quinolyl group, a 7-hydroxyl-2-quinolyl group, a 8-hydroxyl-2-quinolyl group, a 5-methyl-2-quinolyl group, a 6-methyl-2-quinolyl group, a 7-methyl-2-quinolyl group, a 8-methyl-2-quinolyl group, a 5-trifluoromethyl-2-quinolyl group, a 6-trifluoromethyl-2-quinolyl group, a 7-trifluoromethyl-2-quinolyl group, a 8-trifluoromethyl-2-quinolyl group, a 5-methoxy-2-quinolyl group, a 6-methoxy-2-quinolyl group, a 7-methoxy-2-quinolyl group, a 8-methoxy-2-quinolyl group, a 5-trifluoromethoxy-2-quinolyl group, a 6-trifluoromethoxy-2-quinolyl group, a 7-trifluoromethoxy-2-quinolyl group, a 8-trifluoromethoxy-2-quinolyl group, a 5-methoxycarbonyl-2-quinolyl group, a 6-methoxycarbonyl-2-quinolyl group, a 7-methoxycarbonyl-2-quinolyl group, a 8-methoxycarbonyl-2-quinolyl group, a 5-vinyl-2-quinolyl group, a 6-vinyl-2-quinolyl group, a 7-vinyl-2-quinolyl group, a 8-vinyl-2-quinolyl group, a 5-(2',2'-difluorovinyl)-2-quinolyl group, a 6-(2',2'-difluorovinyl)vinyl-2-quinolyl group, a 7-(2',2'-difluorovinyl)-2-quinolyl group, a 8-(2',2'-difluorovinyl)vinyl-2-quinolyl group, an 5-ethynyl-2-quinolyl group, an 6-ethynyl-2-quinolyl group, an 7-ethynyl-2-quinolyl group, an 8-ethynyl-2-quinolyl group, a 5-(2'-fluoroethynyl)-2-quinolyl group, a 6-(2'-fluoroethynyl)-2-quinolyl group, a 7-(2'-fluoroethynyl)-2-quinolyl group, a 8-(2'-fluoroethynyl)-2-quinolyl group, a 5-fluoro-2-quinolyl group, a 6-fluoro-2-quinolyl group, a 7-fluoro-2-quinolyl group, a 8-fluoro-2-quinolyl group, a 5,6,7,8-tetrafluoro-2-quinolyl group, a 5-chloro-2-quinolyl group, a 6-chloro-2-quinolyl group, a 7-chloro-2-quinolyl group and a 8-chloro-2-quinolyl group; "3-quinolyl groups optionally having one or more atoms or groups selected from group B" such as a 3-quinolyl group, a 5-cyano-3-quinolyl group, a 6-cyano-3-quinolyl group, a 7-cyano-3-quinolyl group, a 8-cyano-3-quinolyl group, a 5-nitro-3-quinolyl group, a 6-nitro-3-quinolyl group, a 7-nitro-3-quinolyl group, a 7-nitro-3-quinolyl group, a 5-carboxyl-3-quinolyl group, a 6-carboxyl-3-quinolyl group, a 7-carboxyl-3-quinolyl group, a 8-carboxyl-3-quinolyl group, a 5-hydroxyl-3-quinolyl group, a 6-hydroxyl-3-quinolyl group, a 7-hydroxyl-3-quinolyl group, a 8-hydroxyl-3-quinolyl group, a 5-methyl-3-quinolyl group, a 6-methyl-3-quinolyl group, a 7-methyl-3-quinolyl group, a 8-methyl-3-quinolyl group, a 5-trifluoromethyl-3-quinolyl group, a 6-trifluoromethyl-3-quinolyl group, a 7-trifluoromethyl-3-quinolyl group, a 8-trifluoromethyl-3-quinolyl group, a 5-methoxy-3-quinolyl group, a 6-methoxy-3-quinolyl group, a 7-methoxy-3-quinolyl group, a 8-methoxy-3-quinolyl group, a 5-trifluoromethoxy-3-quinolyl group, a 6-trifluoromethoxy-3-quinolyl group, a 7-trifluoromethoxy-3-quinolyl group, a 8-trifluoromethoxy-3-quinolyl group, a 5-methoxycarbonyl-3-quinolyl group, a 6-methoxycarbonyl-3-quinolyl group, a 7-methoxycarbonyl-3-quinolyl group, a 8-methoxycarbonyl-3-quinolyl group, a 5-vinyl-3-quinolyl group, a 6-vinyl-3-quinolyl group, a 7-vinyl-3-quinolyl group, a 8-vinyl-3-quinolyl group, a 5-(2',2'-difluorovinyl)-3-quinolyl group, a 6-(2',2'-difluorovinyl)vinyl-3-quinolyl group, a 7-(2',2'-difluorovinyl)-3-quinolyl group, a 8-(2',2'-difluorovinyl)vinyl-3-quinolyl group, an 5-ethynyl-3-quinolyl group, an 6-ethynyl-3-quinolyl group, an 7-ethynyl-3-quinolyl group, an 8-ethynyl-3-quinolyl group, a 5-(2'-fluoroethynyl)-3-quinolyl group, a 6-(2'-fluoroethynyl)-3-quinolyl group, a 7-(2'-fluoroethynyl)-3-quinolyl group, a 8-(2'-fluoroethynyl)-3-quinolyl group, a 5-fluoro-3-quinolyl group, a 6-fluoro-3-quinolyl group, a 7-fluoro-3-quinolyl group, a 8-fluoro-3-quinolyl group, a 5,6,7,8-tetrafluoro-3-quinolyl group, a 5-chloro-3-quinolyl group, a 6-chloro-3-quinolyl group, a 7-chloro-3-quinolyl group and a 8-chloro-3-quinolyl group; "4-quinolyl groups optionally having one or more atoms or groups selected from group B" such as a 4-quinolyl group, a 2-cyano-4-quinolyl group, a 3-cyano-4-quinolyl group, a 2-nitro-4-quinolyl group, a 3-nitro-4-quinolyl group, a 2-carboxyl-4-quinolyl group, a 3-carboxyl-4-quinolyl group, a 2-hydroxyl-4-quinolyl group, a 3-hydroxyl-4-quinolyl group, a 2-methyl-4-quinolyl group, a 3-methyl-4-quinolyl group, a 2-trifluoromethyl-4-quinolyl group, a 3-trifluoromethyl-4-quinolyl group, a 2-methoxy-4-quinolyl group, a 3-methoxy-4-quinolyl group, a 2-trifluoromethoxy-4-quinolyl group, a 3-trifluoromethoxy-4-quinolyl group, a 2-methoxycarbonyl-4-quinolyl group, a 3-methoxycarbonyl-4-quinolyl group, a 2-vinyl-4-quinolyl group, a 3-vinyl-4-quinolyl group, a 2-(2',2'-difluorovinyl)-4-quinolyl group, a 3-(2',2'-difluorovinyl)vinyl-4-quinolyl group, an 2-ethynyl-4-quinolyl group, an 3-ethynyl-4-quinolyl group, a 2-(2'-fluoroethynyl)-4-quinolyl group, a 3-(2'-fluoroethynyl)-4-quinolyl group, a 2-fluoro-4-quinolyl group, a 3-fluoro-4-quinolyl group, a 2-chloro-4-quinolyl group and a 3-chloro-4-quinolyl group; '2-furyl groups optionally having one or more atoms or groups selected from group B" such as a 2-furyl group, a 3-cyano-2-furyl group, a 4-cyano-2-furyl group, a 5-cyano-2-furyl group, a 3-nitro-2-furyl group, a 4-nitro-2-furyl group, a 5-nitro-2-furyl group, a 3-carboxyl-2-furyl group, a 4-carboxyl-2-furyl group, a 5-carboxyl-2-furyl group, a 3-hydroxyl-2-furyl group, a 4-hydroxyl-2-furyl group, a 5-hydroxyl-2-furyl group, a 3-methyl-2-furyl group, a 4-methyl-2-furyl group, a 5-methyl-2-furyl group, a 3-trifluoromethyl-2-furyl group, a 4-trifluoromethyl-2-furyl group, a 5-trifluoromethyl-2-furyl group, a 3-methoxy-2-furyl group, a 4-methoxy-2-furyl group, a 5-methoxy-2-furyl group, a 3-trifluoromethoxy-2-furyl group, a 4-trifluoromethoxy-2-furyl group, a 5-trifluoromethoxy-2-furyl group, a 3-methoxycarbonyl-2-furyl group, a 4-methoxycarbonyl-2-furyl group, a 5-methoxycarbonyl-2-furyl group, a 3-vinyl-2-furyl group, a 4-vinyl-2-furyl group, a 5-vinyl-2-furyl group, a 3-(2',2'-difluorovinyl)-2-furyl group, a 4-(2',2'-difluorovinyl)vinyl-2-furyl group, a 5-(2',2'-difluorovinyl)vinyl-2-furyl group, an 3-ethynyl-2-furyl group, an 4-ethynyl-2-furyl group, an 5-ethynyl-2-furyl group, a 3-(2'-fluoroethynyl)-2-furyl group, a 4-(2'-fluoroethynyl)-2-furyl group, a 5-(2'-fluoroethynyl)-2-furyl group, a 3-fluoro-2-furyl group, a 4-fluoro-2-furyl group, a 5-fluoro-2-furyl group, a 3-chloro-2-furyl group, a 4-chloro-2-furyl group and a 5-chloro-2-furyl group; "3-furyl groups optionally having one or more atoms or groups selected from group B" such as a 3-furyl group, a 2-cyano-3-furyl group, a 4-cyano-3-furyl group, a 5-cyano-3-furyl group, a 2-nitro-3-furyl group, a 4-nitro-3-furyl group, a 5-nitro-3-furyl group, a 2-carboxyl-3-furyl group, a 4-carboxyl-3-furyl group, a 5-carboxyl-3-furyl group, a 2-hydroxyl-3-furyl group, a 4-hydroxyl-3-furyl group, a 5-hydroxyl-3-furyl group, a 2-methyl-3-furyl group, a 4-methyl-3-furyl group, a 5-methyl-3-furyl group, a 2-trifluoromethyl-3-furyl group, a 4-trifluoromethyl-3-furyl group, a 5-trifluoromethyl-3-furyl group, a 2-methoxy-3-furyl group, a 4-methoxy-3-furyl group, a 5-methoxy-3-furyl group, a 2-trifluoromethoxy-3-furyl group, a 4-trifluoromethoxy-3-furyl group, a 5-trifluoromethoxy-3-furyl group, a 2-methoxycarbonyl-3-furyl group, a 4-methoxycarbonyl-3-furyl group, a 5-methoxycarbonyl-3-furyl group, a 2-vinyl-3-furyl group, a 4-vinyl-3-furyl group, a 5-vinyl-3-furyl group, a 2-(2',2'-difluorovinyl)-3-furyl group, a 4-(2',2'-difluorovinyl)vinyl-3-furyl group, a 5-(2',2'-difluorovinyl)vinyl-3-furyl group, an 2-ethynyl-3-furyl group, an 4-ethynyl-3-furyl group, an 5-ethynyl-3-furyl group, a 2-(2'-fluoroethynyl)-3-furyl group, a 4-(2'-fluoroethynyl)-3-furyl group, a 5-(2'-fluoroethynyl)-3-furyl group, a 2-fluoro-3-furyl group, a 4-fluoro-3-furyl group, a 5-fluoro-3-furyl group, a 2-chloro-3-furyl group, a 4-chloro-3-furyl group and a 5-chloro-3-furyl group; "2-thienyl groups optionally having one or more atoms or groups selected from group B" such as a 2-thienyl group, a 3-cyano-2-thienyl group, a 4-cyano-2-thienyl group, a 5-cyano-2-thienyl group, a 3-nitro-2-thienyl group, a 4-nitro-2-thienyl group, a 5-nitro-2-thienyl group, a 3-carboxyl-2-thienyl group, a 4-carboxyl-2-thienyl group, a 5-carboxyl-2-thienyl group, a 3-hydroxyl-2-thienyl group, a 4-hydroxyl-2-thienyl group, a 5-hydroxyl-2-thienyl group, a 3-methyl-2-thienyl group, a 4-methyl-2-thienyl group, a 5-methyl-2-thienyl group, a 3-trifluoromethyl-2-thienyl group, a 4-trifluoromethyl-2-thienyl group, a 5-trifluoromethyl-2-thienyl group, a 3-methoxy-2-thienyl group, a 4-methoxy-2-thienyl group, a 5-methoxy-2-thienyl group, a 3-trifluoromethoxy-2-thienyl group, a 4-trifluoromethoxy-2-thienyl group, a 5-trifluoromethoxy-2-thienyl group, a 3-methoxycarbonyl-2-thienyl group, a 4-methoxycarbonyl-2-thienyl group, a 5-methoxycarbonyl-2-thienyl group, a 3-vinyl-2-thienyl group, a 4-vinyl-2-thienyl group, a 5-vinyl-2-thienyl group, a 3-(2',2'-difluorovinyl)-2-thienyl group, a 4-(2',2'-difluorovinyl)vinyl-2-thienyl group, a 5-(2',2'-difluorovinyl)vinyl-2-thienyl group, an 3-ethynyl-2-thienyl group, an 4-ethynyl-2-thienyl group, an 5-ethynyl-2-thienyl group, a 3-(2'-fluoroethynyl)-2-thienyl group, a 4-(2'-fluoroethynyl)-2-thienyl group, a 5-(2'-fluoroethynyl)-2-thienyl group, a 3-fluoro-2-thienyl group, a 4-fluoro-2-thienyl group, a 5-fluoro-2-thienyl group, a 3-chloro-2-thienyl group, a 4-chloro-2-thienyl group and a 5-chloro-2-thienyl group; "3-thienyl groups optionally having one or more atoms or groups selected from group B" such as a 3-thienyl group, a 2-cyano-3-thienyl group, a 4-cyano-3-thienyl group, a 5-cyano-3-thienyl group, a 2-nitro-3-thienyl group, a 4-nitro-3-thienyl group, a 5-nitro-3-thienyl group, a 2-carboxyl-3-thienyl group, a 4-carboxyl-3-thienyl group, a 5-carboxyl-3-thienyl group, a 2-hydroxyl-3-thienyl group, a 4-hydroxyl-3-thienyl group, a 5-hydroxyl-3-thienyl group, a 2-methyl-3-thienyl group, a 4-methyl-3-thienyl group, a 5-methyl-3-thienyl group, a 2-trifluoromethyl-3-thienyl group, a 4-trifluoromethyl-3-thienyl group, a 5-trifluoromethyl-3-thienyl group, a 2-methoxy-3-thienyl group, a 4-methoxy-3-thienyl group, a 5-methoxy-3-thienyl group, a 2-trifluoromethoxy-3-thienyl group, a 4-trifluoromethoxy-3-thienyl group, a 5-trifluoromethoxy-3-thienyl group, a 2-methoxycarbonyl-3-thienyl group, a 4-methoxycarbonyl-3-thienyl group, a 5-methoxycarbonyl-3-thienyl group, a 2-vinyl-3-thienyl group, a 4-vinyl-3-thienyl group, a 5-vinyl-3-thienyl group, a 2-(2',2'-difluorovinyl)-3-thienyl group, a 4-(2',2'-difluorovinyl)vinyl-3-thienyl group, a 5-(2',2'-difluorovinyl)vinyl-3-thienyl group, an 2-ethynyl-3-thienyl group, an 4-ethynyl-3-thienyl group, an 5-ethynyl-3-thienyl group, a 2-(2'-fluoroethynyl)-3-thienyl group, a 4-(2'-fluoroethynyl)-3-thienyl group, a 5-(2'-fluoroethynyl)-3-thienyl group, a 2-fluoro-3-thienyl group, a 4-fluoro-3-thienyl group, a 5-fluoro-3-thienyl group, a 2-chloro-3-thienyl group, a 4-chloro-3-thienyl group and a 5-chloro-3-thienyl group; "2-(1-benzofuranyl) groups optionally having one or more atoms or groups selected from group B" such as a 2-(1-benzofuranyl) group, a 4-cyano-2-(1-benzofuranyl) group, a 5-cyano-2-(1-benzofuranyl) group, a 6-cyano-2-(1-benzofuranyl) group, a 7-cyano-2-(1-benzofuranyl) group, a 4-nitro-2-(1-benzofuranyl) group, a 5-nitro-2-(1-benzofuranyl) group, a 6-nitro-2-(1-benzofuranyl) group, a 7-nitro-2-(1-benzofuranyl) group, a 4-carboxyl-2-(1-benzofuranyl) group, a 5-carboxyl-2-(1-benzofuranyl) group, a 6-carboxyl-2-(1-benzofuranyl) group, a 7-carboxyl-2-(1-benzofuranyl) group, a 4-hydroxyl-2-(1-benzofuranyl) group, a 5-hydroxyl-2-(1-benzofuranyl) group, a 6-hydroxyl-2-(1-benzofuranyl) group, a 7-hydroxyl-2-(1-benzofuranyl) group, a 4-methyl-2-(1-benzofuranyl) group, a 5-methyl-2-(1-benzofuranyl) group, a 6-methyl-2-(1-benzofuranyl) group, a 7-methyl-2-(1-benzofuranyl) group, a 4-trifluoromethyl-2-(1-benzofuranyl) group, a 5-trifluoromethyl-2-(1-benzofuranyl) group, a 6-trifluoromethyl-2-(1-benzofuranyl) group, a 7-trifluoromethyl-2-(1-benzofuranyl) group, a 4-methoxy-2-(1-benzofuranyl) group, a 5-methoxy-2-(l-benzofuranyl) group, a 6-methoxy-2-(1-benzofuranyl) group, a 7-methoxy-2-(1-benzofuranyl) group, a 4-trifluoromethoxy-2-(1-benzofuranyl) group, a 5-trifluoromethoxy-2-(1-benzofuranyl) group, a 6-trifluoromethoxy-2-(1-benzofuranyl) group, a 7-trifluoromethoxy-2-(1-benzofuranyl) group, a 4-methoxycarbonyl-2-(1-benzofuranyl) group, a 5-methoxycarbonyl-2-(1-benzofuranyl) group, a 6-methoxycarbonyl-2-(1-benzofuranyl) group, a 7-methoxycarbonyl-2-(1-benzofuranyl) group, a 4-vinyl-2-(1-benzofuranyl) group, a 5-vinyl-2-(1-benzofuranyl) group, a 6-vinyl-2-(1-benzofuranyl) group, a 7-vinyl-2-(1-benzofuranyl) group, a 4-(2',2'-difluorovinyl)-2-(1-benzofuranyl) group, a 5-(2',2'-difluorovinyl)vinyl-2-(1-benzofuranyl) group, a 6-(2',2'-difluorovinyl)-2-(1-benzofuranyl) group, a 7-(2',2'-difluorovinyl)vinyl-2-(1-benzofuranyl) group, an 4-ethynyl-2-(1-benzofuranyl) group, an 5-ethynyl-2-(1-benzofuranyl) group, an 6-ethynyl-2-(1-benzofuranyl) group, an 7-ethynyl-2-(1-benzofuranyl) group, a 4-(2'-fluoroethynyl)-2-(1-benzofuranyl) group, a 5-(2'-fluoroethynyl)-2-(1-benzofuranyl) group, a 6-(2'-fluoroethynyl)-2-(1-benzofuranyl) group, a 7-(2'-fluoroethynyl)-2-(1-benzofuranyl) group, a 4-fluoro-2-(1-benzofuranyl) group, a 5-fluoro-2-(1-benzofuranyl) group, a 6-fluoro-2-(1-benzofuranyl) group, a 7-fluoro-2-(1-benzofuranyl) group, a 4,5,6,7-tetrafluoro-2-(1-benzofuranyl) group, a 4-chloro-2-(1-benzofuranyl) group, a 5-chloro-2-(1-benzofuranyl) group, a 6-chloro-2-(1-benzofuranyl) group and a 7-chloro-2-(1-benzofuranyl) group; "3-(1-benzofuranyl) groups optionally having one or more atoms or groups selected from group B" such as a 3-(1-benzofuranyl) group, a 4-cyano-3-(1-benzofuranyl) group, a 5-cyano-3-(1-benzofuranyl) group, a 6-cyano-3-(1-benzofuranyl) group, a 7-cyano-3-(1-benzofuranyl) group, a 4-nitro-3-(1-benzofuranyl) group, a 5-nitro-3-(1-benzofuranyl) group, a 6-nitro-3-(1-benzofuranyl) group, a 7-nitro-3-(1-benzofuranyl) group, a 4-carboxyl-3-(1-benzofuranyl) group, a 5-carboxyl-3-(1-benzofuranyl) group, a 6-carboxyl-3-(1-benzofuranyl) group, a 7-carboxyl-3-(1-benzofuranyl) group, a 4-hydroxyl-3-(1-benzofuranyl) group, a 5-hydroxyl-3-(1-benzofuranyl) group, a 6-hydroxyl-3-(1-benzofuranyl) group, a 7-hydroxyl-3-(1-benzofuranyl) group, a 4-methyl-3-(1-benzofuranyl) group, a 5-methyl-3-(1-benzofuranyl) group, a 6-methyl-3-(1-benzofuranyl) group, a 7-methyl-3-(1-benzofuranyl) group, a 4-trifluoromethyl-3-(1-benzofuranyl) group, a 5-trifluoromethyl-3-(1-benzofuranyl) group, a 6-trifluoromethyl-3-(1-benzofuranyl) group, a 7-trifluoromethyl-3-(1-benzofuranyl) group, a 4-methoxy-3-(1-benzofuranyl) group, a 5-methoxy-3-(1-benzofuranyl) group, a 6-methoxy-3-(1-benzofuranyl) group, a 7-methoxy-3-(1-benzofuranyl) group, a 4-trifluoromethoxy-3-(1-benzofuranyl) group, a 5-trifluoromethoxy-3-(1-benzofuranyl) group, a 6-trifluoromethoxy-3-(1-benzofuranyl) group, a 7-trifluoromethoxy-3-(1-benzofuranyl) group, a 4-methoxycarbonyl-3-(1-benzofuranyl) group, a 5-methoxycarbonyl-3-(1-benzofuranyl) group, a 6-methoxycarbonyl-3-(1-benzofuranyl) group, a 7-methoxycarbonyl-3-(1-benzofuranyl) group, a 4-vinyl-3-(1-benzofuranyl) group, a 5-vinyl-3-(1-benzofuranyl) group, a 6-vinyl-3-(1-benzofuranyl) group, a 7-vinyl-3-(1-benzofuranyl) group, a 4-(2',2'-difluorovinyl)-3-(1-benzofuranyl) group, a 5-(2',2'-difluorovinyl)vinyl-3-(1-benzofuranyl) group, a 6-(2',2'-difluorovinyl)-3-(1-benzofuranyl) group, a 7-(2',2'-difluorovinyl)vinyl-3-(1-benzofuranyl) group, an 4-ethynyl-3-(1-benzofuranyl) group, an 5-ethynyl-3-(1-benzofuranyl) group, an 6-ethynyl-3-(1-benzofuranyl) group, an 7-ethynyl-3-(1-benzofuranyl) group, a 4-(2'-fluoroethynyl)-3-(1-benzofuranyl) group, a 5-(2'-fluoroethynyl)-3-(1-benzofuranyl) group, a 6-(2'-fluoroethynyl)-3-(1-benzofuranyl) group, a 7-(2'-fluoroethynyl)-3-(1-benzofuranyl) group, a 4-fluoro-3-(1-benzofuranyl) group, a 5-fluoro-3-(1-benzofuranyl) group, a 6-fluoro-3-(1-benzofuranyl) group, a 7-fluoro-3-(1-benzofuranyl) group, a 4,5,6,7-tetrafluoro-3-(1-benzofuranyl) group, a 4-chloro-3-(1-benzofuranyl) group, a 5-chloro-3-(1-benzofuranyl) group, a 6-chloro-3-(1-benzofuranyl) group and a 7-chloro-3-(1-benzofuranyl) group; "2-(1-benzothienyl) groups optionally having one or more atoms or groups selected from group B" such as a 2-(1-benzothienyl) group, a 4-cyano-2-(1-benzothienyl) group, a 5-cyano-2-(1-benzothienyl) group, a 6-cyano-2-(1-benzothienyl) group, a 7-cyano-2-(1-benzothienyl) group, a 4-nitro-2-(1-benzothienyl) group, a 5-nitro-2-(1-benzothienyl) group, a 6-nitro-2-(1-benzothienyl) group, a 7-nitro-2-(1-benzothienyl) group, a 4-carboxyl-2-(1-benzothienyl) group, a 5-carboxyl-2-(1-benzothienyl) group, a 6-carboxyl-2-(1-benzothienyl) group, a 7-carboxyl-2-(1-benzothienyl) group, a 4-hydroxyl-2-(1-benzothienyl) group, a 5-hydroxyl-2-(1-benzothienyl) group, a 6-hydroxyl-2-(1-benzothienyl) group, a 7-hydroxyl-2-(1-benzothienyl) group, a 4-methyl-2-(1-benzothienyl) group, a 5-methyl-2-(1-benzothienyl) group, a 6-methyl-2-(1-benzothienyl) group, a 7-methyl-2-(1-benzothienyl) group, a 4-trifluoromethyl-2-(1-benzothienyl) group, a 5-trifluoromethyl-2-(1-benzothienyl) group, a 6-trifluoromethyl-2-(1-benzothienyl) group, a 7-trifluoromethyl-2-(1-benzothienyl) group, a 4-methoxy-2-(1-benzothienyl) group, a 5-methoxy-2-(1-benzothienyl) group, a 6-methoxy-2-(1-benzothienyl) group, a 7-methoxy-2-(1-benzothienyl) group, a 4-trifluoromethoxy-2-(1-benzothienyl) group, a 5-trifluoromethoxy-2-(1-benzothienyl) group, a 6-trifluoromethoxy-2-(1-benzothienyl) group, a 7-trifluoromethoxy-2-(1-benzothienyl) group, a 4-methoxycarbonyl-2-(1-benzothienyl) group, a 5-methoxycarbonyl-2-(1-benzothienyl) group, a 6-methoxycarbonyl-2-(1-benzothienyl) group, a 7-methoxycarbonyl-2-(1-benzothienyl) group, a 4-vinyl-2-(1-benzothienyl) group, a 5-vinyl-2-(1-benzothienyl) group, a 6-vinyl-2-(1-benzothienyl) group, a 7-vinyl-2-(1-benzothienyl) group, a 4-(2',2'-difluorovinyl)-2-(1-benzothienyl) group, a 5-(2',2'-difluorovinyl)vinyl-2-(1-benzothienyl) group, a 6-(2',2'-difluorovinyl)-2-(1-benzothienyl) group, a 7-(2',2'-difluorovinyl)vinyl-2-(1-benzothienyl) group, an 4-ethynyl-2-(1-benzothienyl) group, an 5-ethynyl-2-(1-benzothienyl) group, an 6-ethynyl-2-(1-benzothienyl) group, an 7-ethynyl-2-(1-benzothienyl) group, a 4-(2'-fluoroethynyl)-2-(1-benzothienyl) group, a 5-(2'-fluoroethynyl)-2-(1-benzothienyl) group, a 6-(2'-fluoroethynyl)-2-(1-benzothienyl) group, a 7-(2'-fluoroethynyl)-2-(1-benzothienyl) group, a 4-fluoro-2-(1-benzothienyl) group, a 5-fluoro-2-(1-benzothienyl) group, a 6-fluoro-2-(1-benzothienyl) group, a 7-fluoro-2-(1-benzothienyl) group, a 4,5,6,7-tetrafluoro-2-(1-benzothienyl) group, a 4-chloro-2-(1-benzothienyl) group, a 5-chloro-2-(1-benzothienyl) group, a 6-chloro-2-(1-benzothienyl) group and a 7-chloro-2-(1-benzothienyl) group; "3-(1-benzothienyl) groups optionally having one or more atoms or groups selected from group B" such as a 3-(1-benzothienyl) group, a 4-cyano-3-(1-benzothienyl) group, a 5-cyano-3-(1-benzothienyl) group, a 6-cyano-3-(1-benzothienyl) group, a 7-cyano-3-(1-benzothienyl) group, a 4-nitro-3-(1-benzothienyl) group, a 5-nitro-3-(1-benzothienyl) group, a 6-nitro-3-(1-benzothienyl) group, a 7-nitro-3-(1-benzothienyl) group, a 4-carboxyl-3-(1-benzothienyl) group, a 5-carboxyl-3-(1-benzothienyl) group, a 6-carboxyl-3-(1-benzothienyl) group, a 7-carboxyl-3-(1-benzothienyl) group, a 4-hydroxyl-3-(1-benzothienyl) group, a 5-hydroxyl-3-(1-benzothienyl) group, a 6-hydroxyl-3-(1-benzothienyl) group, a 7-hydroxyl-3-(1-benzothienyl) group, a 4-methyl-3-(1-benzothienyl) group, a 5-methyl-3-(1-benzothienyl) group, a 6-methyl-3-(1-benzothienyl) group, a 7-methyl-3-(1-benzothienyl) group, a 4-trifluoromethyl-3-(1-benzothienyl) group, a 5-trifluoromethyl-3-(1-benzothienyl) group, a 6-trifluoromethyl-3-(1-benzothienyl) group, a 7-trifluoromethyl-3-(1-benzothienyl) group, a 4-methoxy-3-(1-benzothienyl) group, a 5-methoxy-3-(1-benzothienyl) group, a 6-methoxy-3-(1-benzothienyl) group, a 7-methoxy-3-(1-benzothienyl) group, a 4-trifluoromethoxy-3-(1-benzothienyl) group, a 5-trifluoromethoxy-3-(1-benzothienyl) group, a 6-trifluoromethoxy-3-(1-benzothienyl) group, a 7-trifluoromethoxy-3-(1-benzothienyl) group, a 4-methoxycarbonyl-3-(1-benzothienyl) group, a 5-methoxycarbonyl-3-(1-benzothienyl) group, a 6-methoxycarbonyl-3-(1-benzothienyl) group, a 7-methoxycarbonyl-3-(1-benzothienyl) group, a 4-vinyl-3-(1-benzothienyl) group, a 5-vinyl-3-(1-benzothienyl) group, a 6-vinyl-3-(1-benzothienyl) group, a 7-vinyl-3-(1-benzothienyl) group, a 4-(2',2'-difluorovinyl)-3-(1-benzothienyl) group, a 5-(2',2'-difluorovinyl)vinyl-3-(1-benzothienyl) group, a 6-(2',2'-difluorovinyl)-3-(1-benzothienyl) group, a 7-(2',2'-difluorovinyl)vinyl-3-(1-benzothienyl) group, an 4-ethynyl-3-(1-benzothienyl) group, an 5-ethynyl-3-(1-benzothienyl) group, an 6-ethynyl-3-(1-benzothienyl) group, an 7-ethynyl-3-(1-benzothienyl) group, a 4-(2'-fluoroethynyl)-3-(1-benzothienyl) group, a 5-(2'-fluoroethynyl)-3-(1-benzothienyl) group, a 6-(2'-fluoroethynyl)-3-(1-benzothienyl) group, a 7-(2'-fluoroethynyl)-3-(1-benzothienyl) group, a 4-fluoro-3-(1-benzothienyl) group, a 5-fluoro-3-(1-benzothienyl) group, a 6-fluoro-3-(1-benzothienyl) group, a 7-fluoro-3-(1-benzothienyl) group, a 4,5,6,7-tetrafluoro-3-(1-benzothienyl) group, a 4-chloro-3-(1-benzothienyl) group, a 5-chloro-3-(1-benzothienyl) group, a 6-chloro-3-(1-benzothienyl) group and a 7-chloro-3-(1-benzothienyl) group.

Examples of the "C1 to C8 chain hydrocarbon group" in the "C1 to C8 chain hydrocarbon group having one group that is selected from group A" represented by X include "C1 to C8 alkyl groups" such as a methyl group, an ethyl group, an iso-isopropyl group, a tert-butyl group, a propyl group, a sec-butyl group, an iso-isobutyl group, a 1,1-dimethylpropyl group, a 2,2-dimethylpropyl group, a 1,2-dimethylpropyl group, an 1-ethylpropyl group, a butyl group, a 1-methylbutyl group, a 2-methylbutyl group, a 3-methylbutyl group, a 1,1-dimethylbutyl group, a 2,2-dimethylbutyl group, a 3,3-dimethylbutyl group, a 1,2,3-trimethylbutyl group, an 1-ethylbutyl group, an 2-ethylbutyl group, a pentyl group, a 1-methylpentyl group, a 2-methylpentyl group, a 3-methylpentyl group, a 4-methylpentyl group, a 1,1-dimethylpentyl group, a 2,2-dimethylpentyl group, a 3,3-dimethylpentyl group, a 4,4-dimethylpentyl group, an 1-ethylpentyl group, an 2-ethylpentyl group, an 3-ethylpentyl group, a 1-propylpentyl group, a 2-propylpentyl group, a hexyl group, a 1-methylhexyl group, a 2-methylhexyl group, a 3-methylhexyl group, a 4-methylhexyl group, a 5-methylhexyl group, a 1,1-dimethylhexyl group, a 2,2-dimethylhexyl group, a 3,3-dimethylhexyl group, a 4,4-dimethylhexyl group, a 5,5-dimethylhexyl group, an 1-ethylhexyl group, an 2-ethylhexyl group, an 3-ethylhexyl group, an 4-ethylhexyl group, a heptyl group, a 1-methylheptyl group, a 2-methylheptyl group, a 3-methylheptyl group, a 4-methylheptyl group, a 5-methylheptyl group, a 6-methylheptyl group and an octyl group; "C12 to C8 alkenyl groups" such as a vinyl group, a 1-propenyl group, a 2-propenyl group, a 1-methyl-l-propenyl group, a 1-methyl-2-propenyl group, a 1,2-dimethyl-1-propenyl group, a 1,1-dimethyl-2-propenyl group, an 1-ethyl-1-propenyl group, an 1-ethyl-2-propenyl group, a 1-butenyl group, a 2-butenyl group, a 3-butenyl group, a 1-methyl-1-butenyl group, a 2-methyl-1-butenyl group, a 2-methyl-2-butenyl group, a 2-methyl-3-butenyl group, a 3-methyl-3-butenyl group, a 1,1-dimethyl-3-butenyl group, a 1,1-dimethyl-2-butenyl group, an 1-ethyl-1-butenyl group, an 1-ethyl-2-butenyl group, an 1-ethyl-3-butenyl group, a 1-pentenyl group, a 2-pentenyl group, a 3-pentenyl group, a 4-pentenyl group, a 1-methyl-1-pentenyl group, a 1-methyl-2-pentenyl group, a 1-methyl-3-pentenyl group, a 1-methyl-4-pentenyl group, a 2-methyl-1-pentenyl group, a 2-methyl-2-pentenyl group, a 3-methyl-2-pentenyl group, a 3-methyl-3-pentenyl group, a 4-methyl-3-pentenyl group, a 4-methyl-4-pentenyl group, a 1,1-dimethyl-2-pentenyl group, a 1,1-dimethyl-3-pentenyl group, a 1,1-dimethyl-4-pentenyl group, an 1-ethyl-1-pentenyl group, an 1-ethyl-2-pentenyl group, an 1-ethyl-3-pentenyl group, an 1-ethyl-4-pentenyl group, a 1-hexenyl group, a 2-hexenyl group, a 3-hexenyl group, a 4-hexenyl group, a 5-hexenyl group, a 1-methyl-1-hexenyl group, a 1-methyl-2-hexenyl group, a 1-methyl-3-hexenyl group, a 1-methyl-4-hexenyl group, a 1-methyl-5-hexenyl group, a 2-methyl-1-hexenyl group, a 2-methyl-2-hexenyl group, a 3-methyl-2-hexenyl group, a 3-methyl-3-hexenyl group, a 4-methyl-3-hexenyl group, a 4-methyl-4-hexenyl group, a 5-methyl-4-hexenyl group, a 5-methyl-5-hexenyl group, a 1,1-dimethyl-2-hexenyl group, a 1,1-dimethyl-3-hexenyl group, a 1,1-dimethyl-4-hexenyl group, a 1,1-dimethyl-5-hexenyl group, an 1-ethyl-1-hexenyl group, an 1-ethyl-2-hexenyl group, an 1-ethyl-3-hexenyl group, an 1-ethyl-4-hexenyl group, an 1-ethyl-5-hexenyl group, a 1-heptenyl group, a 2-heptenyl group, a 3-heptenyl group, a 4-heptenyl group, a 5-heptenyl group, a 6-heptenyl group, a 1-methyl-1heptenyl group, a 1-methyl-2-heptenyl group, a 1-methyl-3-heptenyl group, a 1-methyl-4-heptenyl group, a 1-methyl-5-heptenyl group, a 1-methyl-6-heptenyl group, a 2-methyl-1-heptenyl group, a 2-methyl-2-heptenyl group, a 3-methyl-2-heptenyl group, a 3-methyl-3-heptenyl group, a 4-methyl-3-heptenyl group, a 4-methyl-4-heptenyl group, a 5-methyl-4-heptenyl group, a 5-methyl-5-heptenyl group, a 6-methyl-5-heptenyl group, a 6-methyl-6-heptenyl group, an 1-octenyl group, an 2-octenyl group, an 3-octenyl group, an 4-octenyl group, an 5-octenyl group, an 6-octenyl group and an 7-octenyl group; and "C21 to C8 alkynyl groups" such as an ethynyl group, a 1-propynyl group, a 2-propynyl group, a 1-methyl-2-propynyl group, a 1,1-dimethyl-2-propynyl group, an 1-ethyl-2-propynyl group, a 1-butynyl group, a 2-butynyl group, a 3-butynyl group, a 1-methyl-2-butynyl group, a 1-methyl-3-butynyl group, a 1,1-dimethyl-2-butynyl group, a 1,1-dimethyl-3-butynyl group, an 1-ethyl-2-butynyl group, an 1-ethyl-3-butynyl group, a 1-pentynyl group, a 2-pentynyl group, a 3-pentynyl group, a 4-pentynyl group, a 1-methyl-2-pentynyl group, a 1-methyl-3-pentynyl group, a 1-methyl-4-pentynyl group, a 1,1-dimethyl-2-pentynyl group, a 1,1-dimethyl-3-pentynyl group, a 1,1-dimethyl-4-pentynyl group, an 1-ethyl-2-pentynyl group, an 1-ethyl-3-pentynyl group, an 1-ethyl-4-pentynyl group, a 1-hexynyl group, a 2-hexynyl group, a 3-hexynyl group, a 4-hexynyl group, a 5-hexynyl group, a 1-methyl-2-hexynyl group, a 1-methyl-3-hexynyl group, a 1-methyl-4-hexynyl group, a 1-methyl-5-hexynyl group, a 1,1-dimethyl-2-hexynyl group, a 1,1-dimethyl-3-hexynyl group, a 1,1-dimethyl-4-hexynyl group, a 1,1-dimethyl-5-hexynyl group, an 1-ethyl-2-hexynyl group, an 1-ethyl-3-hexynyl group, an l-ethyl-4-hexynyl group, an 1-ethyl-5-hexynyl group, a 1-heptynyl group, a 2-heptynyl group, a 3-heptynyl group, a 4-heptynyl group, a 5-heptynyl group, a 6-heptynyl group, a 1-methyl-2-heptynyl group, a 1-methyl-3-heptynyl group, a 1-methyl-4-heptynyl group, a 1-methyl-5-heptynyl group, a 1-methyl-6-heptynyl group, an 1-octynyl group, an 2-octynyl group, an 3-octynyl group, an 4-octynyl group, an 5-octynyl group, an 6-octynyl group and an 7-octynyl group.

In the present invention, examples of "C1 to C8 hydrocarbon group having one group that is selected from group A" represented by X include a benzyl group, a 1-phenylethyl group, a 2-phenylethyl group, a 3-phenylpropyl group, a 4-phenylbutyl group, a 5-phenylpentyl group, a 6-phenylhexyl group, a 7-phenylheptyl group, a 8-phenyloctyl group, a 4-cyanobenzyl group, a 4-nitrobenzyl group, a 4-carboxylbenzyl group, a 4-hydroxylbenzyl group, a 4-(N-methylcarboamide)benzyl group, a 4-(N,N-dimethylcarboamide)benzyl group, a 4-methylbenzyl group, a 4-trifluoromethylbenzyl group, a 4-methoxybenzyl group, a 4-trifluoromethoxybenzyl group, a 4-methylthiobenzyl group, a 4-methylsulfinylbenzyl group, a 4-methylsulfonylbenzyl group, a 4-methoxycarbonylbenzyl group, a 4-vinylbenzyl group, a 4-(2',2'-difluorovinyl)benzyl group, an 4-ethynylbenzyl group, a 4-(2'-fluoroethynyl)benzyl group, a 4-fluorobenzyl group, a 4-chlorobenzyl group, a 3,4-dichlorobenzyl group, a 1-naphthylmethyl group, a 1-(1'-naphthyl)ethyl group, a 2-(1'-naphthyl)ethyl group, a 3-(1'-naphthyl)propyl group, a 4-(1'-naphthyl)butyl group, a 5-(1'-naphthyl)pentyl group, a 6-(1'-naphthyl)hexyl group, a 7-(1'-naphthyl)heptyl group, a 8-(1'-naphthyl)octyl group, a 6-cyano-1-naphthylmethyl group, a 6-nitro-1-naphthylmethyl group, a 6-carboxyl-1-naphthylmethyl group, a 6-hydroxyl-1-naphthylmethyl group, a 6-(N-methylcarboamide)-1-naphthylmethyl group, a 6-(N,N-dimethylcarboamide)-1-naphthylmethyl group, a 6-methyl-1-naphthylmethyl group, a 6-trifluoromethyl-1-naphthylmethyl group, a 6-methoxy-1-naphthylmethyl group, a 6-trifluoromethoxy-1-naphthylmethyl group, a 6-methylthio-1-naphthylmethyl group, a 6-methylsulfinyl-1-naphthylmethyl group, a 6-methylsulfonyl-1-naphthylmethyl group, a 6-methoxycarbonyl-1-naphthylmethyl group, a 6-vinyl-1-naphthylmethyl group, a 6-(2',2'-difluorovinyl)-1-naphthylmethyl group, an 6-ethynyl-1-naphthylmethyl group, a 6-(2'-fluoroethynyl)-1-naphthylmethyl group, a 6-fluoro-1-naphthylmethyl group, a 6-chloro-1-naphthylmethyl group, a 2-naphthylmethyl group, a 1-(2'-naphthyl)ethyl group, a 2-(2'-naphthyl)ethyl group, a 3-(2'-naphthyl)propyl group, a 4-(2'-naphthyl)butyl group, a 5-(2'-naphthyl)pentyl group, a 6-(2'-naphthyl)hexyl group, a 7-(2'-naphthyl)heptyl group, a 8-(2'-naphthyl)octyl group, a 6-cyano-2-naphthylmethyl group, a 6-nitro-2-naphthylmethyl group, a 6-carboxyl-2-naphthylmethyl group, a 6-hydroxyl-2-naphthylmethyl group, a 6-(N-methylcarboamide)-2-naphthylmethyl group, a 6-(N,N-dimethylcarboamide)-2-naphthylmethyl group, a 6-methyl-2-naphthylmethyl group, a 6-trifluoromethyl-2-naphthylmethyl group, a 6-methoxy-2-naphthylmethyl group, a 6-trifluoromethoxy-2-naphthylmethyl group, a 6-methylthio-2-naphthylmethyl group, a 6-methylsulfinyl-2-naphthylmethyl group, a 6-methylsulfonyl-2-naphthylmethyl group, a 6-methoxycarbonyl-2-naphthylmethyl group, a 6-vinyl-2-naphthylmethyl group, a 6-(2',2'-difluorovinyl)-2-naphthylmethyl group, an 6-ethynyl-2-naphthylmethyl group, a 6-(2'-fluoroethynyl)-2-naphthylmethyl group, a 6-fluoro-2-naphthylmethyl group, a 6-chloro-2-naphthylmethyl group, a 2-pyridylmethyl group, a 1-(2'-pyridyl)ethyl group, a 2-(2'-pyridyl)ethyl group, a 3-(2'-pyridyl)propyl group, a 4-(2'-pyridyl)butyl group, a 5-(2'-pyridyl)pentyl group, a 6-(2'-pyridyl)hexyl group, a 7-(2'-pyridyl)heptyl group, a 8-(2'-pyridyl)octyl group, a 4-cyano-2-pyridylmethyl group, a 4-nitro-2-pyridylmethyl group, a 4-carboxyl-2-pyridylmethyl group, a 4-hydroxyl-2-pyridylmethyl group, a 4-(N-methylcarboamide)-2-pyridylmethyl group, a 4-(N,N-dimethylcarboamide)-2-pyridylmethyl group, a 4-methyl-2-pyridylmethyl group, a 4-trifluoromethyl-2-pyridylmethyl group, a 4-methoxy-2-pyridylmethyl group, a 4-trifluoromethoxy-2-pyridylmethyl group, a 4-methylthio-2-pyridylmethyl group, a 4-methylsulfinyl-2-pyridylmethyl group, a 4-methylsulfonyl-2-pyridylmethyl group, a 4-methoxycarbonyl-2-pyridylmethyl group, a 4-vinyl-2-pyridylmethyl group, a 4-(2',2'-difluorovinyl)-2-pyridylmethyl group, an 4-ethynyl-2-pyridylmethyl group, a 4-(2'-fluoroethynyl)-2-pyridylmethyl group, a 4-fluoro-2-pyridylmethyl group, a 4-chloro-2-pyridylmethyl group, a 5-cyano-2-pyridylmethyl group, a 5-nitro-2-pyridylmethyl group, a 5-carboxyl-2-pyridylmethyl group, a 5-hydroxyl-2-pyridylmethyl group, a 5-(N-methylcarboamide)-2-pyridylmethyl group, a 5-(N,N-dimethylcarboamide)-2-pyridylmethyl group, a 5-methyl-2-pyridylmethyl group, a 5-trifluoromethyl-2-pyridylmethyl group, a 5-methoxy-2-pyridylmethyl group, a 5-trifluoromethoxy-2-pyridylmethyl group, a 5-methylthio-2-pyridylmethyl group, a 5-methylsulfinyl-2-pyridylmethyl group, a 5-methylsulfonyl-2-pyridylmethyl group, a 5-methoxycarbonyl-2-pyridylmethyl group, a 5-vinyl-2-pyridylmethyl group, a 5-(2',2'-difluorovinyl)-2-pyridylmethyl group, an 5-ethynyl-2-pyridylmethyl group, a 5-(2'-fluoroethynyl)-2-pyridylmethyl group, a 5-fluoro-2-pyridylmethyl group, a 5-chloro-2-pyridylmethyl group, a 5,5-dichloro-2-pyridylmethyl group, a 6-cyano-2-pyridylmethyl group, a 6-nitro-2-pyridylmethyl group, a 6-carboxyl-2-pyridylmethyl group, a 6-hydroxyl-2-pyridylmethyl group, a 6-(N-methylcarboamide)-2-pyridylmethyl group, a 6-(N,N-dimethylcarboamide)-2-pyridylmethyl group, a 6-methyl-2-pyridylmethyl group, a 6-trifluoromethyl-2-pyridylmethyl group, a 6-methoxy-2-pyridylmethyl group, a 6-trifluoromethoxy-2-pyridylmethyl group, a 6-methylthio-2-pyridylmethyl group, a 6-methylsulfinyl-2-pyridylmethyl group, a 6-methylsulfonyl-2-pyridylmethyl group, a 6-methoxycarbonyl-2-pyridylmethyl group, a 6-vinyl-2-pyridylmethyl group, a 6-(2',2'-difluorovinyl)-2-pyridylmethyl group, an 6-ethynyl-2-pyridylmethyl group, a 6-(2'-fluoroethynyl)-2-pyridylmethyl group, a 6-fluoro-2-pyridylmethyl group, a 6-chloro-2-pyridylmethyl group, a 6,6-dichloro-2-pyridylmethyl group, a 3-pyridylmethyl group, a 1-(3'-pyridyl)ethyl group, a 2-(3'-pyridyl)ethyl group, a 3-(3'-pyridyl)propyl group, a 4-(3'-pyridyl)butyl group, a 5-(3'-pyridyl)pentyl group, a 6-(3'-pyridyl)hexyl group, a 7-(3'-pyridyl)heptyl group, a 8-(3'-pyridyl)octyl group, a 5-cyano-3-pyridylmethyl group, a 5-nitro-3-pyridylmethyl group, a 5-carboxyl-3-pyridylmethyl group, a 5-hydroxyl-3-pyridylmethyl group, a 5-(N-methylcarboamide)-3-pyridylmethyl group, a 5-(N,N-dimethylcarboamide)-3-pyridylmethyl group, a 5-methyl-3-pyridylmethyl group, a 5-trifluoromethyl-3-pyridylmethyl group, a 5-methoxy-3-pyridylmethyl group, a 5-trifluoromethoxy-3-pyridylmethyl group, a 5-methylthio-3-pyridylmethyl group, a 5-methylsulfinyl-3-pyridylmethyl group, a 5-methylsulfonyl-3-pyridylmethyl group, a 5-methoxycarbonyl-3-pyridylmethyl group, a 5-vinyl-3-pyridylmethyl group, a 5-(2',2'-difluorovinyl)-3-pyridylmethyl group, an 5-ethynyl-3-pyridylmethyl group, a 5-(2'-fluoroethynyl)-3-pyridylmethyl group, a 5-fluoro-3-pyridylmethyl group, a 5-chloro-3-pyridylmethyl group, a 6-cyano-3-pyridylmethyl group, a 6-nitro-3-pyridylmethyl group, a 6-carboxyl-3-pyridylmethyl group, a 6-hydroxyl-3-pyridylmethyl group, a 6-(N-methylcarboamide)-3-pyridylmethyl group, a 6-(N,N-dimethylcarboamide)-3-pyridylmethyl group, a 6-methyl-3-pyridylmethyl group, a 6-trifluoromethyl-3-pyridylmethyl group, a 6-methoxy-3-pyridylmethyl group, a 6-trifluoromethoxy-3-pyridylmethyl group, a 6-methylthio-3-pyridylmethyl group, a 6-methylsulfinyl-3-pyridylmethyl group, a 6-methylsulfonyl-3-pyridylmethyl group, a 6-methoxycarbonyl-3-pyridylmethyl group, a 6-vinyl-3-pyridylmethyl group, a 6-(2',2'-difluorovinyl)-3-pyridylmethyl group, an 6-ethynyl-3-pyridylmethyl group, a 6-(2'-fluoroethynyl)-3-pyridylmethyl group, a 6-fluoro-3-pyridylmethyl group, a 6-chloro-3-pyridylmethyl group, a 4-pyridylmethyl group, a 1-(4'-pyridyl)ethyl group, a 2-(4'-pyridyl)ethyl group, a 3-(4'-pyridyl)propyl group, a 4-(4'-pyridyl)butyl group, a 5-(4'-pyridyl)pentyl group, a 6-(4'-pyridyl)hexyl group, a 7-(4'-pyridyl)heptyl group, a 8-(4'-pyridyl)octyl group, a 2-cyano-4-pyridylmethyl group, a 2-nitro-4-pyridylmethyl group, a 2-carboxyl-4-pyridylmethyl group, a 2-hydroxyl-4-pyridylmethyl group, a 2-(N-methylcarboamide)-4-pyridylmethyl group, a 2-(N,N-dimethylcarboamide)-4-pyridylmethyl group, a 2-methyl-4-pyridylmethyl group, a 2-trifluoromethyl-4-pyridylmethyl group, a 2-methoxy-4-pyridylmethyl group, a 2-trifluoromethoxy-4-pyridylmethyl group, a 2-methylthio-4-pyridylmethyl group, a 2-methylsulfinyl-4-pyridylmethyl group, a 2-methylsulfonyl-4-pyridylmethyl group, a 2-methoxycarbonyl-4-pyridylmethyl group, a 2-vinyl-4-pyridylmethyl group, a 2-(2',2'-difluorovinyl)-4-pyridylmethyl group, an 2-ethynyl-4-pyridylmethyl group, a 2-(2'-fluoroethynyl)-4-pyridylmethyl group, a 2-fluoro-4-pyridylmethyl group, a 2-chloro-4-pyridylmethyl group, a 6-cyano-4-pyridylmethyl group, a 6-nitro-4-pyridylmethyl group, a 6-carboxyl-4-pyridylmethyl group, a 6-hydroxyl-4-pyridylmethyl group, a 6-(N-methylcarboamide)-4-pyridylmethyl group, a 6-(N,N-dimethylcarboamide)-4-pyridylmethyl group, a 6-methyl-4-pyridylmethyl group, a 6-trifluoromethyl-4-pyridylmethyl group, a 6-methoxy-4-pyridylmethyl group, a 6-trifluoromethoxy-4-pyridylmethyl group, a 6-methylthio-4-pyridylmethyl group, a 6-methylsulfinyl-4-pyridylmethyl group, a 6-methylsulfonyl-4-pyridylmethyl group, a 6-methoxycarbonyl-4-pyridylmethyl group, a 6-vinyl-4-pyridylmethyl group, a 6-(2',2'-difluorovinyl)-4-pyridylmethyl group, an 6-ethynyl-4-pyridylmethyl group, a 6-(2'-fluoroethynyl)-4-pyridylmethyl group, a 6-fluoro-4-pyridylmethyl group, a 6-chloro-4-pyridylmethyl group, a 2-quinolylmethyl group, a 1-(2'-quinolyl)ethyl group, a 2-(2'-quinolyl)ethyl group, a 3-(2'-quinolyl)propyl group, a 4-(2'-quinolyl)butyl group, a 5-(2'-quinolyl)pentyl group, a 6-(2'-quinolyl)hexyl group, a 7-(2'-quinolyl)heptyl group, a 8-(2'-quinolyl)octyl group, a 6-cyano-2-quinolylmethyl group, a 6-nitro-2-quinolylmethyl group, a 6-carboxyl-2-quinolylmethyl group, a 6-hydroxyl-2-quinolylmethyl group, a 6-(N-methylcarboamide)-2-quinolylmethyl group, a 6-(N,N-dimethylcarboamide)-2-quinolylmethyl group, a 6-methyl-2-quinolylmethyl group, 6-trifluoromethyl-2-quinolylmethyl group, a 6-methoxy-2-quinolylmethyl group, a 6-trifluoromethoxy-2-quinolylmethyl group, a 6-methylthio-2-quinolylmethyl group, a 6-methylsulfinyl-2-quinolylmethyl group, a 6-methylsulfonyl-2-quinolylmethyl group, a 6-methoxycarbonyl-2-quinolylmethyl group, a 6-vinyl-2-quinolylmethyl group, a 6-(2',2'-difluorovinyl) -2-quinolylmethyl group, an 6-ethynyl-2-quinolylmethyl group, a 6-(2'-fluoroethynyl)-2-quinolylmethyl group, a 6-fluoro-2-quinolylmethyl group, a 6-chloro-2-quinolylmethyl group, a 3-quinolylmethyl group, a 1-(3'-quinolyl)ethyl group, a 2-(3'-quinolyl)ethyl group, a 3-(3'-quinolyl)propyl group, a 4-(3'-quinolyl)butyl group, a 5-(3'-quinolyl)pentyl group, a 6-(3'-quinolyl)hexyl group, a 7-(3'-quinolyl)heptyl group, a 8-(3'-quinolyl)octyl group, a 6-cyano-3-quinolylmethyl group, a 6-nitro-3-quinolylmethyl group, a 6-carboxyl-3-quinolylmethyl group, a 6-hydroxyl-3-quinolylmethyl group, a 6-(N-methylcarboamide)-3-quinolylmethyl group, a 6-(N,N-dimethylcarboamide)-3-quinolylmethyl group, a 6-methyl-3-quinolylmethyl group, a 6-trifluoromethyl-3-quinolylmethyl group, a 6-methoxy-3-quinolylmethyl group, a 6-trifluoromethoxy-3-quinolylmethyl group, a 6-methylthio-3-quinolylmethyl group, a 6-methylsulfinyl-3-quinolylmethyl group, a 6-methylsulfonyl-3-quinolylmethyl group, a 6-methoxycarbonyl-3-quinolylmethyl group, a 6-vinyl-3-quinolylmethyl group, a 6-(2',2'-difluorovinyl) -3-quinolylmethyl group, an 6-ethynyl-3-quinolylmethyl group, a 6-(2'-fluoroethynyl)-3-quinolylmethyl group, a 6-fluoro-3-quinolylmethyl group, a 6-chloro-3-quinolylmethyl group, a 4-quinolylmethyl group, a 1-(4'-quinolyl)ethyl group, a 2-(4'-quinolyl)ethyl group, a 3-(4'-quinolyl)propyl group, a 4-(4'-quinolyl)butyl group, a 5-(4'-quinolyl)pentyl group, a 6-(4'-quinolyl)hexyl group, a 7-(4'-quinolyl)heptyl group, a 8-(4'-quinolyl)octyl group, a 6-cyano-4-quinolylmethyl group, a 6-nitro-4-quinolylmethyl group, a 6-carboxyl-4-quinolylmethyl group, a 6-hydroxyl-4-quinolylmethyl group, a 6-(N-methylcarboamide)-4-quinolylmethyl group, a 6-(N,N-dimethylcarboamide)-4-quinolylmethyl group, a 6-methyl-4-quinolylmethyl group, a 6-trifluoromethyl-4-quinolylmethyl group, a 6-methoxy-4-quinolylmethyl group, a 6-trifluoromethoxy-4-quinolylmethyl group, a 6-methylthio-4-quinolylmethyl group, a 6-methylsulfinyl-4-quinolylmethyl group, a 6-methylsulfonyl-4-quinolylmethyl group, a 6-methoxycarbonyl-4-quinolylmethyl group, a 6-vinyl-4-quinolylmethyl group, a 6-(2',2'-difluorovinyl) -4-quinolylmethyl group, an 6-ethynyl-4-quinolylmethyl group, a 6-(2'-fluoroethynyl)-4-quinolylmethyl group, a 6-fluoro-4-quinolylmethyl group, a 6-chloro-4-quinolylmethyl group, a 2-furylmethyl group, a 1-(2'-furyl)ethyl group, a 2-(2'-furyl)ethyl group, a 3-(2'-furyl)propyl group, a 4-(2'-furyl)butyl group, a 5-(2'-furyl)pentyl group, a 6-(2'-furyl)hexyl group, a 7-(2'-furyl)heptyl group, a 8-(2'-furyl)octyl group, a 4-cyano-2-furylmethyl group, a 4-nitro-2-furylmethyl group, a 4-carboxyl-2-furylmethyl group, a 4-hydroxyl-2-furylmethyl group, a 4-(N-methylcarboamide)-2-furylmethyl group, a 4-(N,N-dimethylcarboamide)-2-furylmethyl group, a 4-methyl-2-furylmethyl group, a 4-trifluoromethyl-2-furylmethyl group, a 4-methoxy-2-furylmethyl group, a 4-trifluoromethoxy-2-furylmethyl group, a 4-methylthio-2-furylmethyl group, a 4-methylsulfinyl-2-furylmethyl group, a 4-methylsulfonyl-2-furylmethyl group, a 4-methoxycarbonyl-2-furylmethyl group, a 4-vinyl-2-furylmethyl group, a 4-(2',2'-difluorovinyl)-2-furylmethyl group, an 4-ethynyl-2-furylmethyl group, a 4-(2'-fluoroethynyl)-2-furylmethyl group, a 4-fluoro-2-furylmethyl group, a 4-chloro-2-furylmethyl group, a 3-furylmethyl group, a 1-(3'-furyl)ethyl group, a 2-(3'-furyl)ethyl group, a 3-(3'-furyl)propyl group, a 4-(3'-furyl)butyl group, a 5-(3'-furyl)pentyl group, a 6-(3'-furyl)hexyl group, a 7-(3'-furyl)heptyl group, a 8-(3'-furyl)octyl group, a 4-cyano-3-furylmethyl group, a 4-nitro-3-furylmethyl group, a 4-carboxyl-3-furylmethyl group, a 4-hydroxyl-3-furylmethyl group, a 4-(N-methylcarboamide)-3-furylmethyl group, a 4-(N,N-dimethylcarboamide)-3-furylmethyl group, a 4-methyl-3-furylmethyl group, a 4-trifluoromethyl-3-furylmethyl group, a 4-methoxy-3-furylmethyl group, a 4-trifluoromethoxy-3-furylmethyl group, a 4-methylthio-3-furylmethyl group, a 4-methylsulfinyl-3-furylmethyl group, a 4-methylsulfonyl-3-furylmethyl group, a 4-methoxycarbonyl-3-furylmethyl group, a 4-vinyl-3-furylmethyl group, a 4-(2',2'-difluorovinyl)-3-furylmethyl group, an 4-ethynyl-3-furylmethyl group, a 4-(2'-fluoroethynyl)-3-furylmethyl group, a 4-fluoro-3-furylmethyl group, a 4-chloro-3-furylmethyl group, a 2-thienylmethyl group, a 1-(2'-thienyl)ethyl group, a 2-(2'-thienyl)ethyl group, a 3-(2'-thienyl)propyl group, a 4-(2'-thienyl)butyl group, a 5-(2'-thienyl)pentyl group, a 6-(2'-thienyl)hexyl group, a 7-(2'-thienyl)heptyl group, a 8-(2'-thienyl)octyl group, a 4-cyano-2-thienylmethyl group, a 4-nitro-2-thienylmethyl group, a 4-carboxyl-2-thienylmethyl group, a 4-hydroxyl-2-thienylmethyl group, a 4-(N-methylcarboamide)-2-thienylmethyl group, a 4-(N,N-dimethylcarboamide)-2-thienylmethyl group, a 4-methyl-2-thienylmethyl group, a 4-trifluoromethyl-2-thienylmethyl group, a 4-methoxy-2-thienylmethyl group, a 4-trifluoromethoxy-2-thienylmethyl group, a 4-methylthio-2-thienylmethyl group, a 4-methylsulfinyl-2-thienylmethyl group, a 4-methylsulfonyl-2-thienylmethyl group, a 4-methoxycarbonyl-2-thienylmethyl group, a 4-vinyl-2-thienylmethyl group, a 4-(2',2'-difluorovinyl)-2-thienylmethyl group, an 4-ethynyl-2-thienylmethyl group, a 4-(2'-fluoroethynyl)-2-thienylmethyl group, a 4-fluoro-2-thienylmethyl group, a 4-chloro-2-thienylmethyl group, a 3-thienylmethyl group, a 1-(3'-thienyl)ethyl group, a 2-(3'-thienyl)ethyl group, a 3-(3'-thienyl)propyl group, a 4-(3'-thienyl)butyl group, a 5-(3'-thienyl)pentyl group, a 6-(3'-thienyl)hexyl group, a 7-(3'-thienyl)heptyl group, a 8-(3'-thienyl)octyl group, a 4-cyano-3-thienylmethyl group, a 4-nitro-3-thienylmethyl group, a 4-carboxyl-3-thienylmethyl group, a 4-hydroxyl-3-thienylmethyl group, a 4-(N-methylcarboamide)-3-thienylmethyl group, a 4-(N,N-dimethylcarboamide)-3-thienylmethyl group, a 4-methyl-3-thienylmethyl group, a 4-trifluoromethyl-3-thienylmethyl group, a 4-methoxy-3-thienylmethyl group, a 4-trifluoromethoxy-3-thienylmethyl group, a 4-methylthio-3-thienylmethyl group, a 4-methylsulfinyl-3-thienylmethyl group, a 4-methylsulfonyl-3-thienylmethyl group, a 4-methoxycarbonyl-3-thienylmethyl group, a 4-vinyl-3-thienylmethyl group, a 4-(2',2'-difluorovinyl)-3-thienylmethyl group, an 4-ethynyl-3-thienylmethyl group, a 4-(2'-fluoroethynyl)-3-thienylmethyl group, a 4-fluoro-3-thienylmethyl group, a 4-chloro-3-thienylmethyl group, a 2-(1-benzofuranyl)methyl group, a 1-(2'-(1'-benzofuranyl))ethyl group, a 2-(2'-(1'-benzofuranyl))ethyl group, a 3-(2'-(1'-benzofuranyl))propyl group, a 4-(2'-(1'-benzofuranyl))butyl group, a 5-(2'-(1'-benzofuranyl))pentyl group, a 6-(2'-(1'-benzofuranyl))hexyl group, a 7-(2'-(1'-benzofuranyl))heptyl group, a 8-(2'-(1'-benzofuranyl))octyl group, a 5-cyano-2-(1-benzofuranyl)methyl group, a 5-nitro-2-(1-benzofuranyl)methyl group, a 5-carboxyl-2-(1-benzofuranyl)methyl group, a 5-hydroxyl-2-(1-benzofuranyl)methyl group, a 5-(N-methylcarboamide)-2-(1-benzofuranyl)methyl group, a 5-(N,N-dimethylcarboamide)-2-(1-benzofuranyl)methyl group, a 5-methyl-2-(1-benzofuranyl)methyl group, a 5-trifluoromethyl-2-(1-benzofuranyl)methyl group, a 5-methoxy-2-(1-benzofuranyl)methyl group, a 5-trifluoromethoxy-2-(1-benzofuranyl)methyl group, a 5-methylthio-2-(1-benzofuranyl)methyl group, a 5-methylsulfinyl-2-(1-benzofuranyl)methyl group, a 5-methylsulfonyl-2-(1-benzofuranyl)methyl group, a 5-methoxycarbonyl-2-(1-benzofuranyl)methyl group, a 5-vinyl-2-(1-benzofuranyl)methyl group, a 5-(2',2'-difluorovinyl)-2-(1-benzofuranyl)methyl group, an 5-ethynyl-2-(1-benzofuranyl)methyl group, a 5-(2'-fluoroethynyl)-2-(1-benzofuranyl)methyl group, a 5-fluoro-2-(1-benzofuranyl)methyl group, a 5-chloro-2-(1-benzofuranyl)methyl group, a 3-(1-benzofuranyl)methyl group, a 1-(3'-(1'-benzofuranyl))ethyl group, a 2-(3'-(1'-benzofuranyl))ethyl group, a 3-(3'-(1'-benzofuranyl))propyl group, a 4-(3'-(1'-benzofuranyl))butyl group, a 5-(3'-(1'-benzofuranyl))pentyl group, a 6-(3'-(1'-benzofuranyl))hexyl group, a 7-(3'-(1'-benzofuranyl))heptyl group, a 8-(3'-(1'-benzofuranyl))octyl group, a 5-cyano-3-(1-benzofuranyl)methyl group, a 5-nitro-3-(1-benzofuranyl)methyl group, a 5-carboxyl-3-(1-benzofuranyl)methyl group, a 5-hydroxyl-3-(1-benzofuranyl)methyl group, a 5-(N-methylcarboamide)-3-(1-benzofuranyl)methyl group, a 5-(N,N-dimethylcarboamide)-3-(1-benzofuranyl)methyl group, a 5-methyl-3-(1-benzofuranyl)methyl group, a 5-trifluoromethyl-3-(1-benzofuranyl)methyl group, a 5-methoxy-3-(1-benzofuranyl)methyl group, a 5-trifluoromethoxy-3-(1-benzofuranyl)methyl group, a 5-methylthio-3-(1-benzofuranyl)methyl group, a 5-methylsulfinyl-3-(1-benzofuranyl)methyl group, a 5-methylsulfonyl-3-(1-benzofuranyl)methyl group, a 5-methoxycarbonyl-3-(1-benzofuranyl)methyl group, a 5-vinyl-3-(1-benzofuranyl)methyl group, a 5-(2',2'-difluorovinyl)-3-(1-benzofuranyl)methyl group, an 5-ethynyl-3-(1-benzofuranyl)methyl group, a 5-(2'-fluoroethynyl)-3-(1-benzofuranyl)methyl group, a 5-fluoro-3-(1-benzofuranyl)methyl group, a 5-chloro-3-(1-benzofuranyl)methyl group, a 2-(1-benzothienyl)methyl group, a 1-(2'-(1'-benzothienyl))ethyl group, a 2-(2'-(1'-benzothienyl))ethyl group, a 3-(2'-(1'-benzothienyl))propyl group, a 4-(2'-(1'-benzothienyl))butyl group, a 5-(2'-(1'-benzothienyl))pentyl group, a 6-(2'-(1'-benzothienyl))hexyl group, a 7-(2'-(1'-benzothienyl))heptyl group, a 8-(2'-(1'-benzothienyl))octyl group, a 5-cyano-2-(1-benzothienyl)methyl group, a 5-nitro-2-(1-benzothienyl)methyl group, a 5-carboxyl-2-(1-benzothienyl)methyl group, a 5-hydroxyl-2-(1-benzothienyl)methyl group, a 5-(N-methylcarboamide)-2-(1-benzothienyl)methyl group, a 5-(N,N-dimethylcarboamide)-2-(1-benzothienyl)methyl group, a 5-methyl-2-(1-benzothienyl)methyl group, a 5-trifluoromethy-2-(1-benzothienyl)methyl group, a 5-methoxy-2-(1-benzothienyl)methyl group, a 5-trifluoromethoxy-2-(1-benzothienyl)methyl group, a 5-methylthio-2-(1-benzothienyl)methyl group, a 5-methylsulfinyl-2-(1-benzothienyl)methyl group, a 5-methylsulfonyl-2-(1-benzothienyl)methyl group, a 5-methoxycarbonyl-2-(1-benzothienyl)methyl group, a 5-vinyl-2-(1-benzothienyl)methyl group, a 5-(2',2'-difluorovinyl)-2-(1-benzothienyl)methyl group, an 5-ethynyl-2-(1-benzothienyl)methyl group, a 5-(2'-fluoroethynyl)-2-(1-benzothienyl)methyl group, a 5-fluoro-2-(1-benzofuranyl)methyl group, a 5-chloro-2-(1-benzofuranyl)methyl group, a 3-(1-benzothienyl)methyl group, a 1-(3'-(1'-benzothienyl))ethyl group, a 2-(3'-(1'-benzothienyl))ethyl group, a 3-(3'-(1'-benzothienyl))propyl group, a 4-(3'-(1'-benzothienyl))butyl group, a 5-(3'-(1'-benzothienyl))pentyl group, a 6-(3'-(1'-benzothienyl))hexyl group, a 7-(3'-(1'-benzothienyl))heptyl group, a 8-(3'-(1'-benzothienyl))octyl group, a 5-cyano-3-(1-benzothienyl)methyl group, a 5-nitro-3-(1-benzothienyl)methyl group, a 5-carboxyl-3-(1-benzothienyl)methyl group, a 5-hydroxyl-3-(1-benzothienyl)methyl group, a 5-(N-methylcarboamide)-3-(1-benzothienyl)methyl group, a 5-(N,N-dimethylcarboamide)-3-(1-benzothienyl)methyl group, a 5-methyl-3-(1-benzothienyl)methyl group, a 5-trifluoromethy-3-(1-benzothienyl)methyl group, a 5-methoxy-3-(1-benzothienyl)methyl group, a 5-trifluoromethoxy-3-(1-benzothienyl)methyl group, a 5-methylthio-3-(1-benzothienyl)methyl group, a 5-methylsulfinyl-3-(1-benzothienyl)methyl group, a 5-methylsulfonyl-3-(1-benzothienyl)methyl group, a 5-methoxycarbonyl-3-(1-benzothienyl)methyl group, a 5-vinyl-3-(1-benzothienyl)methyl group, a 5-(2',2'-difluorovinyl)-3-(1-benzothienyl)methyl group, an 5-ethynyl-3-(1-benzothienyl)methyl group, a 5-(2'-fluoroethynyl)-3-(1-benzothienyl)methyl group, a 5-fluoro-3-(1-benzofuranyl)methyl group, a 5-chloro-3-(1-benzofuranyl)methyl group, and the like.

Examples of the compound of the present invention include the following compounds.

In the formula (I), compounds wherein X is a C1 to C5 chain hydrocarbon group having a phenyl group; In the formula (I), compounds wherein X is a benzyl group; In the formula (I), compounds wherein X is a 2-phenylethyl group;

In the formula (I), compounds wherein X is a 3-phenylpropyl group;

In the formula (I), compounds wherein X is a 4-phenylbutyl group;

In the formula (I), compounds wherein X is a 5-phenylpentyl group;

In the formula (I), compounds wherein X is a C1 to C5 alkyl group having a phenyl group;

In the formula (I), compounds wherein X is a C1 to C5 chain hydrocarbon group having a 1-naphthyl group;

In the formula (I), compounds wherein X is a 1-naphthylmethyl group;

In the formula (I), compounds wherein X is a 2-(1'-naphthyl)ethyl group;

In the formula (I), compounds wherein X is a 3-(1'-naphthyl)propyl group;

In the formula (I), compounds wherein X is a 4-(1'-naphthyl)butyl group;

In the formula (I), compounds wherein X is a 5-(1'-naphthyl)pentyl group;

In the formula (I), compounds wherein X is a C1 to C5 alkyl group having a 1-naphthyl group;

In the formula (I), compounds wherein X is a C1 to C5 chain hydrocarbon group having a 2-naphthyl group;

In the formula (I), compounds wherein X is a 2-naphthylmethyl group;

In the formula (I), compounds wherein X is a 2-(2'-naphthyl)ethyl group;

In the formula (I), compounds wherein X is a 3-(2'-naphthyl)propyl group;

In the formula (I), compounds wherein X is a 4-(2'-naphthyl)butyl group;

In the formula (I), compounds wherein X is a 5-(2'-naphthyl)pentyl group;

In the formula (I), compounds wherein X is a C1 to C5 alkyl group having a 2-naphthyl group;

In the formula (I), compounds wherein X is a C1 to C5 chain hydrocarbon group having a 2-pyridyl group;

In the formula (I), compounds wherein X is a 2-pyridylmethyl group;

In the formula (I), compounds wherein X is a 2-(2'-pyridyl) ethyl group;

In the formula (I), compounds wherein X is a 3-(2'-pyridyl)propyl group;

In the formula (I), compounds wherein X is a 4-(2'-pyridyl)butyl group;

In the formula (I), compounds wherein X is a 5-(2'-pyridyl)pentyl group;

In the formula (I), compounds wherein X is a C1 to C5 alkyl group having a 2-pyridyl group;

In the formula (I), compounds wherein X is a C1 to C5 chain hydrocarbon group having a 3-pyridyl group;

In the formula (I), compounds wherein X is a 3-pyridylmethyl group;

In the formula (I), compounds wherein X is a 2-(3'-pyridyl) ethyl group;

In the formula (I), compounds wherein X is a 3-(3'-pyridyl)propyl group;

In the formula (I), compounds wherein X is a 4-(3'-pyridyl)butyl group;

In the formula (I), compounds wherein X is a 5-(3'-pyridyl)pentyl group;

In the formula (1), compounds wherein X is a C1 to C5 alkyl group having a 3-pyridyl group;

In the formula (I), compounds wherein X is a C1 to C5 chain hydrocarbon group having a 4-pyridyl group;

In the formula (I), compounds wherein X is a 4-pyridylmethyl group;

In the formula (I), compounds wherein X is a 2- (4'-pyridyl) ethyl group;

In the formula (I), compounds wherein X is a 3-(4'-pyridyl)propyl group;

In the formula (I), compounds wherein X is a 4- (4'-pyridyl) butyl group;

In the formula (I), compounds wherein X is a 5-(4'-pyridyl)pentyl group;

In the formula (I), compounds wherein X is a C1 to C5 alkyl group having a 4-pyridyl group;

In the formula (I), compounds wherein X is a C1 to C5 chain hydrocarbon group having a 2-quinolyl group;

In the formula (I), compounds wherein X is a 2-quinolylmethyl group;

In the formula (I), compounds wherein X is a 2-(2'-quinolyl)ethyl group;

In the formula (I), compounds wherein X is a 3-(2'-quinolyl)propyl group;

In the formula (I), compounds wherein X is a 4-(2'-quinolyl)butyl group;

In the formula (I), compounds wherein X is a 5-(2'-quinolyl)pentyl group;

In the formula (I), compounds wherein X is a C1 to C5 alkyl group having a 2-quinolyl group;

In the formula (I), compounds wherein X is a C1 to C5 chain hydrocarbon group having a 3-quinolyl group;

In the formula (I), compounds wherein X is a 3-quinolylmethyl group;

In the formula (I), compounds wherein X is a 2-(3'-quinolyl)ethyl group;

In the formula (I), compounds wherein X is a 3-(3'-quinolyl)propyl group;

In the formula (I), compounds wherein X is a 4-(3'-quinolyl)butyl group;

In the formula (I), compounds wherein X is a 5-(3'-quinolyl)pentyl group;

In the formula (I), compounds wherein X is a C1 to C5 alkyl group having a 3-quinolyl group;

In the formula (I), compounds wherein X is a C1 to C5 chain hydrocarbon group having a 4-quinolyl group;

In the formula (I), compounds wherein X is a 4-quinolylmethyl group;

In the formula (I), compounds wherein X is a 2-(4'-quinolyl)ethyl group;

In the formula (I), compounds wherein X is a 3-(4'-quinolyl)propyl group;

In the formula (I), compounds wherein X is a 4-(4'-quinolyl)butyl group;

In the formula (I), compounds wherein X is a 5-(4'-quinolyl)pentyl group;

In the formula (I), compounds wherein X is a C1 to C5 alkyl group having a 4-quinolyl group;

In the formula (I), compounds wherein X is a C1 to C5 chain hydrocarbon group having a 2-furyl group;

In the formula (I), compounds wherein X is a 2-furylmethyl group;

In the formula (I), compounds wherein X is a 2- (2'-furyl) ethyl group;

In the formula (I), compounds wherein X is a 3- (2'-furyl)propyl group;

In the formula (I), compounds wherein X is a 4-(2'-furyl)butyl group;

In the formula (I), compounds wherein X is a 5- (2' -furyl) pentyl group;

In the formula (I), compounds wherein X is a C1 to C5 alkyl group having a 2-furyl group;

In the formula (I), compounds wherein X is a C1 to C5 chain hydrocarbon group having a 3-furyl group;

In the formula (I), compounds wherein X is a 3-furylmethyl group;

In the formula (I), compounds wherein X is a 2-(3'-furyl) ethyl group;

In the formula (I), compounds wherein X is a 3-(3'-furyl) propyl group;

In the formula (I), compounds wherein X is a 4-(3'-furyl)butyl group;

In the formula (I), compounds wherein X is a 5-(3'-furyl)pentyl group;

In the formula (I), compounds wherein X is a C1 to C5 alkyl group having a 3-furyl group;

In the formula (I), compounds wherein X is a C1 to C5 chain hydrocarbon group having a 2-thienyl group;

In the formula (I), compounds wherein X is a 2-thienylmethyl group;

In the formula (I), compounds wherein X is a 2-(2'-thienyl) ethyl group;

In the formula (I), compounds wherein X is a 3-(2'-thienyl)propyl group;

In the formula (I), compounds wherein X is a 4-(2'-thienyl)butyl group;

In the formula (I), compounds wherein X is a 5-(2'-thienyl)pentyl group;

In the formula (I), compounds wherein X is a C1 to C5 alkyl group having a 2-thienyl group;

In the formula (I), compounds wherein X is a C1 to C5 chain hydrocarbon group having a 3-thienyl group; In the formula (I), compounds wherein X is a 3-thienylmethyl group;

In the formula (I), compounds wherein X is a 2-(3'-thienyl) ethyl group;

In the formula (I), compounds wherein X is a 3-(3'-thienyl)propyl group;

In the formula (I), compounds wherein X is a 4-(3'-thienyl) butyl group;

In the formula (I), compounds wherein X is a 5-(3'-thienyl)pentyl group;

In the formula (I), compounds wherein X is a C1 to C5 alkyl group having a 3-thienyl group;

In the formula (I), compounds wherein X is a C1 to C5 chain hydrocarbon group having a 2-(1-benzofuranyl) group;

In the formula (I), compounds wherein X is a 2-(1-benzofuranyl)methyl group;

In the formula (I), compounds wherein X is a 2-(2'-(1'-benzofuranyl))ethyl group;

In the formula (I), compounds wherein X is a 3-(2'-(1'-benzofuranyl))propyl group;

In the formula (I), compounds wherein X is a 4-(2'-(1'-benzofuranyl))butyl group;

In the formula (I), compounds wherein X is a 5-(2'-(1'-benzofuranyl))pentyl group;

In the formula (I), compounds wherein X is a C1 to C5 alkyl group having a 2-(1-benzofuranyl) group;

In the formula (I), compounds wherein X is a C1 to C5 chain hydrocarbon group having a 3-(1-benzofuranyl) group;

In the formula (I), compounds wherein X is a 3-(1-benzofuranyl)methyl group;

In the formula (I), compounds wherein X is a 2-(3'-(1'-benzofuranyl))ethyl group;

In the formula (I), compounds wherein X is a 3-(3'-(1'-benzofuranyl))propyl group;

In the formula (I), compounds wherein X is a 4-(3'-(1'-benzofuranyl))butyl group;

In the formula (I), compounds wherein X is a 5-(3'-(1'-benzofuranyl))pentyl group;

In the formula (I), compounds wherein X is a C1 to C5 alkyl group having a 3-(1-benzofuranyl) group;

In the formula (I), compounds wherein X is a C1 to C5 chain hydrocarbon group having a 2-(1-benzothienyl) group;

In the formula (I), compounds wherein X is a 2-(1-benzothienyl)methyl group;

In the formula (I), compounds wherein X is a 2-(2'-(1'-benzothienyl))ethyl group;

In the formula (I), compounds wherein X is a 3-(2'-(1'-benzothienyl))propyl group;

In the formula (I), compounds wherein X is a 4-(2'-(1'-benzothienyl))butyl group;

In the formula (I), compounds wherein X is a 5-(2'-(1'-benzothienyl))pentyl group;

In the formula (I), compounds wherein X is a C1 to C5 alkyl group having a 2-(1-benzothienyl) group;

In the formula (I), compounds wherein X is a C1 to C5 chain hydrocarbon group having a 3-(1-benzothienyl) group;

In the formula (I), compounds wherein X is a 3-(1-benzothienyl)methyl group;

In the formula (I), compounds wherein X is a 2-(3'-(1'-benzothienyl))ethyl group;

In the formula (I), compounds wherein X is a 3-(3'-(1'-benzothienyl))propyl group;

In the formula (I), compounds wherein X is a 4-(3'-(1'-benzothienyl))butyl group;

In the formula (I), compounds wherein X is a 5-(3'-(1'-benzothienyl))pentyl group;

In the formula (I), compounds wherein X is a C1 to C5 alkyl group having a 3-(1-benzothienyl) group;

In the formula (I), compounds wherein the relative arrangement of R¹ and R² is a trans configuration, more specifically, compounds represented by formula (III);

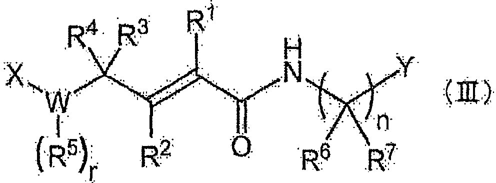

In the formula (I), compounds that are rich in compounds wherein the relative arrangement of R¹ and R² is a trans configuration; In the formula (I), compounds that contain 80% or more of compounds wherein the relative arrangement of R¹ and R² is a trans configuration;

In the formula (I), compounds that contain 90% or more of compounds wherein the relative arrangement of R¹ and R² is a trans configuration;

In the formula (I), compounds wherein R¹ is a hydrogen atom or a halogen atom, and R² is a C1 to C4 alkyl group optionally having one or more halogen atoms or a hydrogen atom;

In the formula (I), compounds wherein R¹ is a hydrogen atom or a fluorine atom, and R² is a methyl group or a hydrogen atom; In the formula (I), compounds wherein W is -CR⁸-, and r is 1, more specifically, compounds represented by formula (IV);

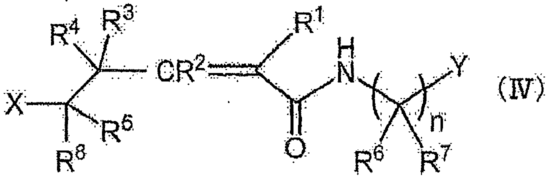

In the formula (IV), compounds wherein R⁸ is a C1 to C4 alkyl group optionally having one or more halogen atoms, a hydrogen atom or a halogen atom, R⁵ is a C1 to C4 alkyl group optionally having one or more halogen atoms, a hydrogen atom or a halogen atom, and R³ and R⁴ are the same or different and are a C1 to C4 alkyl group optionally having one or more halogen atoms, a hydrogen atom or a halogen atom;

In the formula (IV), compounds wherein R⁸ is a methyl group optionally having one or more halogen atoms, a hydrogen atom or a halogen atom, R⁵ is a methyl group optionally having one or more halogen atoms, a hydrogen atom or a halogen atom, and R³ and R⁴ are the same or different and are a methyl group optionally having one or more halogen atoms, a hydrogen atom or a halogen atom;

In the formula (IV), compounds wherein R⁸ is a methyl group or a hydrogen atom, R⁵ is a methyl group or a hydrogen atom, and R³ and R⁴ are a hydrogen atom;

In the formula (IV), compounds wherein R⁸ is a hydrogen atom, R⁵ is a hydrogen atom, and R³ and R⁴ are a hydrogen atom;

In the formula (IV), compounds wherein R⁸ is a methyl group or a hydrogen atom, R⁵ is a methyl group or a hydrogen atom, R³ and R⁴ are a hydrogen atom, R¹ is a hydrogen atom or a fluorine atom, and R² is a methyl group or a hydrogen atom (hereinafter represented as compound (IV-1));

In the formula (IV), compounds wherein R⁸ is a hydrogen atom, R⁵ is a hydrogen atom, R³ and R⁴ are a hydrogen atom, R¹ is a hydrogen atom or a fluorine atom, and R² is a methyl group or a hydrogen atom (hereinafter represented as compound (IV-2)); In the formula (IV-1), compounds wherein the relative arrangement of R¹ and R² is a trans configuration;

In the formula (IV-1), compounds that are rich in compounds wherein the relative arrangement of R¹ and R² is a trans configuration;

In the formula (IV-1), compounds that contain 80% or more of compounds wherein the relative arrangement of R¹ and R² is a trans configuration;

In the formula (IV-1), compounds that contain 90% or more of compounds wherein the relative arrangement of R¹ and R² is a trans configuration;

In the formula (IV-2), compounds wherein the relative arrangement of R¹ and R² is a trans configuration;

In the formula (IV-2), compounds that are rich in compounds wherein the relative arrangement of R¹ and R² is a trans configuration;

In the formula (IV-2), compounds that contain 80% or more of compounds wherein the relative arrangement of R¹ and R² is a trans configuration;

In the formula (IV-2), compounds that contain 90% or more of compounds wherein the relative arrangement of R¹ and R² is a trans configuration;

In the formula (I), compounds wherein W is -CR⁸-, r is 1, and R³ and R⁵ are taken together to be -(CR⁹R¹⁰)ᵥ-, more specifically, compounds represented by formula (V);

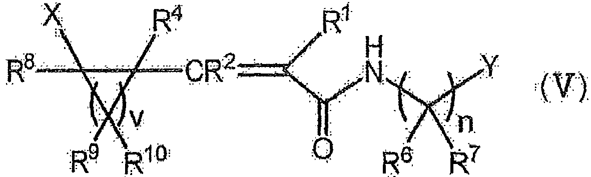

In the formula (V), compounds wherein R⁴ is a C1 to C4 alkyl group optionally having one or more halogen atoms, a hydrogen atom or a halogen atom, R⁸ is a C1 to C4 alkyl group optionally having one or more halogen atoms, a hydrogen atom or a halogen atom, and R⁹ and R¹⁰ are the same or different and are a C1 to C4 alkyl group optionally having one or more halogen atoms, a hydrogen atom or a halogen atom;

In the formula (V), compounds wherein v is 1, R⁴ is a C1 to C4 alkyl group optionally having one or more halogen atoms, a hydrogen atom or a halogen atom, R⁸ is a C1 to C4 alkyl group optionally having one or more halogen atoms, a hydrogen atom or a halogen atom, and R⁹ and R¹⁰ are the same or different and are a C1 to C4 alkyl group optionally having one or more halogen atoms, a hydrogen atom or a halogen atom;

In the formula (V), compounds wherein v is 1, R⁴ is a methyl group optionally having one or more halogen atoms, a hydrogen atom or a halogen atom, R⁸ is a methyl group optionally having one or more halogen atoms, a hydrogen atom or a halogen atom, and R⁹ and R¹⁰ are the same or different and are a methyl group optionally having one or more halogen atoms, a hydrogen atom or a halogen atom;

In the formula (V), compounds wherein v is 1, R⁴ is a methyl group, a hydrogen atom or a fluorine atom, R⁸ is a methyl group or a hydrogen atom, and R⁹ and R¹⁰ are the same or different and are a methyl group or a hydrogen atom;

In the formula (V), compounds wherein v is 1, R⁴ is a hydrogen atom, R⁸ is a hydrogen atom, and R⁹ and R¹⁰ are a hydrogen atom; In the formula (V), compounds wherein v is 1, the relative arrangement of R⁴ and R⁸ is trans, R⁴ is a methyl group, a hydrogen atom or a fluorine group, R⁸ is a methyl group or a hydrogen atom, and R⁹ and R¹⁰ are the same or different and are a methyl group or a hydrogen atom;

In the formula (V), compounds wherein v is 1, the relative arrangement of R⁴ and R⁸ is trans, R⁴ is a hydrogen atom, R⁸ is a hydrogen atom, and R⁹ and R¹⁰ are a hydrogen atom;

In the formula (V), compounds wherein v is 1, the relative arrangement of R⁴ and R⁸ is trans, R⁴ is a methyl group, a hydrogen atom or a fluorine atom, R⁸ is a methyl group or a hydrogen atom, R⁹ and R¹⁰ are the same or different and are a methyl group or a hydrogen atom, R¹ is a hydrogen atom or a fluorine atom, and R² is a methyl group or a hydrogen atom (hereinafter represented as compound (V-1));

In the formula (V), compounds wherein v is 1, the relative arrangement of R⁴ and R⁸ is trans, R⁴ is a hydrogen atom, R⁸ is a hydrogen atom, R⁹ and R¹⁰ are a hydrogen atom, R¹ is a hydrogen atom or a fluorine atom, and R² is a methyl group or a hydrogen atom (hereinafter represented as compound (V-2));

In the formula (V-1), compounds wherein the relative arrangement of R¹ and R² is a trans configuration;

In the formula (V-1), compounds that are rich in compounds wherein the relative arrangement of R¹ and R² is a trans configuration;

In the formula (V-1), compounds that contain 80% or more of compounds wherein the relative arrangement of R¹ and R² is a trans configuration;

In the formula (V-1), compounds that contain 90% or more of compounds wherein the relative arrangement of R¹ and R² is a trans configuration;

In the formula (V-2), compounds wherein the relative arrangement of R¹ and R² is a trans configuration;

In the formula (V-2), compounds that are rich in compounds wherein the relative arrangement of R¹ and R² is a trans configuration;

In the formula (V-2), compounds that contain 80% or more of compounds wherein the relative arrangement of R¹ and R² is a trans configuration;

In the formula (V-2), compounds that contain 90% or more of compounds wherein the relative arrangement of R¹ and R² is a trans configuration;

In the formula (I), compounds wherein W is an oxygen atom, and r is 0, more specifically, compounds represented by formula (VI);

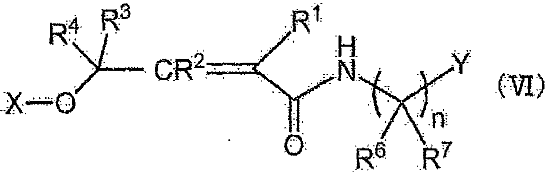

In the formula (VI), compounds wherein R³ and R⁴ are the same or different and are a C1 to C4 alkyl group optionally having one or more halogen atoms, a hydrogen atom or a halogen atom; In the formula (VI), compounds wherein R³ and R⁴ are the same or different and are a methyl group optionally having one or more halogen atoms, a hydrogen atom or a halogen atom;

In the formula (VI), compounds wherein R³ and R⁴ are a hydrogen atom;

In the formula (VI), compounds wherein R³ and R⁴ are a hydrogen atom, R¹ is a hydrogen atom or a fluorine atom, and R² is a methyl group or a hydrogen atom (hereinafter represented as compound (VI-1));

In the formula (VI-1), compounds wherein the relative arrangement of R¹ and R² is a trans configuration;

In the formula (VI-1), compounds that are rich in compounds wherein the relative arrangement of R¹ and R² is a trans configuration;

In the formula (VI-1), compounds that contain 80% or more of compounds wherein the relative arrangement of R¹ and R² is a trans configuration;

In the formula (VI-1), compounds that contain 90% or more of compounds wherein the relative arrangement of R¹ and R² is a trans configuration;

In the formula (I), compounds wherein R⁶ and R⁷ are the same or different and are a C1 to C4 alkyl group optionally having one or more halogen atoms or a hydrogen atom;

In the formula (I), compounds wherein R⁶ and R⁷ are the same or different and are a methyl group optionally having one or more halogen atoms or a hydrogen atom;

In the formula (I), compounds wherein R⁶ and R⁷ are the same or different and are a methyl group or a hydrogen atom;

In the formula (I), compounds wherein Y is a 4 to 6-membered saturated heterocyclic ring which contains, as ring-forming component(s), one or two oxygen atoms;

In the formula (I), compounds wherein Y is a 4 to 6-membered saturated heterocyclic ring which contains, as ring-forming component(s), one oxygen atom;

In the formula (I), compounds wherein Y is a 4 to 6-membered saturated heterocyclic ring which contains, as ring-forming component(s), two oxygen atoms;

In the formula (I), compounds wherein Y is a saturated 4 to 6-membered ring which contains, as ring-forming companent(s), one oxygen atom or one -S(O)ₜ-;

In the formula (I), compounds wherein Y is a 4 to 6-membered saturated heterocyclic ring which contains, as ring-forming component(s), one oxygen atom or one -S(O)ₜ-, and t is 0; In the formula (I), compounds wherein Y is a group represented by formula (II-a),

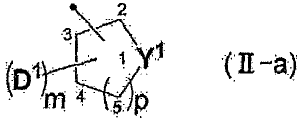

Y¹ is an oxygen atom or a sulfur atom, m is 0 or 1, and p is 0, 1 or 2, more specifically, compounds represented by formula (I-A);

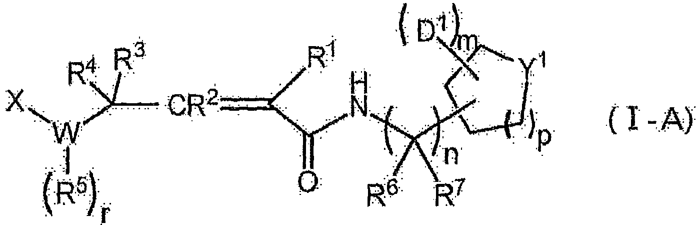

In the formula (I-A), compounds wherein Y¹ is an oxygen atom or a sulfur atom, m is 0 or 1, p is 0, 1 or 2, and D¹ is an ethyl group or a methyl group;

In the formula (I-A), compounds wherein Y¹ is an oxygen atom or a sulfur atom, m is 0 or 1, p is 0, 1 or 2, D¹ is an ethyl group or a methyl group, and n is 1;

In the formula (IV), compounds wherein Y is a group represented by formula (II-a), Y¹ is an oxygen atom or a sulfur atom, m is 0 or l, and p is 0, 1 or 2, more specifically, compounds represented by formula (IV-A);

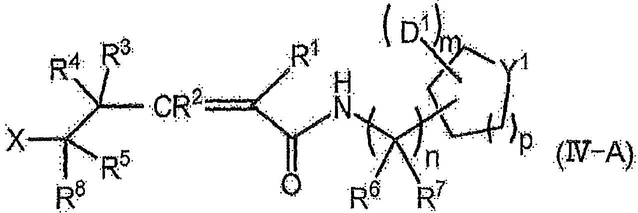

In the formula (IV-A), compounds wherein D¹ is an ethyl group or a methyl group;

In the formula (IV-A), compounds wherein D¹ is an ethyl group or a methyl group, and n is 1;

In the formula (IV-A), compounds wherein D¹ is an ethyl group or a methyl group, n is 1, and R⁶ and R⁷ are the same or different and are a methyl group or a hydrogen atom;

In the formula (IV-A), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, and R³ and R⁴ are a hydrogen atom;

In the formula (IV-A), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁹ are a hydrogen atom, R¹ is a hydrogen atom or a fluorine atom, and R² is a methyl group or a hydrogen atom;

In the formula (IV-A), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁴ are a hydrogen atom, R¹ is a hydrogen atom or a fluorine atom, R² is a methyl group or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (IV-A-1));

In the formula (IV-A-1), compounds wherein the relative arrangement of R¹ and R² is a trans configuration;

In the formula (IV-A-1), compounds that are rich in compounds wherein the relative arrangement of R¹ and R² is a trans configuration;

In the formula (IV-A-1), compounds that contain 80% or more of compounds wherein the relative arrangement of R¹ and R² is a trans configuration;

In the formula (IV-A-1), compounds that contain 90% or more of compounds wherein the relative arrangement of R¹ and R² is a trans configuration;

In the formula (V), compounds wherein Y is a group represented by formula (II-a), Y¹ is an oxygen atom or a sulfur atom, m is 0 or 1, and p is 0, 1 or 2, more specifically, compounds represented by formula (V-A);

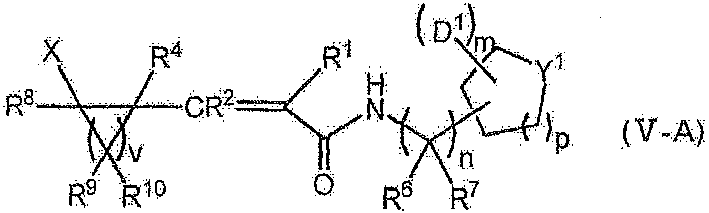

In the formula (V-A), compounds wherein D¹ is an ethyl group or a methyl group;

In the formula (V-A), compounds wherein D¹ is an ethyl group or a methyl group, and n is 1;

In the formula (V-A), compounds wherein D¹ is an ethyl group or a methyl group, n is 1, and R⁶ and R⁷ are the same or different and are a methyl group or a hydrogen atom;

In the formula (V-A), compounds wherein D¹ is an ethyl group or a methyl group, n is 1, R⁶ and R⁷ are the same or different and are a methyl group or a hydrogen atom, R⁴ is a C1 to C4 alkyl group optionally having one or more halogen atoms, a hydrogen atom or a halogen atom, R⁸ is a C1 to C4 alkyl group optionally having one or more halogen atoms, a hydrogen atom or a halogen atom, and R⁹ and R¹⁰ are the same or different and are a C1 to C4 alkyl group optionally having one or more halogen atoms, a hydrogen atom or a halogen atom;

In the formula (V-A), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, and R⁹ and R¹⁰ are the same or different and are a methyl group or a hydrogen atom;

In the formula (V-A), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom;

In the formula (V-A), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, R⁹ and R¹⁰ are the same or different and are a methyl group or a hydrogen atom, R¹ is a hydrogen atom or a fluorine atom, and R² is a methyl atom or a hydrogen atom;

In the formula (V-A), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom, R¹ is a hydrogen atom or a fluorine atom, R² is a methyl atom or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (V-A-1));

In the formula (V-A-1), compounds wherein the relative arrangement of R¹ and R² is a trans configuration;

In the formula (V-A-1), compounds that are rich in compounds wherein the relative arrangement of R¹ and R² is a trans configuration;

In the formula (V-A-1), compounds that contain 80% or more of compounds wherein the relative arrangement of R¹ and R² is a trans configuration;

In the formula (V-A-1), compounds that contain 90% or more of compounds wherein the relative arrangement of R¹ and R² is a trans configuration;

In the formula (VI), compounds wherein Y is a group represented by formula (II-a), Y¹ is an oxygen atom or a sulfur atom, m is 0 or 1, and p is 0, 1, or 2, more specifically, compounds represented by formula (VI-A);

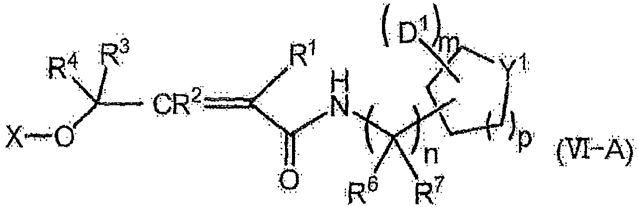

In the formula (VI-A), compounds wherein D¹ is an ethyl group or a methyl group;

In the formula (VI-A), compounds wherein D¹ is an ethyl group or a methyl group, and n is 1;

In the formula (VI-A), compounds wherein D¹ is an ethyl group or a methyl group, n is 1, and R⁶ and R⁷ are the same or different and are a methyl group or a hydrogen atom;

In the formula (VI-A), compounds wherein D¹ is an ethyl group or a methyl group, n is 1, R⁶ and R⁷ are the same or different and are a methyl group or a hydrogen atom, and R³ and R⁴ are a hydrogen atom;

In the formula (IV-A), compounds wherein D¹ is an ethyl group or a methyl group, n is 1, R⁶ and R⁷ are the same or different and are a methyl group or a hydrogen atom, R³ and R⁴ are a hydrogen atom, R¹ is a hydrogen atom or a fluorine atom, and R² is a methyl atom or a hydrogen atom;

In the formula (VI-A), compounds wherein D¹ is an ethyl group or a methyl group, n is 1, R⁶ and R⁷ are the same or different and are a methyl group or a hydrogen atom, R³ and R⁴ are a hydrogen atom, R¹ is a hydrogen atom or a fluorine atom, R² is a methyl atom or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (VI-A-1));

In the formula (VI-A-1), compounds wherein the relative arrangement of R¹ and R² is a trans configuration;

In the formula (VI-A-1), compounds that are rich in compounds wherein the relative arrangement of R¹ and R² is a trans configuration;

In the formula (VI-A-1), compounds that contain 80% or more of compounds wherein the relative arrangement of R¹ and R² is a trans configuration;

In the formula (VI-A-1), compounds that contain 90% or more of compounds wherein the relative arrangement of R¹ and R² is a trans configuration;

In the formula (I-A), compounds wherein Y¹ is an oxygen atom, and p is 1 or 2, more specifically, compounds represented by formula (I-B);

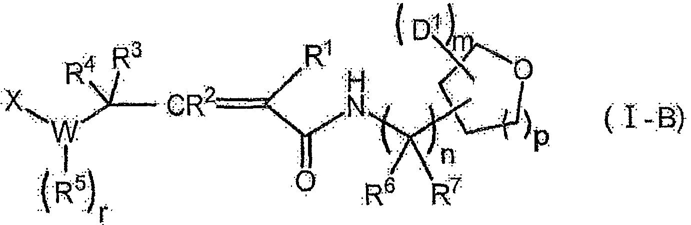

In the formula (I-B), compounds wherein the relative arrangement of R¹ and R² is a trans configuration;

In the formula (I-B), compounds wherein m is 0, X is a C1 to C5 alkyl group having one phenyl group, W is -CR⁸- or an oxygen atom, and when W is -CR⁸-, r is 1, and when W is an oxygen atom, r is 0, R⁸ is a hydrogen atom, R¹ is a hydrogen atom, R² is a C1 to C4 alkyl group optionally having one or more halogen atoms or a hydrogen atom, R³, R⁴ and R⁵ are a hydrogen atom, R⁶ and R⁷ are a hydrogen atom, and n is 1;

In the formula (I-B), compounds wherein m is 0, X is a C1 to C5 alkyl group having one 2-pyridyl group, W is -CR⁸- or an oxygen atom, and when W is -CR⁸-, r is 1, and when W is an oxygen atom, r is 0, R⁸ is a hydrogen atom, R¹ is a hydrogen atom, R² is a C1 to C4 alkyl group optionally having one or more halogen atoms or a hydrogen atom, R³, R⁴ and R⁵ are a hydrogen atom, R⁶ and R⁷ are a hydrogen atom, and n is 1;

In the formula (I-B), compounds wherein m is 0, X is a C1 to C5 alkyl group having one 2-naphthyl group, W is -CR⁸- or an oxygen atom, and when W is -CR⁸-, r is 1, and when W is an oxygen atom, r is 0, R⁸ is a hydrogen atom, R¹ is a hydrogen atom, R² is a C1 to C4 alkyl group optionally having one or more halogen atoms or a hydrogen atom, R³, R⁴ and R⁵ are a hydrogen atom, R⁶ and R⁷ are a hydrogen atom, and n is 1;

In the formula (IV-A), compounds wherein Y¹ is an oxygen atom, and p is 1 or 2, more specifically, compounds represented by formula (IV-B);

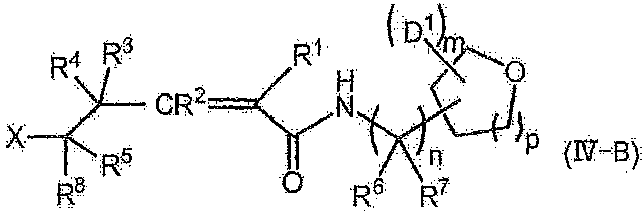

In the formula (IV-B), compounds wherein D¹ is an ethyl group or a methyl group;

In the formula (IV-B), compounds wherein D¹ is an ethyl group or a methyl group, and n is 1;

In the formula (IV-B), compounds wherein D¹ is an ethyl group or a methyl group, n is 1, and R⁶ and R⁷ are the same or different and are a methyl group or a hydrogen atom;

In the formula (IV-B), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, and R³ and R⁴ are a hydrogen atom;

In the formula (IV-B), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁴ are a hydrogen atom, R¹ is a hydrogen atom or a fluorine atom, and R² is a methyl group or a hydrogen atom;

In the formula (IV-B), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁴ are a hydrogen atom, R¹ is a hydrogen atom or a fluorine atom, R² is a methyl group or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (IV-B-1));

In the formula (IV-B-1), compounds wherein the relative arrangement of R¹ and R² is a trans configuration;

In the formula (IV-B-1), compounds that are rich in compounds wherein the relative arrangement of R¹ and R² is a trans configuration;

In the formula (IV-B-1), compounds that contain 80% or more of compounds wherein the relative arrangement of R¹ and R² is a trans configuration;

In the formula (IV-B-1), compounds that contain 90% or more of compounds wherein the relative arrangement of R¹ and R² is a trans configuration;

In the formula (V-A), compounds wherein Y¹ is an oxygen atom, m is 0 or 1, and p is 1 or 2, more specifically, compounds represented by formula (V-B);

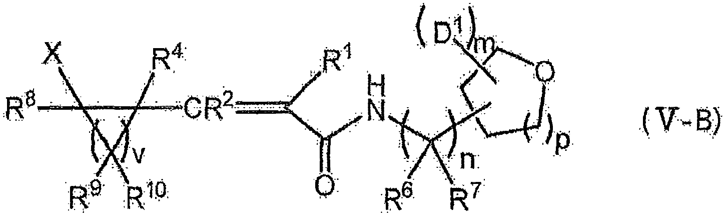

In the formula (V-B), compounds wherein D¹ is an ethyl group or a methyl group;

In the formula (V-B), compounds wherein D¹ is an ethyl group or a methyl group, and n is 1;

In the formula (V-B), compounds wherein D¹ is an ethyl group or a methyl group, n is 1, and R⁶ and R⁷ are the same or different and are a methyl group or a hydrogen atom;

In the formula (V-B), compounds wherein D¹ is an ethyl group or a methyl group, n is 1, R⁶ and R⁷ are the same or different and are a methyl group or a hydrogen atom, R⁴ is a C1 to C4 alkyl group optionally having one or more halogen atoms, a hydrogen atom or a halogen atom, R⁸ is a C1 to C4 alkyl group optionally having one or more halogen atoms, a hydrogen atom or a halogen atom, and R⁹ and R¹⁰ are the same or different and are a C1 to C4 alkyl group optionally having one or more halogen atoms, a hydrogen atom or a halogen atom;

In the formula (V-B), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, and R⁹ and R¹⁰ are the same or different and are a methyl group or a hydrogen atom;

In the formula (V-B), compounds wherein D¹ is an ethyl group or a methyl group, n is 1, R⁶ and R⁷ are the same or different, and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom;

In the formula (V-B), compounds wherein D¹ is an ethyl group or a methyl group, n is 1, R⁶ and R⁷ are the same or different, and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, R⁹ and R¹⁰ are the same or different and are a methyl group or a hydrogen atom, R¹ is a hydrogen atom or a fluorine atom, and R² is a methyl atom or a hydrogen atom;

In the formula (V-B), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom, R¹ is a hydrogen atom or a fluorine atom, R² is a methyl atom or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (V-B-1));

In the formula (V-B-1), compounds wherein the relative arrangement of R¹ and R² is a trans configuration;

In the formula (V-B-1), compounds that are rich in compounds wherein the relative arrangement of R¹ and R² is a trans configuration;

In the formula (V-B-1), compounds that contain 80% or more of compounds wherein the relative arrangement of R¹ and R² is a trans configuration;

In the formula (V-B-1), compounds that contain 90% or more of compounds wherein the relative arrangement of R¹ and R² is a trans configuration;

In the formula (VI-A), compounds wherein Y¹ is an oxygen atom, m is 0 or 1, and p is 1 or 2, more specifically, compounds represented by formula (VI-B);

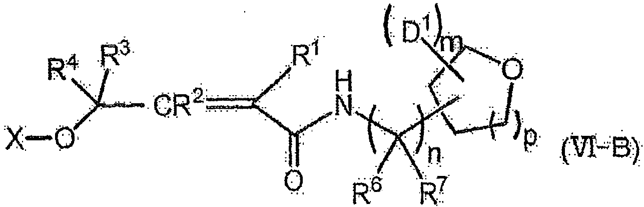

In the formula (VI-B), compounds wherein D¹ is an ethyl group or a methyl group;

In the formula (VI-B), compounds wherein D¹ is an ethyl group or a methyl group, and n is 1;

In the formula (VI-B), compounds wherein D¹ is an ethyl group or a methyl group, n is 1, and R⁶ and R⁷ are the same or different and are a methyl group or a hydrogen atom;

In the formula (VI-B), compounds wherein D¹ is an ethyl group or a methyl group, n is 1, R⁶ and R⁷ are the same or different and are a methyl group or a hydrogen atom, and R³ and R⁴ are a hydrogen atom;

In the formula (VI-B), compounds wherein D¹ is an ethyl group or a methyl group, n is 1, R⁶ and R⁷ are the same or different, and are a methyl group or a hydrogen atom, R³ and R⁴ are a hydrogen atom, R¹ is a hydrogen atom or a fluorine atom, and R² is a methyl group or a hydrogen atom;

In the formula (VI-B), compounds wherein D¹ is an ethyl group or a methyl group, n is 1, R⁶ and R⁷ are the same or different and are a methyl group or a hydrogen atom, R³ and R⁴ are a hydrogen atom, R¹ is a hydrogen atom or a fluorine atom, R² is a methyl group or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (VI-B-1));

In the formula (VI-B-1), compounds wherein the relative arrangement of R¹ and R² is a trans configuration;

In the formula (VI-B-1), compounds that are rich in compounds wherein the relative arrangement of R¹ and R² is a trans configuration;

In the formula (VI-B-1), compounds that contain 80% or more of compounds wherein the relative arrangement of R¹ and R² is a trans configuration;

In the formula (VI-B-1), compounds that contain 90% or more of compounds wherein the relative arrangement of R¹ and R² is a trans configuration;

In the formula (I), compounds wherein Y is a group represented by formula (II-b), 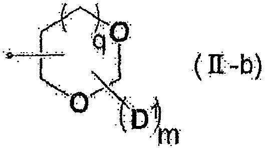 m is 0 or 1, and q is 0 or 1, more specifically, compounds represented by formula (I-C);

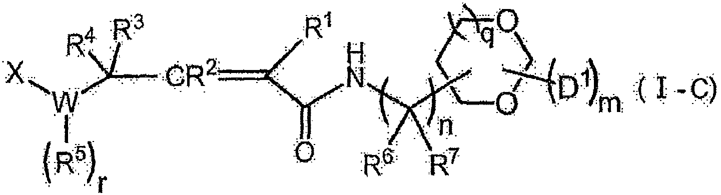

In the formula (I-C), compounds wherein m is 0 or 1, q is 0 or 1, and D¹ is an ethyl group or a methyl group;

In the formula (I-C), compounds wherein m is 0 or 1, q is 0 or 1, D¹ is an ethyl group or a methyl group, and n is 1;

In the formula (IV), compounds wherein Y is a group represented by the formula (II-b), m is 0 or 1, and q is 0 or 1, more specifically, compounds represented by formula (IV-C);

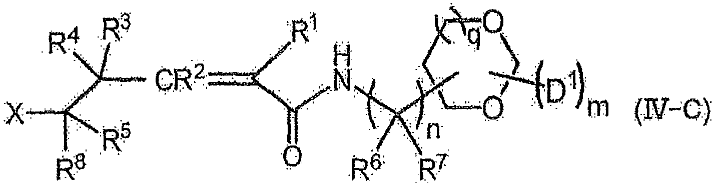

In the formula (IV-C), compounds wherein D¹ is an ethyl group or a methyl group;

In the formula (IV-C), compounds wherein D¹ is an ethyl group or a methyl group, and n is 1;

In the formula (IV-C), compounds wherein D¹ is an ethyl group or a methyl group, n is 1, and R⁶ and R⁷ are the same or different and are a methyl group or a hydrogen atom;

In the formula (IV-C), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, and R³ and R⁴ are a hydrogen atom;

In the formula (IV-C), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁴ are a hydrogen atom, R¹ is a hydrogen atom or a fluorine atom, and R² is a methyl group or a hydrogen atom;

In the formula (IV-C), compounds wherein D¹ is an ethyl group or a methyl group, n is 1, R⁶ and R⁷ are the same or different and are a methyl group or a hydrogen atom, R⁸ is a methyl group or a hydrogen atom, R⁵ is a methyl group or a hydrogen atom, R³ and R⁴ are a hydrogen atom, R¹ is a hydrogen atom or a fluorine atom, R² is a methyl group or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (IV-C-1));

In the formula (IV-C-1), compounds wherein the relative arrangement of R¹ and R² is a trans configuration;

In the formula (IV-C-1), compounds that are rich in compounds wherein the relative arrangement of R¹ and R² is a trans configuration;

In the formula (IV-C-1), compounds that contain 80% or more of compounds wherein the relative arrangement of R¹ and R² is a trans configuration;

In the formula (IV-C-1), compounds that contain 90% or more of compounds wherein the relative arrangement of R¹ and R² is a trans configuration;

In the formula (V), compounds wherein Y is a group represented by the formula (II-b), m is 0 or 1, and q is 0 or 1, more specifically, compounds represented by formula (V-C);

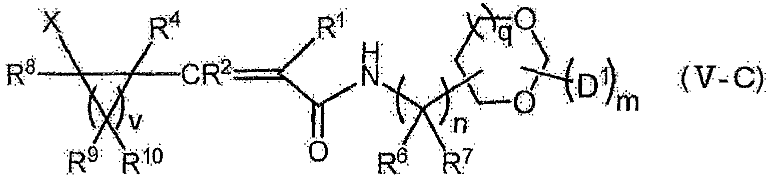

In the formula (V-C), compounds wherein D¹ is an ethyl group or a methyl group;

In the formula (V-C), compounds wherein D¹ is an ethyl group or a methyl group, and n is 1;

In the formula (V-C), compounds wherein D¹ is an ethyl group or a methyl group, n is 1, and R⁶ and R⁷ are the same or different and are a methyl group or a hydrogen atom;

In the formula (V-C), compounds wherein D¹ is an ethyl group or a methyl group, n is 1, R⁶ and R⁷ are the same or different and are a methyl group or a hydrogen atom, R⁴ is a C1 to C4 alkyl group optionally having one or more halogen atoms, a hydrogen atom or a halogen atom, R⁸ is a C1 to C4 alkyl group optionally having one or more halogen atoms, a hydrogen atom or a halogen atom, and R⁹ and R¹⁰ are the same or different and are a C1 to C4 alkyl group optionally having one or more halogen atoms, a halogen atom or a hydrogen atom;

In the formula (V-C), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, and R⁹ and R¹⁰ are the same or different and are a methyl group or a hydrogen atom;

In the formula (V-C), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom;

In the formula (V-C), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, R⁹ and R¹⁰ are the same or different and are a methyl group or a hydrogen atom, R¹ is a hydrogen atom or a fluorine atom, and R² is a methyl atom or a hydrogen atom;

In the formula (V-C), compounds wherein D¹ is an ethyl group or a methyl group, n is 1, R⁶ and R⁷ are the same or different and are a methyl group or a hydrogen atom, v is 1, R⁴ is a methyl group, a hydrogen atom or a fluorine atom, R⁸ is a methyl group or a hydrogen atom, the relative arrangement of R⁴ and R⁸ is trans, and R⁹ and R¹⁰ are the same or different and are a methyl group or a hydrogen atom, R¹ is a hydrogen atom or a fluorine atom, R² is a methyl atom or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (V-C-1));

In the formula (V-C-1), compounds wherein the relative arrangement of R¹ and R² is a trans configuration; In the formula (V-C-1), compounds that are rich in compounds wherein the relative arrangement of R¹ and R² is a trans configuration;

In the formula (V-C-1), compounds that contain 80% or more of compounds wherein the relative arrangement of R¹ and R² is a trans configuration;

In the formula (V-C-1), compounds that contain 90% or more of compounds wherein the relative arrangement of R¹ and R² is a trans configuration;

In the formula (VI), compounds wherein Y is a group represented by the formula (II-b), m is 0 or 1, and q is 0 or 1, more specifically, compounds represented by formula (VI-C);

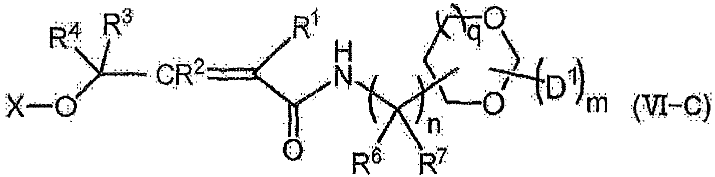

In the formula (VI-C), compounds wherein D¹ is an ethyl group or a methyl group;

In the formula (VI-C), compounds wherein D¹ is an ethyl group or a methyl group, and n is 1;

In the formula (VI-C), compounds wherein D¹ is an ethyl group or a methyl group, n is 1, and R⁶ and R⁷ are the same or different and are a methyl group or a hydrogen atom;

In the formula (VI-C), compounds wherein D¹ is an ethyl group or a methyl group, n is 1, R⁶ and R⁷ are the same or different and are a methyl group or a hydrogen atom, and R³ and R⁴ are a hydrogen atom;

In the formula (VI-C), compounds wherein D¹ is an ethyl group or a methyl group, n is 1, R⁶ and R⁷ are the same or different and are a methyl group or a hydrogen atom, R³ and R⁴ are a hydrogen atom, R¹ is a hydrogen atom or a fluorine atom, and R² is a methyl group or a hydrogen atom;

In the formula (VI-C), compounds wherein D¹ is an ethyl group or a methyl group, n is 1, R⁶ and R⁷ are the same or different and are a methyl group or a hydrogen atom, R³ and R⁴ are a hydrogen atom, R¹ is a hydrogen atom or a fluorine atom, R² is a methyl group or a hydrogen atom, X is a C1 to C5 alkyl group having one group selected from group A, the group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, any of the group in group A may have one or more atoms or groups selected from group B, and the one or more atoms or groups selected from group B is a methyl group, a trifluoromethyl group, a methoxy group, a methylthio group, a cyano group, a nitro group, a hydroxyl group, a fluorine group or a chlorine atom (hereinafter represented as compound (VI-C-1));

In the formula (VI-C-1), compounds wherein the relative arrangement of R¹ and R² is a trans configuration; In the formula (VI-C-1), compounds that are rich in compounds wherein the relative arrangement of R¹ and R² is a trans configuration;

In the formula (VI-C-1), compounds that contain 80% or more of compounds wherein the relative arrangement of R¹ and R² is a trans configuration;

In the formula (VI-C-1), compounds that contain 90% or more of compounds wherein the relative arrangement of R¹ and R² is a trans configuration;

Next, the method for producing the compound of the present invention will be described.

The compound of the present invention can be produced, for example, according to the following (Production Method 1) to (Production Method 2).

### (Production Method 1)

The compound of the present invention can be produced by reacting a compound represented by formula (4) with a compound represented by formula (6), in the presence of a condensing agent. 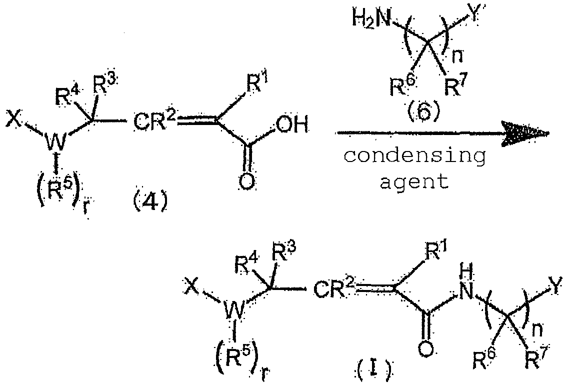 wherein W, X, Y, R¹, R², R³, R⁴, R⁵, R⁶, R⁷, n and r represent the same meaning as above.

The reaction is usually carried out in a solvent, in the presence of a condensing agent, and in the presence of a base as necessary.

Examples of the condensing agent include dicyclohexylcarbodiimide, 1-ethyl-3-(3-dimethylaminopropyl) carbodiimide hydrochloride, and benzotriazol-1-yloxy-trisdimethylamino phosphonium salt.

Examples of the base include carbonates such as sodium carbonate and potassium carbonate, tertiary amines such as triethylamine, diisopropylethylamine, 1,8-diazabicyclo[5.4.0]undec-7-ene and 1,5-diazabicyclo[4.3.0]non-5-ene, and nitrogen containing aromatic compounds such as pyridine and 4-dimethylaminopyridine.

Examples of the solvent include aromatic hydrocarbons such as benzene and toluene, aliphatic hydrocarbons such as hexane, ethers such as diethyl ether, tetrahydrofuran, 1,4-dioxane and tert-butyl methyl ether, halogenated hydrocarbons such as dichloromethane, chloroform, 1,2-dichloroethane and chlorobenzene and acid amides such as N,N-dimethylformamide, and mixtures thereof.

The reaction time is usually within the range of 5 minutes to 72 hours.

The reaction temperature is usually within the range of -20 to 100°C.

The molar ratio of the compound represented by the formula (4) and the compound represented by the formula (6) used can be arbitrarily set, and is preferably an equimolar ratio or a ratio close thereto, for example, the ratio of the compound represented by the formula (6) is 0.5 to 3 mol, based on 1 mol of the compound represented by the formula (4).

The amount of the condensing agent used is arbitrary, and is usually 1 mol to an excess amount, and preferably a ratio of 1 to 3 mol, based on 1 mol of the compound represented by the formula (4).

The amount of the base is usually 1 mol to an excess amount, and preferably a ratio of 1 to 3 mol, based on 1 mol of the compound represented by the formula (4).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

### (Production Method 2)

The compound of the present invention can be produced by reacting a compound represented by formula (5) with a compound represented by formula (6), in the presence of a base. 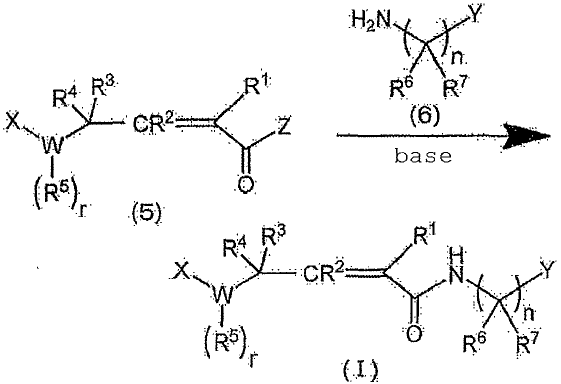 wherein Z represents a chlorine atom, a bromine atom or an iodine atom, and W, X, Y, R¹, R², R³, R⁴, R⁵, R⁶, R⁷, r and n represent the same meaning as above.

The reaction is usually carried out in a solvent, in the presence of a base.

Examples of the base include carbonates such as sodium carbonate and potassium carbonate, tertiary amines such as triethylamine, diisopropylethylamine, 1,8-diazabicyclo[5.4.0]undec-7-ene and 1,5-diazabicyclo[4.3.0]non-5-ene, and nitrogen containing aromatic compounds such as pyridine and 4-dimethylaminopyridine.

Examples of the solvent include ethers such as 1,4-dioxane, tetrahydrofuran, ethylene glycol dimethyl ether and tert-butyl methyl ether, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile and butyronitrile, acid amides such as N,N-dimethylformamide, sulfoxides such as dimethyl sulfoxide, and mixtures thereof.

The reaction time is usually within the range of 5 minutes to 72 hours.

The reaction temperature is usually within the range of -20 to 100°C.

The molar ratio of the compound represented by the formula (5) and the compound represented by the formula (6) used can be arbitrarily set, and is preferably an equimolar ratio or a ratio close thereto, specifically, the ratio of the compound represented by the formula (6) is 0.5 to 3 mol, based on 1 mol of the compound represented by the formula (5).

The amount of the base is arbitrary, but is usually 1 mol to an excess amount, and preferably a ratio of 1 to 3 mol, based on 1 mol of the compound represented by the formula (5).

After completion of the reaction, the compound of the present invention can be isolated by adding the reaction mixture to water and then subjecting it to usual post-treatment operations such as an extraction with an organic solvent and concentration. In addition, the isolated compound of the present invention can be also purified by procedures such as chromatography, recrystallization and distillation.

Next, the method for producing a production intermediate of the compound of the present invention will be described.

### (Reference Production Method 1)

The compound represented by the formula (5) can be produced, for example, by reacting a compound represented by formula (4) with a halogenating agent. 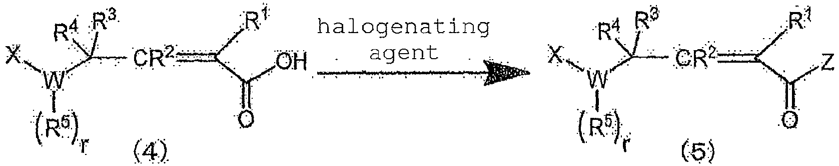 wherein W, X, Z, R¹, R², R³, R⁴, R⁵ and r represent the same meaning as above.

The reaction is carried out in a solvent as necessary.

Examples of the halogenating agent include thionyl chloride, oxalyl chloride and phosphorus oxychloride.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as toluene and xylene, halogenated hydrocarbons such as chlorobenzene, and mixtures thereof.

The reaction time is usually within the range of 5 minutes to 24 hours.

The reaction temperature is usually within the range of 0 to 100°C.

The amount of the halogenating agent is usually 1 mol to an excess amount, and preferably a ratio of 1 to 5 mol, based on 1 mol of the compound represented by the formula (4).

After completion of the reaction, the compound represented by formula (5) can be isolated by a post-treatment operation such as concentrating the reaction mixture as it is. The isolated compound represented by formula (5) is usually used in the following step without purification, but can be also purified by distillation or the like, as necessary.

### (Reference Production Method 2)

The compound represented by the formula (4) can be produced, for example, by subjecting a compound represented by formula (3) to a hydrolysis reaction, in the presence of a base. 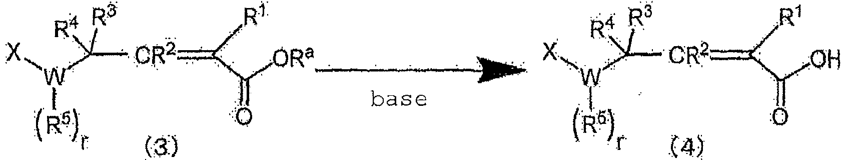 wherein R^{a} represents a methyl group or an ethyl group, and W, X, R¹, R², R³, R⁴, R⁵ and r represent the same meaning as above.

The reaction is carried out in the presence of a base, and in the presence of water and an organic solvent.

Examples of the base include alkali metal hydroxides such as lithium hydroxide, sodium hydroxide and potassium hydroxide.

Examples of the solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, and mixtures thereof.

The reaction time is usually within the range of 5 minutes to 72 hours.

The reaction temperature is usually within the range of 0 to 100°C.

The amount of the base is arbitrary, but is usually 1 mol to an excess amount, and preferably a ratio of 1 to 5 mol, based on 1 mol of the compound represented by the formula (3).

After completion of the reaction, the compound represented by formula (4) can be isolated by post-treatment operations such as adding an acidic aqueous solution (hydrochloric acid, etc.) to the reaction mixture to extract with an organic solvent, and drying and concentrating the organic layer. The isolated compound represented by formula (4) can be also further purified by chromatography, recrystallization and distillation, and can be used in the following step as it is.

### (Reference Production Method 3)

The compound represented by the formula (3) can be produced, for example, by reacting a compound represented by formula (1) with a compound represented by formula (2-a) or a compound represented by formula (2-b), in the presence of a base. 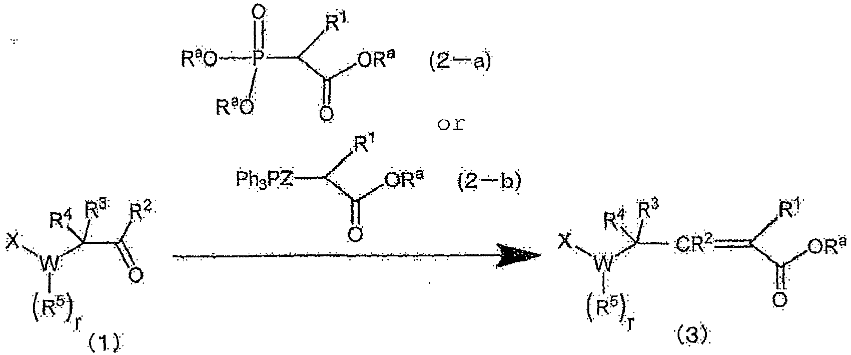 wherein W, X, Z, R^{a}, R¹, R², R³, R⁴, R⁵ and r represent the same meaning as above.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as benzene, toluene and xylene, halogenated hydrocarbons such as chlorobenzene, and mixtures thereof.

The reaction time is usually within the range of 5 minutes to 72 hours.

The reaction temperature is usually within the range of -80 to 100°C.

The molar ratio of the compound represented by the formula (1) and the compound represented by the formula (2-a) or the compound represented by the formula (2-b) used can be arbitrarily set, and is preferably an equimolar ratio or a ratio close thereto. Specifically, the ratio of the compound represented by the formula (2-a) or the compound represented by the formula (2-b) is preferably 0.5 to 3 mol, based on 1 mol of the compound represented by the formula (1).

The amount of the base is arbitrary, but is usually 1 mol to an excess amount, and preferably a ratio of 1 to 5 mol, based on 1 mol of the compound represented by the formula (2-a) or the compound represented by the formula (2-b).

After completion of the reaction, the compound represented by the formula (3) can be obtained by adding the reaction mixture to water and then subjecting it to usual post-treatment operations such as an extraction with an organic solvent and concentration. In addition, the obtained compound represented by the formula (3) can be further purified by procedures such as chromatography, recrystallization and distillation.

### (Reference Production Method 4)

The compound represented by the formula (1) can be produced, for example, by subjecting a compound represented by formula (7) to an oxidation reaction. 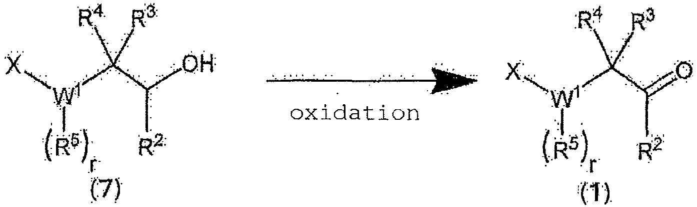 wherein W¹ represents -CR⁸- or an oxygen atom, and X, R², R³, R⁴, R⁵ and r represent the same meaning as above.

Examples of the oxidation reaction include;
(1) a method of using a chromate (for example, pyridinium chlorochromate or pyridinium dichromate),
(2) a method of using a chromic acid (for example, Jones oxidation),
(3) a method of using DMSO oxidation (for example, Swern oxidation),
(4) a method of using a Dess-Martin reagent,
(5) a method of using TEMPO (2,2,6,6-tetramethylpiperidine 1-oxyl),
   and the like.
   (1) The method of using a chromate is specifically exemplified as an example.

The reaction is usually carried out in a solvent.

Examples of the oxidizing agent include chromates such as pyridinium chlorochromate and pyridinium dichromate.

Examples of the solvent include halogenated hydrocarbons such as dichloromethane, chloroform, 1,2-dichloroethane and chlorobenzene, aliphatic hydrocarbons such as hexane and heptane, aromatic hydrocarbons such as toluene and xylene, and mixtures thereof.

The reaction time is usually within the range of 5 minutes to 24 hours.

The reaction temperature is usually within the range of 0 to 100°C.

The amount of the chromate is arbitrary, but is usually 1 mol to an excess amount, and preferably a ratio of 1 to 3 mol, based on 1 mol of the compound represented by the formula (7).

After completion of the reaction, the compound represented by the formula (1) can be isolated by filtering the reaction mixture and then subjecting it to an operation such as concentration. The isolated compound represented by the formula (1) can be further also purified by chromatography, recrystallization, distillation and the like, as necessary.

### (Reference Production Method 5)

The compound represented by the formula (1) can be also produced, for example, by reacting a compound represented by formula (8), in the presence of water and an acid. 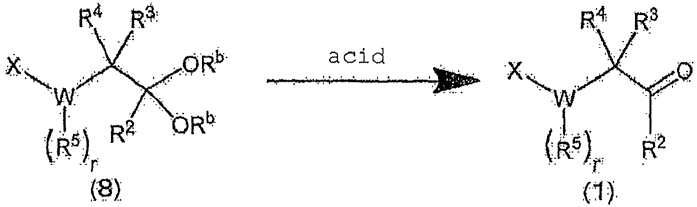 wherein R^{b} represents a methyl group or an ethyl group, and W, X, R², R³, R⁴, R⁵ and r represent the same meaning as above.

The reaction is carried out in the presence of an acid, and in the presence of water and an organic solvent.

Examples of the acid include inorganic acids such as hydrochloric acid and sulfuric acid and organic acids such as methanesulfonic acid and p-toluenesulfonic acid.

Examples of the organic solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, ketones such as acetone and methyl isobutyl ketone, and mixtures thereof.

The reaction time is usually within the range of 5 minutes to 24 hours.

The reaction temperature is usually within the range of 0 to 100°C.

The amount of the acid is arbitrary, and is usually 1 mol to an excess amount, based on 1 mol of the compound represented by the formula (8).

After completion of the reaction, the compound represented by the formula (1) can be isolated by adding the reaction mixture to water and then subjecting it to usual post-treatment operations such as an extraction with an organic solvent and concentration. In addition, the isolated compound represented by the formula (1) can be also purified by procedures such as chromatography, recrystallization and distillation.

### (Reference Production Method 6)

Among the compounds represented by the formula (8), a compound represented by formula (8-1) wherein W is an oxygen atom or a sulfur atom and r is 0 can be also produced, for example, by reacting a compound represented by formula (9) with a compound represented by formula (10), in the presence of a base. 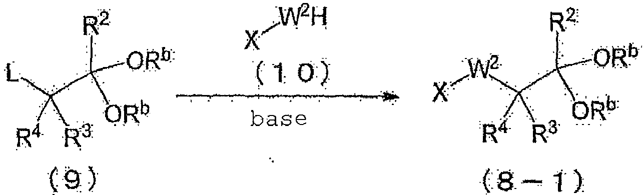 wherein W² represents an oxygen atom or a sulfur atom, L represents a leaving group such as a chlorine atom, a bromine atom, an iodine atom or a methanesulfonyl group, and X, R^{b}, R², R³ and R⁴ represent the same meaning as above.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C.

The molar ratio of the compound represented by the formula (9) and the compound represented by the formula (10) used can be arbitrarily set, and is preferably an equimolar ratio or a ratio close thereto, for example, the ratio of the compound represented by the formula (10) is 1 to 3 mol, based on 1 mol of the compound represented by the formula (9).

The amount of the base is arbitrary, but is usually 1 mol to an excess amount, and preferably 1 to 3 mol, based on 1 mol of the compound represented by the formula (10).

After completion of the reaction, the compound represented by the formula (8-1) can be isolated by adding the reaction mixture to water and then subjecting it to usual post-treatment operations such as an extraction with an organic solvent and concentration. In addition, the isolated compound represented by the formula (8-1) can be also purified by procedures such as chromatography, recrystallization and distillation.

### (Reference Production Method 7)

Among the compounds represented by the formula (8), a compound represented by formula (8-so) wherein W is -S(O)ᵤ-, r is 0, and u is 1 or 2 can be produced, for example, by subj ecting to an oxidation reaction a compound represented by formula (8-s) wherein W² is a sulfur atom in the formula (8-1). 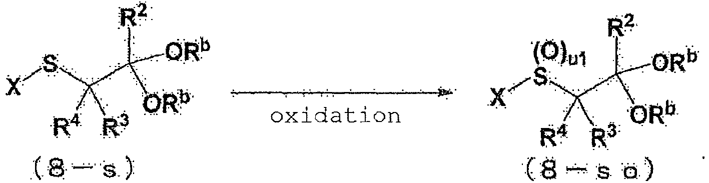 wherein u1 represents 1 or 2, and X, R^{b}, R², R³ and R⁴ represent the same meaning as above.

The reaction is usually carried out in a solvent.

Examples of the oxidizing agent include organic peroxides such as peracetic acid, trifluoroperacetic acid and m-chloroperbenzoic acid, halogen molecules of chlorine and bromine, halogen-containing imides such as N-chlorosuccinimide, halides such as perchloric acid or salts thereof, periodic acid or salts thereof, permanganates such as potassium permanganate, chromates such as potassium chromates, peroxysulfates such as peroxy-potassium sulfate, and hydrogen peroxide.

Examples of the solvent include alcohols such as methanol and ethanol, halogenated hydrocarbons such as dichloromethane and chloroform, aromatic hydrocarbons such as toluene and xylene, aliphatic carbonates such as acetic acid and trifluoroacetic acid, water, and mixtures thereof.

The reaction time is usually within the range of 5 minutes to 24 hours.

The reaction temperature is usually within the range of -80 to 100°C.

The molar ratio of the compound represented by the formula (8-s) and the oxidizing agent used can be arbitrarily set, and is preferably an equimolar ratio or a ratio close thereto, for example, the ratio of the oxidizing agent is 0.5 to 3 mol, based on 1 mol of the compound represented by the formula (8-s).

After completion of the reaction, the compound represented by the formula (8-so) can be isolated by adding the reaction mixture to water and then subjecting it to usual post-treatment operations such as an extraction with an organic solvent and concentration. In addition, the isolated compound represented by the formula (8-so) can be also purified by procedures such as chromatography, recrystallization and distillation.

### (Reference Production Method 8)

Among the compounds represented by the formula (7), a compound represented by formula (7-1) wherein W is an oxygen atom or a sulfur atom and r is 0, can be produced, for example, by reacting a compound represented by formula (11) with a compound represented by formula (10), in the presence of a base. 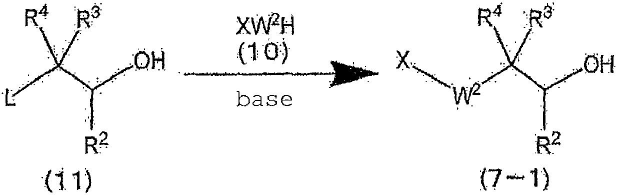 wherein W², L, X, R², R³ and R⁴ represent the same meaning as above.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C.

The molar ratio of the compound represented by the formula (11) and the compound represented by the formula (10) used can be arbitrarily set, and is preferably an equimolar ratio or a ratio close thereto, for example, the ratio of the compound represented by the formula (10) is 1 to 3 mol, based on 1 mol of the compound represented by the formula (11).

The amount of the base is arbitrary, but is usually 1 mol to an excess amount, and preferably 1 to 3 mol, based on 1 mol of the compound represented by the formula (10).

After completion of the reaction, the compound represented by the formula (7-1) can be isolated by adding the reaction mixture to water and then subjecting it to usual post-treatment operations such as an extraction with an organic solvent and concentration. In addition, the isolated compound represented by the formula (7-1) can be also purified by procedures such as chromatography, recrystallization and distillation.

### (Reference Production Method 9)

Among the compounds represented by the formula (7), a compound represented by formula (7-1) wherein W is an oxygen atom or a sulfur atom and r is 0, can be produced, for example, by reacting a compound represented by formula (12) with a compound represented by formula (13), in the presence of a base. 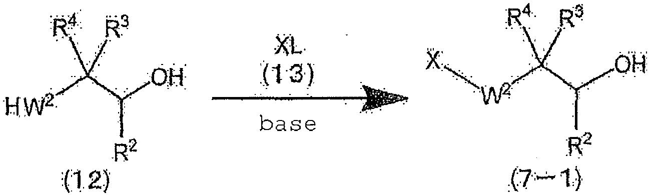 wherein W², L, X, R², R³ and R⁴ represent the same meaning as above.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (12) and the compound represented by the formula (13) used can be arbitrarily set, and is preferably a ratio that the compound represented by the formula (13) is excess, for example, the ratio of the compound represented by the formula (13) is 3 to 5 mol, based on 1 mol of the compound represented by the formula (12).

The amount of the base is arbitrary, but is usually 1 mol to an excess amount, and preferably 1 to 3 mol, based on 1 mol of the compound represented by the formula (12).

After completion of the reaction, the compound represented by the formula (7-1) can be isolated by adding the reaction mixture to water and then subjecting it to usual post-treatment operations such as an extraction with an organic solvent and concentration. In addition, the isolated compound represented by the formula (7-1) can be also purified by procedures such as chromatography, recrystallization and distillation.

### (Reference Production Method 10)

Among the compounds represented by the formula (7), a compound represented by formula (7-2) wherein R³ and R⁵ are taken together to be - (CR⁹R¹⁰)ᵥ-, R⁹ and R¹⁰ are the same or different and are a C1 to C4 alkyl group optionally having one or more halogen atoms or a hydrogen atom, and v is 1, can be produced, for example, by reacting a compound represented by formula (14) with a compound represented by formula (15). 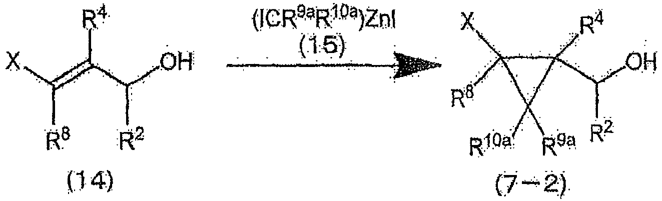 wherein R^{9a} and R^{10a} represent a C1 to C4 alkyl group optionally having one or more halogen atoms or a hydrogen atom, and X, R², R⁴ and R⁸ represent the same meaning as above.

The reaction is usually carried out in a solvent.

Examples of the solvent include halogenated hydrocarbons such as dichloromethane, chloroform, 1,2-dichloroethane and chlorobenzene, ethers such as diethyl ether and tetrahydrofuran, aliphatic hydrocarbons such as hexane and heptane, aromatic hydrocarbons such as toluene and xylene, and mixtures thereof.

The reaction time is usually within the range of 1 hour to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (14) and the compound represented by the formula (15) used is arbitrary, and the ratio of the compound represented by the formula (15) is usually 1 mol to an excess amount, and preferably 1 to 5 mol, based on 1 mol of the compound represented by the formula (14).

After completion of the reaction, the compound represented by the formula (7-2) can be obtained by adding the reaction mixture to an acidic aqueous solution (hydrochloric acid, etc.) and then subjecting it to usual post-treatment operations such as an extraction with an organic solvent and concentration. In addition, the obtained compound represented by the formula (7-2) can be further purified by procedures such as chromatography, recrystallization and distillation.

### (Reference Production Method 11)

Among the compounds represented by the formula (7), a compound represented by formula (7-2-1) wherein R² is a hydrogen atom, R³ and R⁵ are taken together to be -(CR⁹R¹⁰)ᵥ-, R⁹ and R¹⁰ are the same or different and are a C1 to C4 alkyl group optionally having one or more halogen atoms or a hydrogen atom, and v is 1, can be also produced, for example, by the following method. 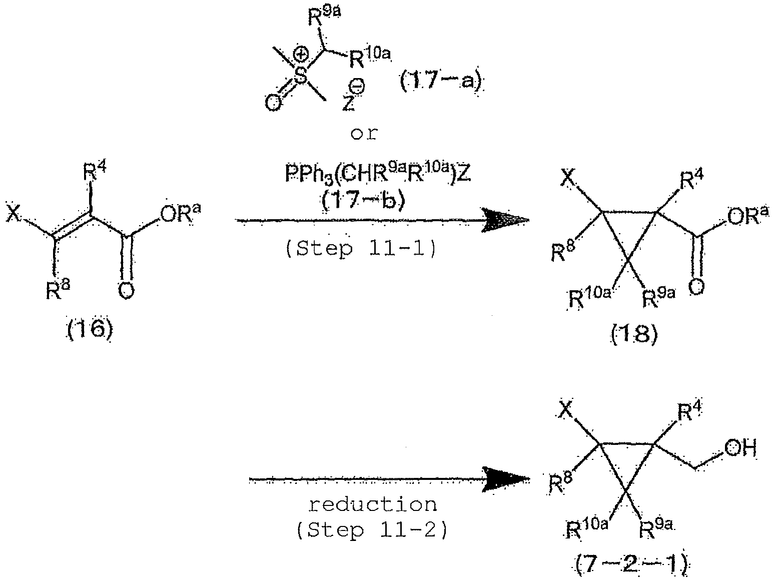 wherein X, Z, R^{a}, R⁴, R⁸, R^{9a} and R^{10a} represent the same meaning as above.

### (Step 11-1)

A compound represented by formula (18) can be produced by reacting a compound represented by formula (16) with a compound represented by formula (17-a) or a compound represented by formula (17-b), in the presence of a base.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as diethyl ether, tetrahydrofuran and dimethoxyethane, organic sulfurs such as dimethylsulfoxide and sulfolane, aliphatic hydrocarbons such as hexane and heptane, aromatic hydrocarbons such as toluene and xylene, and mixtures thereof.

The reaction time is usually within the range of 5 minutes to 72 hours.

The reaction temperature is usually within the range of -80 to 100°C.

The molar ratio of the compound represented by the formula (16) and the compound represented by the formula (17-a) or the compound represented by the formula (17-b) used can be arbitrarily set, and is preferably an equimolar ratio or a ratio close thereto. Specifically, the ratio of the compound represented by the formula (17-a) or the compound represented by the formula (17-b) is preferably 1 to 3 mol, based on 1 mol of the compound represented by the formula (16).

The amount of the base is arbitrary, but is usually 1 mol to an excess amount, and preferably a ratio of 1 to 3 mol, based on 1 mol of the compound represented by the formula (17-a) or the compound represented by the formula (17-b).

After completion of the reaction, the compound represented by the formula (18) can be obtained by adding the reaction mixture to an acidic aqueous solution and then subjecting it to usual post-treatment operations such as an extraction with an organic solvent and concentration. In addition, the obtained compound represented by the formula (18) can be further purified by procedures such as chromatography, recrystallization and distillation.

### (Step 11-2)

A compound represented by formula (7-2-1) can be produced by subjecting the compound represented by the formula (18) to a reduction reaction.

Examples of the reduction reaction include;
(1) A method using a borane complex,
(2) A method using lithium borohydride (LiBH₄),
(3) A method using diisobutylaluminum hydride (DIBAL-H),
(4) A method using lithium aluminum hydride (LAH) (LiAlH₄), and the like.
   (1) The method using a borane complex is specifically exemplified as an example.

The reaction is usually carried out in a solvent.

Examples of the borane complex include a borane/tetrahydrofuran complex, a borane/dimethyl sulfide complex, and diborane.

Examples of the solvent include ethers such as diethyl ether, tetrahydrofuran and dimethoxyethane, aliphatic hydrocarbons such as hexane and heptane, aromatic hydrocarbons such as toluene and xylene, and mixtures thereof.

The reaction time is usually within the range of 5 minutes to 24 hours.

The reaction temperature is usually within the range of -80 to 50°C (with the proviso that, when the boiling point of the used solvent is less than 50°C, it is within the range of -80°C to the boiling point of the solvent).

The amount of the borane complex is arbitrary, but is usually 1 mol to an excess amount, and preferably a ratio of 1 to 2 mol, based on 1 mol of the compound represented by the formula (18).

After completion of the reaction, the compound represented by the formula (7-2-1) can be obtained by adding the reaction mixture to water and then subjecting it to usual post-treatment operations such as an extraction with an organic solvent and concentration. In addition, the obtained compound represented by the formula (7-2-1) can be further purified by procedures such as chromatography, recrystallization and distillation.

### (Reference Production Method 12)

Among the compounds represented by the formula (7), a compound represented by formula (7-3) wherein R³ and R⁵ are taken together to be - (CR⁹R¹⁰)ᵥ-, R⁹ and R¹⁰ are the same or different and are a hydrogen atom or a halogen atom, and v is 1, can be also produced, for example, by reacting a compound represented by formula (14) with a compound represented by formula (19), in the presence of a base. 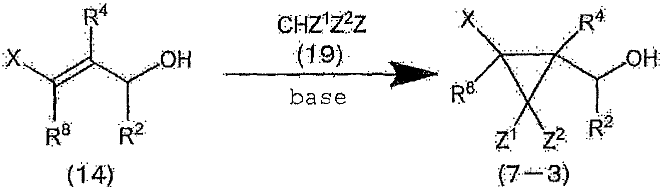 wherein Z¹ and Z² represent a hydrogen atom or a halogen atom, and X, Z, R², R⁹ and R⁸ represent the same meaning as above.

The reaction is carried out, in the presence of a base, in a solvent as necessary.

Examples of the base include alkali metal alkoxides such as sodium hydride, sodium hydroxide and potassium hydroxide.

Still another base is used for the reaction as necessary, and examples of the base include tertiary amines such as triethylamine, diisopropylethylamine, 1,8-diazabicyclo[5.4.0]undec-7-ene and 1,5-diazabicyclo[4.3.0]non-5-ene, and nitrogen containing aromatic compounds such as pyridine and 4-dimethylaminopyridine.

Examples of the solvent include ethers such as diethyl ether and tetrahydrofuran, aliphatic hydrocarbons such as hexane and heptane, aromatic hydrocarbons such as toluene and xylene, and mixtures thereof.

The reaction time is usually within the range of 1 hour to 72 hours.

The reaction temperature is usually within the range of -20 to 100°C (with the proviso that, when the boiling point of the used solvent is less than 100°C, it is within the range of -20°C to the boiling point of the solvent).

The molar ratio of the compound represented by the formula (14) and the compound represented by the formula (19) used is arbitrary, but the ratio of the compound represented by the formula (19) is usually 1 mol to an excess amount, and preferably a ratio of 1 to 5 mol, based on 1 mol of the compound represented by the formula (14).

The amount of the base is arbitrary, but is usually 1 mol to an excess amount, and preferably a ratio of 1 to 5 mol, based on 1 mol of the compound represented by the formula (19).

After completion of the reaction, the compound represented by the formula (7-3) can be obtained by adding the reaction mixture to an acidic aqueous solution and then subjecting it to usual post-treatment operations such as an extraction with an organic solvent and concentration. In addition, the obtained compound represented by the formula (7-3) can be further purified by procedures such as chromatography, recrystallization and distillation.

### (Reference Production Method 13)

Among the compounds represented by the formula (7), a compound represented by formula (7-4) wherein R³ and R⁵ are taken together to be - (CR⁹R¹⁰)ᵥ-, v is 1, 2, 3 or 4, and R⁸ is a hydrogen atom can be also produced, for example, by the following method. 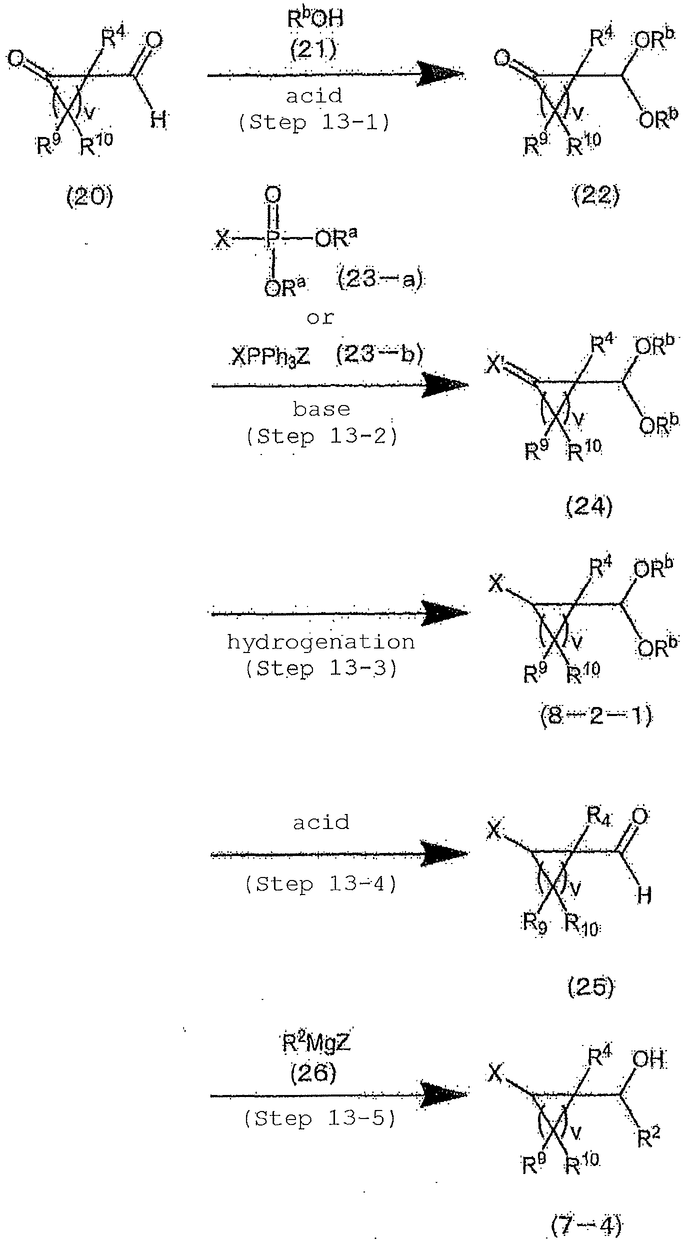 wherein X' represents a divalent group from which a hydrogen atom bonding to a carbon atom at position 1 in X is removed, and X, Z, R^{a}, R^{b}, R², R⁴, R⁹ and R¹⁰ represent the same meaning as above.

### (Step 13-1)

A compound represented by formula (22) can be produced by reacting a compound represented by formula (20) with a compound represented by formula (21), in the presence of an acid.

The reaction is carried out, in the presence of an acid, in a solvent as necessary.

Examples of the acid include inorganic acids such as hydrochloric acid and sulfuric acid and organic acids such as methanesulfonic acid and p-toluenesulfonic acid.

Examples of the solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The amount of the acid is arbitrary, but is usually 0.001 mol to an excess amount, based on 1 mol of the compound represented by the formula (20).

The molar ratio of the compound represented by the formula (21) and the compound represented by the formula (20) used is arbitrary, but the ratio of the compound represented by the formula (21) is usually 2 mol to the amount of the solvent, based on 1 mol of the compound represented by the formula (20).

After completion of the reaction, the compound represented by the formula (22) can be isolated by making the reaction mixture with a basic aqueous solution and then subjecting it to usual post-treatment operations such as an extraction with an organic solvent and concentration. In addition, the isolated compound represented by the formula (22) can be also purified by procedures such as chromatography, recrystallization and distillation.

### (Step 13-2)

A compound represented by formula (24) can be produced by reacting the compound represented by the formula (22) with a compound represented by formula (23-a) or a compound represented by formula (23-b), in the presence of a base.

The reaction is usually carried out in a solvent, in the presence of a base.

Examples of the base include alkali metal alkoxides such as sodium methoxide and potassium tert-butoxide, alkali metal hydrides such as sodium hydride and potassium hydride, and alkali metal amides such as sodium bistrimethylsilylamide, lithium bistrimethylsilylamide and lithium diisopropylamide.

Examples of the solvent include ethers such as 1,4-dioxane, tetrahydrofuran, ethylene glycol dimethyl ether and tert-butyl methyl ether, aliphatic hydrocarbons such as hexane and heptane, aromatic hydrocarbons such as benzene, toluene and xylene, halogenated hydrocarbons such as chlorobenzene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (22) and the compound represented by the formula (23-a) or the compound represented by the formula (23-b) used can be arbitrarily set, and is preferably an equimolar ratio or a ratio close thereto. Specifically, the ratio of the compound represented by the formula (23-a) or the compound represented by the formula (23-b) is preferably 0.5 to 3 mol, based on 1 mol of the compound represented by the formula (22).

The amount of the base is arbitrary, and is usually 1 mol to an excess amount and preferably a ratio of 1 to 5 mol, based on 1 mol of the compound represented by the formula (23-a) or the compound represented by the formula (23-b).

After completion of the reaction, the compound represented by the formula (24) can be isolated by adding the reaction mixture to water and then subjecting it to usual post-treatment operations such as an extraction with an organic solvent and concentration. In addition, the isolated compound represented by the formula (24) can be also purified by procedures such as chromatography, recrystallization and distillation.

### (Step 13-3)

A compound represented by formula (8-2-1) can be produced by subjecting the compound represented by the formula (24) to a reduction reaction under a hydrogen atmosphere, in the presence of a hydrogenation catalyst.

The reaction is usually carried out in a solvent, under a hydrogen atmosphere.

Examples of the hydrogenation catalyst include transition metal compounds such as a palladium carbon, palladium hydroxide, Raney nickel and platinum oxide.

Examples of the solvent include alcohols such as methanol, ethanol and propanol, esters such as ethyl acetate and butyl acetate, ethers such as tetrahydrofuran and 1,4-dioxane, and mixtures thereof.

The reaction is usually carried out under a hydrogen atmosphere at 1 to 100 atm.

The reaction time is usually within the range of 5 minutes to 72 hours.

The reaction temperature is usually within the range of -20 to 100°C (with the proviso that, when the boiling point of the used solvent is less than 100°C, it is within the range of -20°C to the boiling point of the solvent).

The amount of the hydrogenation catalyst is usually a ratio of 0.001 to 0.5 mol, based on 1 mol of the compound represented by the formula (24).

After completion of the reaction, the compound represented by the formula (8-2-1) can be isolated by filtering the reaction mixture and then subjecting it to an operation such as concentration. In addition, the isolated compound represented by the formula (8-2-1) can be further also purified by procedures such as chromatography, recrystallization and distillation.

### (Step 13-4)

A compound represented by formula (25) can be produced by reacting the compound represented by the formula (8-2-1) and water, in the presence of an acid.

The reaction is carried out in the presence of an acid, and in the presence of water and an organic solvent.

Examples of the acid include inorganic acids such as hydrochloric acid and sulfuric acid and organic acids such as methanesulfonic acid and p-toluenesulfonic acid.

Examples of the organic solvent include ethers such as 1,4-dioxane, tetrahydrofuran, ethylene glycol dimethyl ether and tert-butyl methyl ether, aromatic hydrocarbons such as toluene and xylene, halogenated hydrocarbons such as chlorobenzene, nitriles such as acetonitrile and butyronitrile, alcohols such as methanol, ethanol and propanol, ketones such as acetone and methyl isobutyl ketone, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The amount of the acid is arbitrary, and is usually 1 mol to an excess amount, based on 1 mol of the compound represented by the formula (8-2-1).

After completion of the reaction, the compound represented by the formula (25) can be isolated by adding the reaction mixture to water and then subjecting it to usual post-treatment operations such as an extraction with an organic solvent and concentration. In addition, the isolated compound represented by the formula (25) can be also purified by procedures such as chromatography, recrystallization and distillation.

### (Step 13-5)

A compound represented by formula (7-4) can be produced by reacting the compound represented by the formula (25) with the compound represented by the formula (26).

The reaction is usually carried out in a solvent.

Examples of the organic solvent include ethers such as diethyl ether, 1,4-dioxane, tetrahydrofuran, ethylene glycol dimethyl ether and tert-butyl methyl ether, aromatic hydrocarbons such as toluene and xylene, and mixtures thereof.

The reaction time is usually within the range of 5 minutes to 24 hours.

The reaction temperature is usually within the range of -50 to 100°C (with the proviso that, when the boiling point of the used solvent is less than 100°C, it is within the range of -50°C to the boiling point of the solvent).

The molar ratio of the compound represented by the formula (25) and the compound represented by the formula (26) used can be arbitrarily set, and is preferably an equimolar ratio or a ratio close thereto. Specifically, the ratio of the compound represented by the formula (26) is preferably 0. 5 to 3 mol, based on 1 mol of the compound represented by the formula (25).

After completion of the reaction, the compound represented by the formula (7-4) can be isolated by adding the reaction mixture to an acidic aqueous solution (hydrochloric acid, etc.) and then subjecting it to usual post-treatment operations such as an extraction with an organic solvent and concentration. In addition, the isolated compound represented by the formula (7-4) can be also purified by procedures such as chromatography, recrystallization and distillation.

### (Reference Production Method 14)

Among the compounds represented by the formula (7), a compound represented by formula (7-5) wherein R³ and R⁵ are taken together to be an oxygen atom can be produced, for example, by subjecting a compound represented by formula (14) to an oxidation reaction. 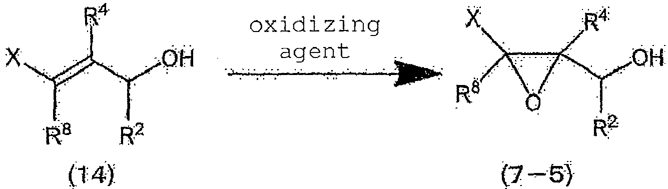 wherein X, R², R⁴ and R⁸ represent the same meaning as above.

The reaction is usually carried out in a solvent.

Examples of the oxidizing agent include peracids such as perbenzoic acid, mCPBA (meta-chloroperbenzoic acid), monoperoxyphthalic acid, trifluoroperacetic acid and peracetic acid, peroxides such as dioxirane, and the like.

Examples of the solvent include halogenated hydrocarbons such as dichloromethane, chloroform, 1,2-dichloroethane and chlorobenzene, aliphatic hydrocarbons such as hexane and heptane, aromatic hydrocarbons such as toluene and xylene, and mixtures thereof.

The reaction time is usually within the range of 5 minutes to 72 hours.

The reaction temperature is usually within the range of -20 to 80°C (with the proviso that, when the boiling point of the used solvent is less than 80°C, it is within the range of -20°C to the boiling point of the solvent).

The molar ratio of the compound represented by the formula (14) and the oxidizing agent used can be arbitrarily set, and is preferably an equimolar ratio or a ratio close thereto. Specifically, the ratio of the oxidizing agent is preferably 0.5 to 1 mol, based on 1 mol of the compound represented by the formula (14).

After completion of the reaction, the compound represented by the formula (7-5) can be obtained by filtering the reaction mixture then sequentially washing the filtrate with an aqueous solution of sodium sulfite, an aqueous solution of sodium bicarbonate and water, and subjecting it to usual post-treatment operations such as concentration. In addition, the obtained compound represented by the formula (7-5) can be further purified by procedures such as chromatography, recrystallization and distillation.

### (Reference Production Method 15)

Among the compounds represented by the formula (7), a compound represented by formula (7-6) wherein R³ and R⁵ are taken together to be a sulfur atom can be produced, for example, by reacting a compound represented by formula (7-5) with a compound represented by formula (27), in the presence of an acid. 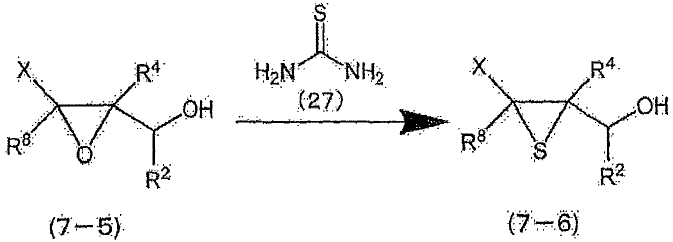 wherein X, R², R⁴ and R⁸ represent the same meaning as above.

The reaction is carried out in a solvent as necessary.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The acid includes inorganic acids such as sulfuric acid and hydrochloric acid.

The reaction time is usually within the range of 5 minutes to 24 hours.

The reaction temperature is usually within the range of -20 to 80°C (with the proviso that, when the boiling point of the used solvent is less than 80°C, it is within the range of -20°C to the boiling point of the solvent).

The molar ratio of the compound represented by the formula (7-5) and the compound represented by the formula (27) used can be arbitrarily set, and is preferably an equimolar ratio or a ratio close thereto. Specifically, the ratio of the compound represented by the formula (27) is preferably 1 to 3 mol, based on 1 mol of the compound represented by the formula (7-5).

The amount of the acid is arbitrary, and is usually an excess amount, based on the compound represented by the formula (7-5).

After completion of the reaction, the compound represented by the formula (7-6) can be obtained by filtering the reaction mixture then sequentially washing the filtrate with an aqueous solution of sodium sulfite, an aqueous solution of sodium bicarbonate and water, and subjecting it to usual post-treatment operations such as concentration. In addition, the obtained compound represented by the formula (7-6) can be further purified by procedures such as chromatography, recrystallization and distillation.

### (Reference Production Method 16)

Among the compounds represented by the formula (8), a compound represented by formula (8-3) wherein W is -CR⁸-, r is 1, and R³ and R⁵ are taken together to be -NR¹¹- , can be also produced, for example, by the following method. 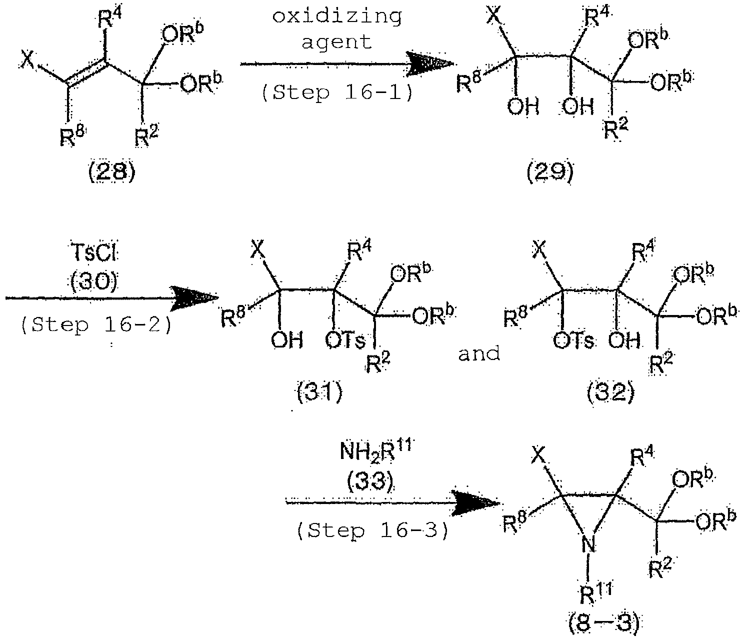 wherein Ts represents a 4-toluenesulfonyl group, and X, R^{b}, R², R⁴, R⁸ and R¹¹ represent the same meaning as above.

### (Step 16-1)

A compound represented by formula (29) can be produced by subjecting a compound represented by formula (28) to an oxidation reaction.

The reaction is usually carried out in a solvent.

Examples of the oxidizing agent include osmium tetroxide and osmium tetroxide salts.

A co-oxidizing agent is used for the reaction together with the oxidizing agent as necessary, and examples of the co-oxidizing agent include aqueous hydrogen peroxide, sodium chlorate, potassium chlorate, tert-butyl hydroperoxide, N-methylmorpholine N-oxide, potassium hexacyanoferrate (III), and the like.

Examples of the solvent include ethers such as diethyl ether, tetrahydrofuran and dimethoxyethane, ketones such as acetone, aliphatic hydrocarbons such as hexane and heptane, aromatic hydrocarbons such as benzene, toluene and xylene, alcohols such as isopropyl alcohol and tert-butyl alcohol, water, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (28) and the oxidizing agent used can be arbitrarily set, and is preferably an equimolar ratio or a ratio close thereto. Specifically, the ratio of the oxidizing agent is preferably 1 to 2 mol, based on 1 mol of the compound represented by the formula (28) . Also, when a co-oxidizing agent is used together with the oxidizing agent, the amount of the oxidizing agent used can be arbitrarily set, and the oxidizing agent is preferably the catalyst amount, more specifically, the ratio of 0.01 to 0.5 mol, based on 1 mol of the compound represented by the formula (28).

After completion of the reaction, the compound represented by the formula (29) can be obtained by adding the reaction mixture to an aqueous solution of sodium bisulfite and then subjecting it to usual post-treatment operations such as an extraction with an organic solvent and concentration. In addition, the obtained compound represented by the formula (29) can be further purified by procedures such as chromatography, recrystallization and distillation.

### (Step 16-2)

A mixture of a compound represented by formula (31) and a compound represented by formula (32) can be produced by reacting the compound represented by the formula (29) with the compound represented by the formula (30), in the presence of a base.

The reaction is carried out in a solvent as necessary.

Examples of the base include tertiary amines such as triethylamine, diisopropylethylamine, 1,8-diazabicyclo[5.4.0]undec-7-ene and 1,5-diazabicyclo[4.3.0]non-5-ene, and pyridine.

Examples of the solvent include ethers such as 1,4-dioxane, tetrahydrofuran, ethylene glycol dimethyl ether and tert-butyl methyl ether, aliphatic hydrocarbons such as hexane, heptane and octane, aromatic hydrocarbons such as toluene and xylene, halogenated hydrocarbons such as chlorobenzene, and mixtures thereof.

The reaction time is usually within the range of 5 minutes to 72 hours.

The reaction temperature is usually within the range of -80 to 100°C (with the proviso that, when the boiling point of the used solvent is less than 100°C, it is within the range of -80°C to the boiling point of the solvent).

The molar ratio of the compound represented by the formula (29) and the compound represented by the formula (30) used can be arbitrarily set, and is preferably an equimolar ratio or a ratio close thereto. Specifically, the ratio of the compound represented by the formula (30) is preferably 1 to 2 mol, based on 1 mol of the compound represented by the formula (29).

The amount of the base is arbitrary, but is usually 1 mol to an excess amount, and preferably a ratio of 1 to 3 mol, based on the compound represented by the formula (30).

After completion of the reaction, the mixture of the compound represented by the formula (31) and the compound represented by the formula (32) can be obtained by adding the reaction mixture to water and then subjecting it to usual post-treatment operations such as an extraction with an organic solvent and concentration. In addition, the obtained mixture of the compound represented by the formula (31) and the compound represented by the formula (32) can be further purified by procedures such as chromatography, recrystallization and distillation.

### (Step 16-3)

A compound represented by formula (8-3) can be produced by reacting the mixture of the compound represented by the formula (31) and the compound represented by the formula (32) with a compound represented by formula (33), in the presence of a base.

The reaction is usually carried out in a solvent, in the presence of a base.

Examples of the base include inorganic bases such as sodium hydride, sodium hydroxide, potassium hydroxide and potassium carbonate, alkali metal alkoxides such as sodium methoxide and potassium tert-butoxide, and organic bases such as triethylamine, 1,4-diazabicyclo[2.2.2]octane and 1,8-diazabicyclo[5.4.0]-7-undecene.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (33) and the mixture of the compound represented by the formula (31) and the compound represented by the formula (32) used can be arbitrarily set, and is preferably an equimolar ratio or a ratio close thereto, for example, the ratio of the compound represented by the formula (33) is 1 to 3 mol, based on 1 mol of the mixture of the compound represented by the formula (31) and the compound represented by the formula (32).

The amount of the base is arbitrary, and is usually 1 mol to an excess amount, and preferably 1 to 3 mol, based on 1 mol of the compound represented by the formula (33).

After completion of the reaction, the compound represented by the formula (8-3) can be isolated by adding the reaction mixture to water and then subjecting it to usual post-treatment operations such as an extraction with an organic solvent and concentration. In addition, the isolated compound represented by the formula (8-3) can be also purified by procedures such as chromatography, recrystallization and distillation.

### (Reference Production Method 17)

A compound represented by formula (6) can be produced, for example, by the following method. 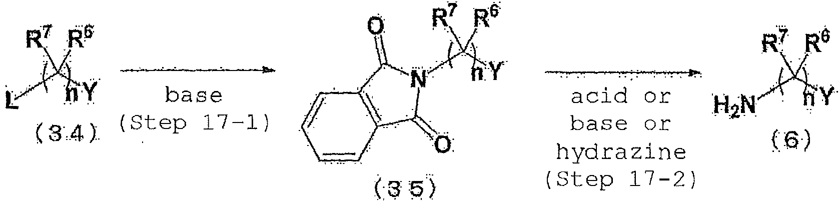 wherein L, Y, R⁶, R⁷ and n represent the same meaning as above.

### (Reference Production Method 18)

A compound represented by formula (6) can be also produced, for example, by the following method. 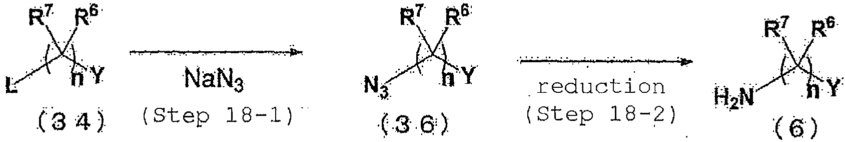 wherein L, Y, R⁶, R⁷ and n represent the same meaning as above.

### (Step 18-1)

A compound represented by formula (36) can be produced, for example, by reacting a compound represented by formula (34) with sodium azide.

The reaction is usually carried out in a solvent.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 72 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (34) and the sodium azide used can be arbitrarily set, and is preferably an equimolar ratio or a ratio close thereto, for example, the ratio of the sodium azide is 1 to 3 mol, based on 1 mol of the compound represented by the formula (34).

After completion of the reaction, the compound represented by the formula (36) can be isolated by adding the reaction mixture to water and then subjecting it to usual post-treatment operations such as an extraction with an organic solvent and concentration. In addition, the isolated compound represented by the formula (36) can be also purified by procedures such as chromatography, recrystallization and distillation.

### (Step 18-2)

A compound represented by formula (6) can be produced by subjecting the compound represented by the formula (36) to a reduction reaction.

Examples of the reduction reaction include;
(1) A method using a hydrogenation catalyst, and
(2) A method using a metal hydride such as sodium borohydride or lithium aluminum hydride.
   (1) The method of using a hydrogenation catalyst is specifically exemplified as an example.

The reaction is usually carried out in a solvent, under a hydrogen atmosphere.

Examples of the hydrogenation catalyst include transition metal compounds such as a palladium carbon, palladium hydroxide, Raney nickel and platinum oxide.

Examples of the solvent include alcohols such as methanol, ethanol and propanol, esters such as ethyl acetate and butyl acetate, ethers such as tetrahydrofuran and 1,4-dioxane, and mixtures thereof.

The reaction is usually carried out under a hydrogen atmosphere at 1 to 100 atm.

The reaction time is usually within the range of 5 minutes to 24 hours.

The reaction temperature is usually within the range of -20 to 100°C (with the proviso that, when the boiling point of the used solvent is less than 100°C, it is within the range of -20°C to the boiling point of the solvent).

The amount of the hydrogenation catalyst is usually a ratio of 0.001 to 0.5 mol, based on 1 mol of the compound represented by the formula (36).

After completion of the reaction, the compound represented by the formula (6) can be isolated by filtering the reaction mixture and then subjecting it to an operation such as concentration. The isolated compound represented by the formula (6) can be also further purified by procedures such as chromatography, recrystallization and distillation.

### (Reference Production Method 19)

Among the compounds represented by the formula (6), a compound represented by formula (6-1) wherein R⁶ and R⁷ are a hydrogen atom can be also produced, for example, by the following method. 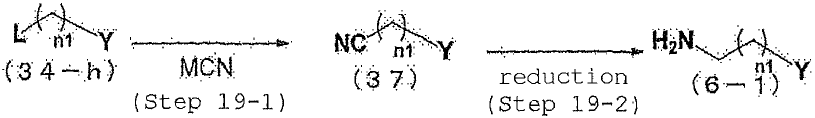 wherein M represents sodium or potassium, nl represents 0 or 1, and L and Y represent the same meaning as above.

### (Step 19-1)

A compound represented by formula (37) can be produced by reacting a compound represented by formula (34-h) with metal cyanide (MCN).

The reaction is usually carried out in a solvent.

The metal cyanide includes sodium cyanide and potassium cyanide.

Examples of the solvent include ethers such as diethyl ether, tetrahydrofuran and dimethoxyethane, acid amides such as N,N-dimethylformamide, organic sulfurs such as dimethylsulfoxide and sulfolane, aliphatic hydrocarbons such as hexane and heptane, aromatic hydrocarbons such as toluene and xylene, halogenated hydrocarbons such as 1,2-dichloroethane and chlorobenzene, water, and mixtures thereof.

The reaction time is usually within the range of 5 minutes to 24 hours.

The reaction temperature is usually within the range of 0 to 100°C (with the proviso that, when the boiling point of the used solvent is less than 100°C, it is within the range of 0°C to the boiling point of the solvent).

The molar ratio of the compound represented by the formula (34-h) and the metal cyanide used can be arbitrarily set, and is preferably an equimolar ratio or a ratio close thereto, for example, the ratio of the metal cyanide is 1 to 3 mol based on 1 mol of the compound represented by the formula (34-h).

After completion of the reaction, the compound represented by the formula (37) can be isolated by adding the reaction mixture to water and then subjecting it to usual post-treatment operations such as an extraction with an organic solvent and concentration. In addition, the isolated compound represented by the formula (37) can be also purified by procedures such as chromatography, recrystallization and distillation.

### (Step 19-2)

A compound represented by formula (6-1) can be produced by subjecting the compound represented by the formula (37) to a reduction reaction.

Examples of the reduction reaction include;
(1) A method using a hydrogenation catalyst,
(2) A method using a metal hydride such as sodium borohydride or lithium aluminum hydride,
and the like.

The method of using a hydrogenation catalyst is specifically exemplified as an example.

The reaction is usually carried out in the presence of a solvent, under a hydrogen atmosphere.

Examples of the hydrogenation catalyst used in the reaction include transition metal compounds such as a palladium carbon, palladium hydroxide, Raney nickel and platinum oxide.

Examples of the solvent used in the reaction include alcohols such as methanol, ethanol and propanol, esters such as ethyl acetate and butyl acetate, ethers such as tetrahydrofuran and 1,4-dioxane, and mixtures thereof.

The reaction is usually carried out under a hydrogen atmosphere at 1 to 100 atm.

In addition, the reaction can be also carried out by further adding ammonia as necessary.

The reaction time is usually within the range of 5 minutes to 24 hours.

The reaction temperature is usually within the range of -20 to 100°C (with the proviso that, when the boiling point of the used solvent is less than 100°C, it is within the range of -20°C to the boiling point of the solvent).

The amount of the hydrogenation catalyst is usually a ratio of 0.001 to 0.5 mol, based on 1 mol of the compound represented by the formula (37).

After completion of the reaction, the compound represented by the formula (6-1) can be isolated by filtering the reaction mixture and then subjecting it to an operation such as concentration. The isolated compound represented by the formula (6-1) can be also further purified by procedures such as chromatography, recrystallization and distillation.

The pest on which the composition of the present invention has a control effect includes arthropod pests such as pest insects and pest mites. More specifically, examples are as described below.
Hemiptera: Delphacidae such as Laodelphax striatellus, Nilaparvatalugens and Sogatella furcifera, Deltocephalidae such as Nephotettix cincticeps and Nephotettix virescens, Aphididae such as Aphis gossypii and Myzus persicae, Pentatomidae such as Nezara antennata, Riptortus clavetus, Eysarcoris lewisi, Bemisia argentifolii, Eysarcoris parvus, Plautia stali, Halyomorpha mista, Stenotus rubrovittatus and Trigonotylus ruficornis, Aleyrodidae such as Trialeurodes vaporariorum and Bemisia argentifolii, Coccidae such as Aonidiella aurantii, Comstockaspis perniciosa, Unaspis citri, Ceroplastes rubens and Icerya purchasi, Tingidae, Cimicoidea such as Cimex lectularius, Psyliidae, etc.;
Lepidoptera: Pyralidae such as Chilo suppressalis, Cnaphalocrocis medinalis, Notarcha derogata and Plodia interpunctella, Noctuidae such as Spodoptera litura, Pseudaletia separata, Trichoplusia spp., Heliothis spp., and Helicoverpa spp., Pieridae such as Pieris rapae, Tortricidae such as Adoxophyes spp. , Grapholita molesta and Cydia pomonella, Carposinidae such as Carposina niponensis, Lyonetiidae such as Lyonetia spp., Lymantriidae such as Lymantria spp. and Euproctis spp., Yponomeutidae such as Plutella xylostella, Gelechiidae such as Pectinophora gossypiella, Arctiidae such as Hyphantria cunea, Tineidae such as Tinea translucens and Tineolabisselliella, etc.;
Diptera: Culex such as Culex pipiens pallens, Culex tritaeniorhynchus and Culex quinquefasciatus, Aedes spp. such as Aedes aegypti and Aedes albopictus, Anopheles spp. such as Anopheles sinensis, Chironomidae, Muscidae such as Musca domestica and Muscina stabulans, Calliphoridae, Sarcophagidae, Fanniidae, Anthomyiidae such as Delia platura and Delia antiqua, Agromyzidae such as Liriomyza trifolii, Tephritidae, Drosophilidae, Phoridae such as Megaselia spiracularis, Psychodidae such as Clogmia albipunctata, Simuliidae, Tabanidae, Stomoxys, etc.;
Coleoptera: Diabrotica such as Diabrotica virgifera virgifera and Diabrotica undecimpunctata howardi, Scarabaeidae such as Anomala cuprea and Anomala rufocuprea, Curculionidae such as Sitophilus zeamais, Lissorhoptrus oryzophilus and Callosobruchuys chienensis, Tenebrionidae such as Tenebrio molitor and Tribolium castaneum, Chrysomelidae such as Oulema oryzae, Aulacophora femoralis, Phyllotreta striolata and Leptinotarsa decemlineata, Dermestidae such as Dermestes maculates, Anobiidae, Epilachna such as Epilachna vigintioctopunctata, Lyctidae, Bostrychidae, Ptinidae, Cerambycidae, Paederus fuscipes, etc.;
Blattodea: Blattella germanica, Periplaneta fuliginosa, Periplaneta americana, Periplaneta brunnea, Blatta orientalis, etc.;
Thysanoptera: Thrips palmi, Thrips tabaci, Frankliniella occidentalis, Frankliniella intonsa, etc.;
Hymenoptera: Formicidae such as Monomorium pharaosis, Formica fusca japonica, Ochetellus glaber, Pristomyrmex pungens and Pheidole noda, Vespidae, Bethylidae, Tenthredinidae such as Athalia japonica, etc.;
Orthoptera: Gryllotalpidae, Acrididae, Grylloidea, etc.; Siphonaptera: Ctenocephalides felis, Ctenocephalides canis, Pulex irritans, Xenopsylla cheopis, etc.;
Anoplura: Pediculus humanus corporis, Phthirus pubis,
Haematopinus eurysternus, Dalmalinia ovis, Haematopinus suis, etc.;
Isoptera: Subterranean termites such as Reticulitermes speratus, Coptotermes formosanus, Reticulitermes flavipes, Reticulitermes hesperus, Reticulitermes virginicus, Reticulitermes tibialis and Heterotermes aureus, Drywood termites such as Incisitermes minor, Dampwood termites such as Zootermopsis nevadensis, etc.;
Acarina: Tetranychidae such as Tetranychus urticae, Tetranychus kanzawai, Panonychus citri, Panonychus ulmi and Oligonychus spp. , Eriophyidae such as Aculops lycopers, Aculops pelekassi and Aculus schlechtendali, Tarsonemidae such as Polyphagotarsonemus latu, Tenuipalpidae, Tuckerellidae, Metastigmata such as Haemaphysalis longicornis, Haemaphysalis flava, Dermacentor variabilis, Haemaphysalis flava, Dermacentor taiwanicus, Ixodes ovatus, Ixodes persulcatus, Ixodes scapularis, Boophilus microplus, Amblyomma americanum and Rhipicephalus sanguineus, Acaridae such as Tyrophagus putrescentiae, Pyroglyphidae such as Dermatophagoides farinae and Dermatophagoides pteronyssinus, Cheyletidae such as Cheyletus eruditus, Cheyletus malaccensis and Cheyletus moorei, Dermanyssidae such as Ornithonyssus bacoti, Ornithonyssus sylvairum and Dermanyssus gallinae, Trombiculidae such as Leptotrombidium akamushi, etc.;
Arachnida: Chiracanthium japonicum, Latrodectus hasseltii, etc.;
Chilopoda: Thereuonema hilgendorfi, Scolopendra subspinipes, etc.;
Diplopoda: Oxidus gracilis, Nedyopus tambanus, etc.;
Isopoda: Armadillidium vulgare, etc.;
Gastropoda: Limax marginatus, Limax flavus, etc.

The pest control agent of the present invention contains the compound of the present invention and an inert carrier. The pest control agent of the present invention is usually obtained by mixing the compound of the present invention and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, flowables, microcapsule formulations, aerosols, fumigants, poisonous baits, resin formulations and the like. These formulations usually contain the compound of the present invention in an amount of 0.01 to 95% by weight.

Examples of the solid carrier which is used in the formulation include fine powder and granules of clay material (kaolin clay, diatomaceous earth, bentonite, Fubasami clay, acid clay, etc.), synthetic hydrated silicon oxide, talc, ceramics, other inorganic minerals (sericite, quartz, sulfur, activated carbon, calcium carbonate, hydrated silica, etc.), chemical fertilizers (ammonium sulfate, ammonium phosphate, ammonium nitrate, urea, ammonium chloride, etc.) and the like.

Examples of the liquid carrier include water, alcohols (methanol, ethanol, isopropyl alcohol, butanol, hexanol, benzyl alcohol, ethylene glycol, propylene glycol, phenoxyethanol, etc.), ketones (acetone, methyl ethyl ketone, cyclohexanone, etc.), aromatic hydrocarbons (toluene, xylene, ethylbenzene, dodecylbenzene, phenylxylylethane, methylnaphthalene, etc.), aliphatic hydrocarbons (hexane, cyclohexane, kerosene, light oil, etc.), esters (ethyl acetate, butyl acetate, isopropyl myristate, ethyl oleate, diisopyl adipate, diisobutyl adipate, propylene glycol monomethyl ether acetate, etc.), nitriles (acetonitrile, isobutyronitrile, etc.), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, etc.), acid amides (N,N-dimethylformamide, N,N-dimethylacetamide, etc.), halogenated hydrocarbons (dichloromethane, trichloroethane, carbon tetrachloride, etc.), sulfoxides (dimethyl sulfoxide, etc.), and propylene carbonate and vegetable oils (soybean oil, cottonseed oil, etc.).

Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkylsulfates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, etc.), lignin derivatives, bentonite, synthetic water-soluble polymers (polyvinyl alcohol, polyvinyl pyrrolidone, polyacrylic acid, etc.), PAP (isopropyl acid phosphate), BHT (2,6-di-tert-butyl-4-methylphenol) and BHA (mixtures of 2-tert-butyl-4-methoxyphenol and 3-tert-butyl-4-methoxyphenol).

The method for controlling pests of the present invention is carried out by applying an effective amount of the composition of the present invention to a pest or a place where a pest inhabits. In the method for controlling pests of the present invention, the compound of the present invention is usually used in the form of the pest control agent of the present invention.

The place where a pest inhabits includes paddy fields, fields, orchards, non-agricultural lands, houses and the like.

The application can be carried out by the application method similar to the conventional one, as long as the compound of the present invention can be brought into contact with or ingested by a pest.

Examples of the application method include spraying treatment, soil treatment, seed treatment and water culture medium treatment.

When the pest control agent of the present invention is used in pest controlling, the application amount is usually 1 to 10000 g per the amount of the compound of the present invention per 10000 m². When the pest control agent of the present invention is formulated into an emulsifiable concentrate, a wettable powder, a flowable or the like, the pest control agent is usually diluted with water so as to have a concentration of the active ingredient of 0.01 to 10000 ppm, and dust formulations, granules and the like are usually applied as they are.

These formulations and formulation solutions diluted with water may be directly treated by being sprayed on a pest or a plant such as crops which should be protected from pests, and also may be treated on a soil in order to control a pest that inhabits in the soil of cultivated land.

Also, the resin formulation processed into a sheet or string can be also treated by a method such as winding it around crops, spreading it in the vicinity of crops, or spreading it to the soil around crop roots.

When the pest control agent of the present invention is used in controlling the pest that inhabits in the house, the application amount is usually 0.01 to 1000 mg in an amount of the compound of the present invention per 1 m² of an area to be treated, in the case of using it on a planar area, and is usually 0.01 to 500 mg in an amount of the compound of the present invention per 1 m² of a space to be treated, in the case of using it in a space. When the pest control agent of the present invention is formulated into an emulsifiable concentrate, a wettable powder, a flowable or the like, the pest control agent is usually diluted with water so as to have a concentration of the active ingredient of 0.1 to 1000 ppm and applied, and oil formulations, aerosols, fumigants, poisonous baits and the like are applied as they are.

The compound of the present invention can be used in the farmland where the following "crops" are grown.

Crops; corn, rice, wheat, barley, rye, oat, sorghum, cotton, soybean, peanut, sarrazin, sugar beet, rapeseed, sunflower, sugar cane, tobacco, etc.

Vegetables; Solanaceae vegetables (eggplant, tomato, green pepper, hot pepper, potato, etc.), Cucurbitaceae vegetables (cucumber, pumpkin, zucchini, watermelon, melon, etc.), Cruciferae vegetables (Japanese radish, turnip, horseradish, kohlrabi, Chinese cabbage, cabbage, brown mustard, broccoli, cauliflower, etc.), Compositae vegetables (burdock, garland chrysanthemum, artichoke, lettuce, etc.), Liliaceae vegetables (Welsh onion, onion, garlic, asparagus, etc.), Umbelliferae vegetables (carrot, parsley, celery, parsnip, etc.), Chenopodiaceae vegetables (spinach, Swiss chard, etc.), Labiatae vegetables (Japanese mint, mint, basil, etc.), strawberry, sweat potato, yarn, aroid, etc.
Flowers;
Foliage plants;
Fruit trees; pomaceous fruits (apple, common pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruits, etc.), nuts (chestnut, walnut, hazel nut, almond, pistachio, cashew nut, macadamia nut, etc.), berry fruits (blueberry, cranberry, blackberry, raspberry, etc.), grape, persimmon, olive, loquat, banana, coffee, date, coconut, oil palm, etc.;

Trees other than fruit trees; tea, mulberry, flowering trees and shrubs, street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew), etc.

The "crops" also contains genetically modified crops.

The pest control agent of the present invention can be used as a mixture with or in combination with other insecticide, miticide, nematicide, fungicide, plant growth regulator, herbicide or synergist. Examples of the active ingredient of said insecticide, miticide, nematicide, fungicide, plant growth regulator, herbicide and synergist are shown below.

### Active Ingredients of Insecticide

### (1) Organic Phosphorus Compounds

acephate, Aluminium phosphide, butathiofos, cadusafos, chlorethoxyfos, chlorfenvinphos, chlorpyrifos, chlorpyrifos-methyl, cyanophos: CYAP, diazinon, DCIP (dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, Hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos, tetrachlorvinphos, terbufos, thiometon, trichlorphon: DEP, vamidothion, phorate, and cadusafos.

### (2) Carbamate Compounds

alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, and aldicarb.

### (3) Pyrethroid Compounds

acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, 2,3,5,6-tetrafluoro-4-(methoxymethyl)benzyl (EZ)-(1RS, 3RS; 1RS, 3SR)-2,2-dimethyl-3-prop-1-enylcyclopropanecarboxylate, 2,3,5,6-tetrafluoro-4-methylbenzyl (EZ)-(1RS, 3RS; 1RS, 3SR)-2,2-dimethyl-3-prop-1-enylcyclopropanecarboxylate, and 2,3,5,6-tetrafluoro-4-(methoxymethyl)benzyl (1RS, 3RS; 1RS, 3SR)-2,2-dimethyl-3-(2-methyl-1-propenyl)cyclopropanecarbox ylate.

### (4) Nereistoxin Compounds

cartap, bensultap, thiocyclam, monosultap, andbisultap.

### (5) Neonicotinoid Compounds

imidacloprid, nitenpyram, acetamiprid, thiamethoxam, thiacloprid, dinotefuran, and clothianidin.

### (6) Benzoyl Urea Compounds

chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron.

### (7) Phenylpyrazole-Based Compounds

acetoprole, ethiprole, fipronil, vaniliprole, pyriprole, and pyrafluprole.

### (8) Bt Toxins

Living spores derived from Bacillus thuringiensis and produced crystalline toxins and mixtures thereof.

### (9) Hydrazine Compounds

chromafenozide, halofenozide, methoxyfenozide, and tebufenozide.

### (10) Organic Chlorine Compounds

aldrin, dieldrin, dienochlor, endosulfan, and methoxychlor.

### (11) Other Active Ingredients of Insecticide

machine oil and nicotine-sulfate; avermectin-B, bromopropylate, buprofezin, chlorphenapyr, cyantraniliprole, cyromazine, D-D (1,3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, Potassium oleate, protrifenbute, spiromesifen, sulfoxaflor, Sulfur, metaflumizone, spirotetramat, pyrifluquinazone, spinetoram, chlorantraniliprole, tralopyril, and cyantraniliprole.

### Active ingredients of Miticide

acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite: BPPS, polynactins, pyridaben, pyrimidifen, tebufenpyrad, tetradifon, spirodiclofen, spiromesifen, spirotetramat, amidoflumet, and cyenopyrafen.

### Active ingredients of Nematicide

DCIP, fosthiazate, levamisol hydrochloride (levamisol), methylisothiocyanate, morantel tartarate, and imicyafos.

### Active ingredients of Fungicide

Azole fungicidal compounds such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, and flutriafol;

Cyclic amine fungicidal compounds such as fenpropimorph, tridemorph, and fenpropidin;

Benzimidazole fungicidal compounds such as carbendezim, benomyl, thiabendazole, and thiophanate-methyl; procymidone; cyprodinil; pyrimethanil; diethofencarb; thiuram; fluazinam; mancozeb; iprodione; vinclozolin; chlorothalonil; captan; mepanipyrim; fenpiclonil; fludioxonil; dichlofluanid; folpet; kresoxim-methyl; azoxystrobin; trifloxystrobin; fluoxastrobin; picoxystrobin; pyraclostrobin; dimoxystrobin; pyribencarb; spiroxamine; quinoxyfen; fenhexamid; famoxadone; fenamidone; zoxamide; ethaboxam; amisulbrom; iprovalicarb; benthiavalicarb; cyazofamid; mandipropamid; boscalid; penthiopyrad; metrafenone; fluopiran; bixafen; cyflufenamid; proquinazid; isotianil, and tiadinil.

### Active ingredients of Herbicide

### (1) Phenoxy fatty acid herbicidal compounds

2,4-PA, MCP, MCPB, phenothiol, mecoprop, fluroxypyr, triclopyr, clomeprop, and naproanilide.

### (2) Benzoate herbicidal compounds

2,3,6-TBA, dicamba, clopyralid, picloram, aminopyralid, quinclorac, and quinmerac.

### (3) Urea herbicidal compounds

diuron, linuron, chlortoluron, isoproturon, fluometuron, isouron, tebuthiuron, methabenzthiazuron, cumyluron, daimuron, and methyl-daimuron.

### (4) Triazine herbicidal compounds

atrazine, ametoryn, cyanazine, simazine, propazine, simetryn, dimethametryn, prometryn, metribuzin, triaziflam, and indaziflam.

### (5) Bipyridinium herbicidal compounds paraquat, and diquat.

### (6) Hydroxybenzonitrile herbicidal compounds

bromoxynil, and ioxynil.

### (7) Dinitroaniline herbicidal compounds

pendimethalin, prodiamine, and trifluralin.

### (8) Organophosphorus herbicidal compounds

amiprofos-methyl, butamifos, bensulide, piperophos, anilofos, glyphosate, glufosinate, glufosinate-P, and bialaphos.

### (9) Carbamate herbicidal compounds

di-allate, tri-allate, EPTC, butylate, benthiocarb, esprocarb, molinate, dimepiperate, swep, chlorpropham, phenmedipham, phenisopham, pyributicarb, and asulam.

### (10) Acid amide herbicidal compounds

propanil, propyzamide, bromobutide, and etobenzanid.

### (11) Chloroacetanilide herbicidal compounds

acetochlor, alachlor, butachlor, dimethenamid, propachlor, metazachlor, metolachlor, pretilachlor, thenylchlor, and pethoxamid.

### (12) Diphenyl ether herbicidal compounds

acifluorfen-sodium, bifenox, oxyfluorfen, lactofen, fomesafen, chlomethoxynil, and aclonifen.

### (13) Cyclic imide herbicidal compounds

oxadiazon, cinidon-ethyl, carfentrazone-ethyl, surfentrazone, flumiclorac-pentyl, flumioxazin, pyraflufen-ethyl, oxadiargyl, pentoxazone, fluthiacet-methyl, butafenacil, benzfendizone, bencarbazone, and saflufenacil.

### (14) Pyrazole herbicidal compounds

benzofenap, pyrazolate, pyrazoxyfen, topramezone, and pyrasulfotole.

### (15) Triketone herbicidal compounds

isoxaflutole, benzobicyclon, sulcotrione, mesotrione, tembotrione, and tefuryltrione.

### (16) Aryloxyphenoxypropionate herbicidal compounds clodinafop-propargyl, cyhalofop-butyl, diclofop-methyl, fenoxaprop-ethyl, fluazifop-butyl, haloxyfop-methyl, and quizalofop-ethyl, metamifop.

### (17) Trione oxime herbicidal compounds

alloxydim-sodium, sethoxydim, butroxydim, clethodim, cloproxydim, cycloxydim, tepraloxydim, tralkoxydim, and profoxydim.

### (18) Sulfonyl urea herbicidal compounds

chlorsulfuron, sulfometuron-methyl, metsulfuron-methyl, chlorimuron-ethyl, tribenuron-methyl, triasulfuron, bensulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.

### (19) Imidazolinone herbicidal compounds

imazamethabenz-methyl, imazamethapyr, imazamox, imazapyr, imazaquin, and imazethapyr.

### (20) Sulfonamide herbicidal compounds

flumetsulam, metosulam, diclosulam, florasulam, cloransulam-methyl, penoxsulam, and pyroxsulam. (21) Pyrimidinyloxybenzoate herbicidal compounds pyrithiobac-sodium, bispyribac-sodium, pyriminobac-methyl, pyribenzoxim, pyriftalid, and pyrimisulfan.

### (22) Other herbicidal compounds

bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium, mefenacet, flufenacet, fentrazamide, cafenstrole, indanofan, oxaziclomefone, benfuresate, ACN, pyridate, chloridazon, norflurazon, flurtamone, diflufenican, picolinafen, beflubutamid, clomazone, amicarbazone, pinoxaden, pyraclonil, pyroxasulfone, thiencarbazone-methyl, aminocyclopyrachlor, ipfencarbazone, and methiozolin.

### Active ingredients of Synergist

piperonyl butoxide, sesamex, sulfoxide, N-(2-ethylhexyl)-8,9,10-trinorborn-5-ene-2,3-dicarboximide (MGK 264), N-declyimidazole, WARF-antiresistant, TBPT, TPP, IBP, PSCP, methyl iodide (CH₃I), t-phenylbutenone, diethylmaleate, DMC, FDMC, ETP, and ETN.

### EXAMPLES

Hereinbelow, the present invention will be further illustrated by production examples, formulation examples, test examples, and the like. However, the present invention is not limited to these examples.

First, the production examples of the compounds of the present invention are shown below.

### Production Example 1

(E)-6-Phenyl-2-hexenoic acid (0.29 mg, 1.5 mmol), triethylamine (0.23 mg, 2.3 mmol) and tetrahydrofuran-3-ylmethylamine hydrochloride (0.31 g, 2.3 mmol) were added to tetrahydrofuran (4 mL). To the mixture solution was added 1-ethyl-3-(3-dimethylaminopropyl)carbodiimide hydrochloride (0.35 mg, 1.8 mmol) at room temperature, then the mixture was stirred at room temperature for 4 hours. Thereafter, water was added to the reaction mixture, and the mixture was extracted twice with ethyl acetate. The organic layer was washed with a saturated salt solution, and dried over anhydrous sodium sulfate, then filtered, and the filtrate was concentrated under reduced pressure. The residue was applied to a silica gel column chromatography, to obtain 0.16 g of
(E)-N-(tetrahydrofuran-3-ylmethyl)-6-phenyl-2-hexeneamide (hereinafter, described as Compound (1) of the present
invention) represented by the following formula.

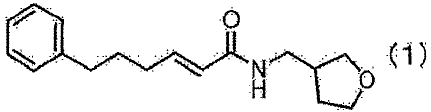

### Compound (1) of the present invention

¹H-NMR (CDCl₃, TMS) δ (ppm) : 1.64 (dd, 1H), 1.75-1.83 (m, 2H), 2.00-2.09 (m, 1H), 2.18-2.25 (m, 2H), 2.48-2.54 (m, 1H), 2.64 (t, 2H), 3.29-3.40 (m, 2H), 3.55 (dd, 1H), 3.73 (dd, 1H), 3.81 (dd, 1H), 3.89 (td, 1H), 5.58 (br s, 1H), 5.76 (dt, 1H), 6.86 (dt, 1H), 7.15-7.21 (m, 3H), 7.26-7.31 (m, 2H) Production Example 2

The reaction was carried out in the same manner using 0.40 g of (E)-7-phenyl-2-heptenoic acid in place of

(E) -6-phenyl-2-hexenoic acid in Production Example 1, to obtain 0.28 g of (E)-N-(tetrahydrofuran-3-ylmethyl)-7-phenyl-2-hepteneamide (hereinafter, described as Compound (2) of the present invention) represented by the following formula.

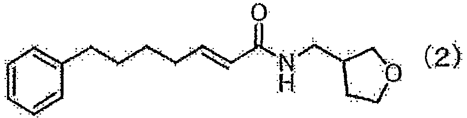

Compound (2) of the present invention

¹H-NMR (CDCl₃, TMS) δ (ppm): 1.44-1.51 (m, 2H), 1.56-1. 68 (m, 3H), 1.97-2.05 (m, 1H), 2.14-2.21 (m, 2H), 2.45-2.51 (m, 1H), 2.60 (t, 2H), 3.23-3.36 (m, 2H), 3.53 (dd, 1H), 3.71 (dd, 1H), 3.79 (dd, 1H), 3.86 (td, 1H), 5.76 (d, 1H), 6.22 (br s, 1H), 6.81(dt, 1H), 7.14-7.20 (m, 3H), 7.24-7.30 (m,2H)

### Production Example 3

The reaction was carried out in the same manner using 0.33 g of (E)-8-phenyl-2-octenoic acid in place of (E) -6-phenyl-2-hexenoic acid in Production Example 1, to obtain 0.40 g of (E)-N-(tetrahydrofuran-3-ylmethyl)-8-phenyl-2-octeneamide (hereinafter, described as Compound (3) of the present invention) represented by the following formula.

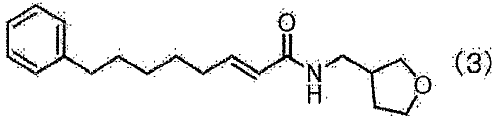

### Compound (3) of the present invention

¹H-NMR (CDCl₃, TMS) δ (ppm): 1.31-1.40 (m, 2H), 1.43-1.51 (m, 2H), 1.58-1.66 (m, 3H), 2.00-2.07 (m, 1H), 2.13-2.20 (m, 2H), 2.47-2.53 (m, 1H), 2.60 (t, 2H), 3.26-3.38 (m, 2H), 3.54 (dd, 1H), 3.73 (dd, 1H), 3.80 (dd, 1H), 3.87 (td, 1H), 5.75 (d, 1H), 5.94 (brs, 1H), 6.82 (dt, 1H), 7.15-7.20 (m, 3H), 7.25-7.30 (m, 2H)

### Production Example 4

The reaction was carried out in the same manner using 0.25 g of (E)-9-phenyl-2-nonenoic acid in place of (E) -6-phenyl-2-hexenoic acid in Production Example 1, to obtain 0.26 g of (E)-N-(tetrahydrofuran-3-ylmethyl)-9-phenyl-2-noneneamide (hereinafter, described as Compound (4) of the present invention) represented by the following formula

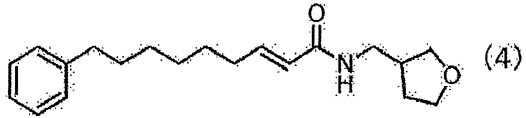

### Compound (4) of the present invention

¹H-NMR (CDCl₃, TMS) δ (ppm) : 1.32-1.35 (m, 4H), 1.38-1. 49 (m, 2H), 1.57-1.67 (m, 3H), 1.99-2.08 (m, 1H), 2.13-2.19 (m, 2H), 2.46-2.56 (m, 1H), 2.60 (t, 2H), 3.24-3.39 (m, 2H), 3.55 (dd, 1H), 3.73 (dd, 1H), 3.81 (dd, 1H), 3.88 (td, 1H), 5.75 (d, 1H), 5.77 (br s, 1H), 6.83 (dt, 1H), 7.17 (dd, 3H), 7.27 (td, 2H)

### Production Example 5

The reaction was carried out in the same manner using 0.21 g of (E)-10-phenyl-2-decenoic acid in place of (E) -6-phenyl-2-hexenoic acid in Production Example 1, to obtain 0.18 g of (E)-N-(tetrahydrofuran-3-ylmethyl)-10-phenyl-2-deceneamide (hereinafter, described as Compound (5) of the present invention) represented by the following formula.

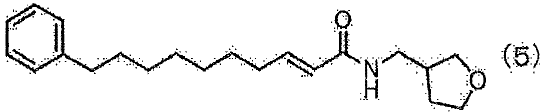

### Compound (5) of the present invention

¹H-NMR (CDCl₃, TMS) δ (ppm): 1.32-1.38 (m, 6H), 1.38-1.48 (m, 2H), 1.55-2.70 (m, 3H), 2.00-2.09 (m, 1H), 2.13-2.20 (m, 2H), 2.48-2.54 (m, 1H), 2.59 (t, 2H), 3.30-3.36 (m, 2H), 3.55 (dd, 1H), 3.73 (dd, 1H), 3.81 (dd, 1H), 3.89 (td, 1H), 5.59 (br s, 1H), 5.74 (dt, 1H), 6.84 (dt, 1H), 7.15-7.20 (m, 3H), 7.25-7.29 (m, 2H)

### Production Example 6

The reaction was carried out in the same manner using 0.15 g of (E)-3-methyl-6-phenyl-2-hexenoic acid in place of (E) -6-phenyl-2-hexenoic acid in Production Example 1, to obtain 0.14 g of (E)-N-(tetrahydrofuran-3-ylmethyl)-3-methyl-6-phenyl-2-hexe neamide (hereinafter, described as Compound (6) of the present invention) represented by the following formula.

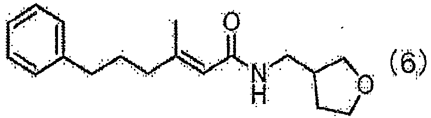

### Compound (6) of the present invention

¹H-NMR (CDCl₃, TMS) δ (ppm): 1.58-1.66 (m, 1H), 1.76-1.83 (m, 2H), 1.98-2.08 (m, 1H), 2.10-2.16 (m, 2H), 2.14 (s, 3H), 2.44-2.55 (m, 1H), 2.61 (t, 2H), 3.24-3.35 (m, 2H), 3.54 (dd, 1H), 3.73 (dd, 1H), 3.81 (dd, 1H), 3.88 (td, 1H), 5.52 (s, 1H), 5.56 (br s, 1H), 7.15-7.22 (m, 3H), 7.25-7.32 (m, 2H) Production Example 7

The reaction was carried out in the same manner using 0.38 g of (E)-4-benzyloxy-2-butenoic acid in place of (E) -6-phenyl-2-hexenoic acid in Production Example 1, to obtain 0.25 g of (E)-N-(tetrahydrofuran-3-ylmethyl)-4-benzyloxy-2-buteneamid e (hereinafter, described as Compound (7) of the present invention) represented by the following formula.

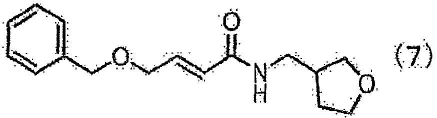

### Compound (7) of the present invention

¹H-NMR (CDCl₃, TMS) 5 (ppm): 1.57-1.68 (m, 1H), 1.99-2.09 (m, 1H), 2.45-2.55 (m, 1H), 3.35 (td, 2H), 3.55 (dd, 1H), 3.73 (dd, 1H), 3.81 (dd, 1H), 3.89 (td, 1H), 4.19 (dd, 2H), 4.57 (s, 2H), 5.72 (br s, 1H), 6.09 (dt, 1H), 6.89 (dt, 1H), 7.31-7.39 (m, 5H)

### Production Example 8

(E)-6-Phenyl-2-hexenoic acid (0.29 g, 1.5 mmol) and tetrahydropyran-4-ylmethylamine (0.26 g, 2.3 mmol) were added to tetrahydrofuran (4 mL). To the mixture solution was added 1-ethyl-3-(3-dimethylaminopropyl)carbodiimide hydrochloride (0.35 g, 1.8 mmol) at room temperature, then the mixture was stirred at room temperature for 4 hours. Thereafter, water was added to the reaction mixture, and the mixture was extracted twice with ethyl acetate. The organic layer was washed with a saturated salt solution, and dried over anhydrous sodium sulfate, then filtered, and the filtrate was concentrated under reduced pressure. The residue was applied to a silica gel column chromatography, to obtain 0.16 g of (E)-N-(tetrahydropyran-4-ylmethyl)-6-phenyl-2-hexeneamide (hereinafter, described as Compound (8) of the present invention) represented by the following formula.

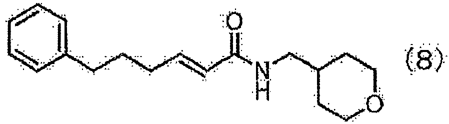

### Compound (8) of the present invention

¹H-NMR (CDCl₃, TMS) δ (ppm) : 1.27-1.38 (m, 2H), 1.60-1.62 (m, 2H), 1.75-1.83 (m, 3H), 2.18-2.25 (m, 2H), 2.64 (t, 2H), 3.22 (t, 2H), 3.37 (td, 2H), 3.97 (dd, 2H), 5.50 (br s, 1H), 5.76 (d, 1H), 6.86 (dt, 1H), 7.15-7.22 (m, 3H), 7.26-7.32 (d, 2H)

### Production Example 9

The reaction was carried out in the same manner using 0.41 g of (E)-7-phenyl-2-heptenoic acid in place of (E) -6-phenyl-2-hexenoic acid in Production Example 8, to obtain 0.28 g of (E)-N-(tetrahydropyran-4-ylmethyl)-7-phenyl-2-hepteneamide (hereinafter, described as Compound (9) of the present invention) represented by the following formula.

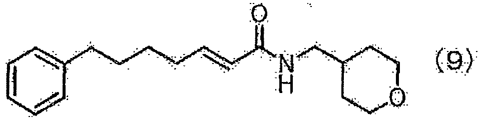

### Compound (9) of the present invention

¹H-NMR (CDCl₃, TMS) δ (ppm) : 1.23-1.34 (m, 2H), 1.43-1.51 (m, 2H), 1.54-1.70 (m, 4H), 1.70-1.81 (m, 1H), 2.18 (q, 2H), 2.60 (t, 2H), 3.17 (t, 2H), 3.30-3.36 (m, 2H), 3.94 (dd, 2H), 5.77 (d, 1H), 6.21 (br s, 1H), 6.81 (dt, 1H), 7.13-7.20 (m, 3H), 7.23-7.30 (m, 2H)

### Production Example 10

The reaction was carried out in the same manner using 0.33 g of (E)-8-phenyl-2-octenoic acid in place of (E) -6-phenyl-2-hexenoic acid in Production Example 8, to obtain 0.42 g of (E)-N-(tetrahydropyran-4-ylmethyl)-8-phenyl-2-octeneamide (hereinafter, described as Compound (10) of the present invention) represented by the following formula.

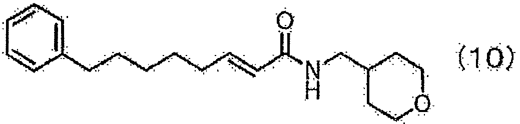

### Compound (10) of the present invention

¹H-NMR (CDCl₃ TMS) δ (ppm): 1.22-1.40 (m, 4H), 1.40-1.50 (m, 2H), 1.56-1.64 (m, 4H), 1.76 (s, 1H), 2.14 (q, 2H), 2.58 (t, 2H), 3.17 (t, 2H), 3.33 (dt, 2H), 3.94 (dd, 2H), 5.83 (d, 1H), 6. 69 (br s, 1H), 6. 81 (dt, 1H), 7.12-7.19 (m, 3H), 7.22-7.29 (m, 2H)

### Production Example 11

The reaction was carried out in the same manner using 0.25 g of (E)-9-phenyl-2-nonenoic acid in place of (E) -6-phenyl-2-hexenoic acid in Production Example 8, to obtain 0.22 g of (E)-N-(tetrahydropyran-4-ylmethyl)-9-phenyl-2-noneneamide (hereinafter, described as Compound (11) of the present invention) represented by the following formula.

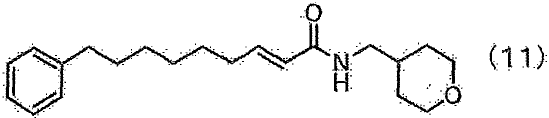

### Compound (11) of the present invention

¹H-NMR (CDCl₃, TMS) δ (ppm): 1.30 (ddd, 6H), 1.43 (t, 2H), 1.61 (d, 4H), 1.77 (dd, 1H), 2.15 (q, 2H), 2.59 (t, 2H), 3.20 (t, 2H), 3.35 (td, 2H), 3.96 (dd, 2H), 5.75 (d, 2H), 6.82 (dt, 1H), 7.14-7.21 (m, 3H), 7.24-7.31 (m, 2H)

### Production Example 12

The reaction was carried out in the same manner using 0.15 g of (E)-3-methyl-6-phenyl-2-hexenoic acid in place of (E) -6-phenyl-2-hexenoic acid in Production Example 8, to obtain 0.21 g of (E)-N-(tetrahydropyran-4-ylmethyl)-3-methyl-6-phenyl-2-hexe neamide (hereinafter, described as Compound (12) of the present invention) represented by the following formula.

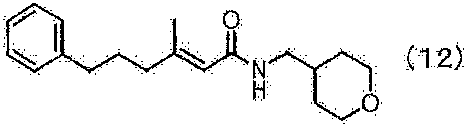

### Compound (12) of the present invention

¹H-NMR (CDCl₃ TMS) δ (ppm) : 1.25-1.37 (m, 2H), 1.61 (dd, 2H), 1.72-1.83 (m, 3H), 2.12 (d, 2H), 2.15 (d, 3H), 2.61 (t, 2H), 3.18 (t, 2H), 3.36 (td, 2H), 3.97 (dd, 2H), 5.50 (br s, 1H), 5.53 (d, 1H), 7.16-7.21 (m, 3H), 7.26-7.31 (m, 2H)

### Production Example 13

The reaction was carried out in the same manner using 0.38 g of (E)-4-benzyloxy-2-butenoic acid in place of (E) -6-phenyl-2-hexenoic acid in Production Example 8, to obtain 0.25 g of (E)-N-(tetrahydropyran-4-ylmethyl)-4-benzyloxy-2-buteneamid e (hereinafter, described as Compound (13) of the present invention) represented by the following formula.

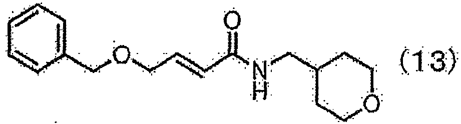

### Compound (13) of the present invention

1.21-1.33 (m, 2H), 1.58 (d, 2H), 1.69-1.79 (m, 1H), 3.17 (t, 2H), 3.32 (td, 2H), 3.93 (dd, 2H), 4.14 (dd, 2H), 4.53 (s, 2H), 6.14 (dd, 1H), 6.53 (br s, 1H), 6.86 (dt, 1H), 7.26-7.39 (m, 5H)

### Production Example 14

The reaction was carried out in the same manner using 0.35 g of (E)-3-(trans-2'-benzylcyclopropyl)-2-propenoic acid in place of (E)-6-phenyl-2-hexenoic acid in Production Example 1, to obtain 0.41 g of (E)-N-(tetrahydrofuran-3-ylmethyl)-3-(trans-2'-benzylcyclop ropyl)-2-propeneamide (hereinafter, described as Compound (14) of the present invention) represented by the following formula.

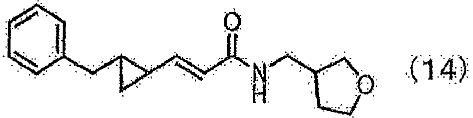

### Compound (14) of the present invention

¹H-NMR (CDCl₃, TMS) δ (ppm) : 0.86 (t, 2H), 1.24-1.32 (m, 1H), 1.38-1.45 (m, 1H), 1.60 (td, 1H), 1.97-2.05 (m, 1H), 2.43-2.52 (m, 1H), 2.65 (d, 2H), 3.23-3.36 (m, 2H), 3.53 (dd, 1H), 3.71 (dd, 1H), 3.79 (dd, 1H), 3.86 (td, 1H), 5.83 (d, 1H), 6.03 (br s, 1H), 6.39 (dd, 1H), 7.17-7.22 (m, 3H), 7.25-7.31 (m, 2H)

### Production Example 15

The reaction was carried out in the same manner using 0.35 g of (E)-3-(trans-2'-benzylcyclopropyl)-2-propenoic acid in place of (E)-6-phenyl-2-hexenoic acid in Production Example 8, to obtain 0.45 g of (E)-N-(tetrahydropyran-4-ylmethyl)-3-(trans-2'-benzylcyclop ropyl)-2-propeneamide (hereinafter, described as Compound (15) of the present invention) represented by the following formula.

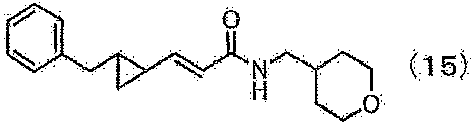

### Compound (15) of the present invention

¹H-NMR (CDCl₃, TMS) δ (ppm) : 0.87 (t, 2H), 1.24-1.36 (m, 3H), 1.38-1.46 (m, 1H), 1.60 (d, 2H), 1.70-1.83 (m, 1H), 2.65 (d, 2H), 3.19 (t, 2H), 3.34 (td, 2H), 3.95 (dd, 2H), 5.75 (br s, 1H), 5. 82 (d, 1H), 6.40 (dd, 1H), 7.17-7.24 (m, 3H), 7.24-7.35 (m, 2H)

### Production Example 16

The reaction was carried out in the same manner using 0.50 g of (E)-6-(2'-naphthyl)-2-hexenoic acid in place of (E) -6-phenyl-2-hexenoic acid in Production Example 8, to obtain 0.65 g of (E)-N-(tetrahydropyran-4-ylmethyl)-6-(2'-naphthyl)-2-hexene amide (hereinafter, described as Compound (16) of the present invention) represented by the following formula.

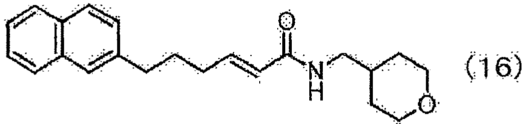

### Compound (16) of the present invention

¹H-NMR (CDCl₃, TMS) δ (ppm): 1.25-1.40 (m, 2H), 1.57-1.65 (m, 2H), 1.70-1.83 (m, 1H), 1.83-1.92 (m, 2H), 2.25 (dd, 2H), 2.81 (t, 2H), 3.21 (t, 2H), 3.36 (td, 2H), 3.97 (dd, 2H), 5.49 (br s, 1H), 5.76 (dt, 1H), 6.88 (dt, 1H), 7.30-7.34 (m, 1H), 7.40-7.48 (m, 2H), 7.60 (s, 1H), 7.75-7.82 (m, 3H)

### Production Example 17

The reaction was carried out in the same manner using 0.73 g of (E)-6-(2'-pyridyl)-2-hexenoic acid in place of (E)-6-phenyl-2-hexenoic acid in Production Example 8, to obtain 0.06 g of (E)-N-(tetrahydropyran-4-ylmethyl)-6-(2'-pyridyl)-2-hexenea mide (hereinafter, described as Compound (17) of the present invention) represented by the following formula.

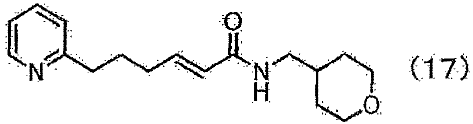

### Compound (17) of the present invention

¹H-NMR (CDCl₃, TMS) δ (ppm): 1.26-1.38 (m, 2H), 1.58-1.65 (m, 2H), 1.73-1.85 (m, 1H), 1.87-1.94 (m, 2H), 2.25 (dd, 2H), 2.81 (t, 2H), 3.22 (t, 2H), 3.36 (td, 2H), 3.97 (dd, 2H), 5.60 (br s, 1H), 5.79 (dt, 1H), 6.86 (dt, 1H), 7.10-7.15 (m, 2H), 7.60 (td, 1H), 8.52 (d, 1H)

### Production Example 18

(E)-3-(Trans-2'-benzylcyclopropyl)-2-propenoic acid (0.10g, 0.50mmol) and tetrahydrofuran-2-ylmethylamine (76 mg, 0.75 mmol) were added to tetrahydrofuran (1 mL) . To the mixture solution was added 1-ethyl-3-(3-dimethylaminopropyl)carbodiimide hydrochloride (0.12 g, 0.60 mmol) at room temperature, then the mixture was stirred at room temperature for 4 hours. Thereafter, water was added to the reaction mixture, and the mixture was extracted twice with ethyl acetate. The organic layer was washed with a saturated salt solution, and dried over anhydrous sodium sulfate, then filtered, and the filtrate was concentrated under reduced pressure. The residue was applied to a silica gel column chromatography, to obtain 0.07 g of (E)-N-(tetrahydropyran-2-ylmethyl)-3-(trans-2'-benzylcyclop ropyl)-2-propeneamide (hereinafter, described as Compound (18) of the present invention) represented by the following formula.

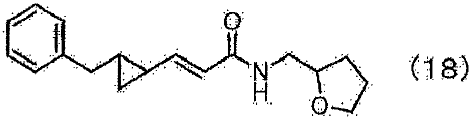

### Compound (18) of the present invention

¹H-NMR (CDCl₃, TMS) δ (ppm) : 0.83-0.89 (m, 2H), 1.25-1.32 (m, 1H), 1.39-1.46 (m, 1H), 1.54 (dt, 1H), 1.85-2.01 (m, 3H), 2.66 (d, 2H), 3.16-3.22 (m, 1H), 3.63 (ddd, 1H), 3.71-3.77 (m, 1H), 3.85 (q, 1H), 3.94-4.00 (m, 1H), 5.82 (d, 1H), 5.88 (br s, 1H), 6.40 (dd, 1H), 7.17-7.24 (m, 3H), 7.24-7.31 (m, 2H)

Next, reference production examples of intermediates used in the production of the compound of the present invention are shown.

### Reference Production Example 1

Under a nitrogen atmosphere, to a mixture of 25 g of (tetrahydrofuran-3-yl)-methanol represented by the following formula 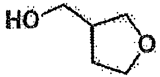 and 125 ml of pyridine was added 56 g of p-toluenesulfonyl chloride under ice cooling, and the mixture was stirred under ice cooling for 4 hours. Thereafter, water was added to the reaction mixture, and the mixture was extracted twice with tert-butyl methyl ether. The organic layer was washed with 1 mol/L hydrochloric acid followed by a saturated salt solution. The organic layer was dried over anhydrous sodium sulfate, and then filtered. The filtrate was concentrated under reduced pressure to obtain 58 g of a crude product of tetrahydrofuran-3-ylmethyl p-toluenesulfonate represented by the following formula.

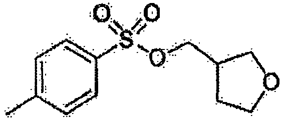

The product was used in Reference Production Example 2 without purification.

### Reference Production Example 2

Under a nitrogen atmosphere, to a mixture of 29 g of tetrahydrofuran-3-ylmethyl p-toluenesulfonate, 23 g of potassium iodide and 300 ml of N,N-dimethylformamide was added 22 g of phthalimide potassium salt, and the mixture was stirred at 80°C for 3 hours. Thereafter, the reaction mixture was ice-cooled, water was added to the mixture, and the resulting mixture was stirred for 30 minutes. The reaction mixture was filtered to collect a solid. The solid was dried under reduced pressure to obtain 14 g of 2-(tetrahydrofuran-3-ylmethyl)-isoindole-1,3-dione represented by the following formula.

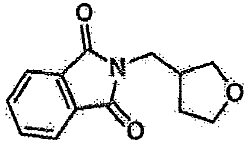

¹H-NMR (CDCl₃, TMS) δ (ppm): 7.86 (2H, dd), 7.73 (2H, dd), 3.94 (1H, td), 3.85-3.67 (4H, m), 3.61 (1H, dd), 2.79-2.68 (1H, m), 2.06-1.97 (1H, m), 1.77-1.68 (1H, m)

### Reference Production Example 3

Under a nitrogen atmosphere, to a mixture of 14 g of 2-(tetrahydrofuran-3-ylmethyl)-isoindole-1,3-dione and 150 ml of ethanol was added 4.6 g of hydrazine monohydrate, and the mixture was stirred at 70°C for 6 hours. Thereafter, the reaction mixture was filtered, and the residue was washed with ethanol. To the ethanol solution was added 1 ml of a suspension of Raney nickel, and the mixture was stirred at 60°C for 4 hours. The reaction solution was cooled to room temperature, and then filtered. To the filtrate was added 90 ml of a 11 mol/L solution of hydrogen chloride in diethyl ether. The mixture solution was concentrated under reduced pressure to obtain 10 g of a crude product of (tetrahydrofuran-3-yl)-methylamine hydrochloride represented by the following formula.

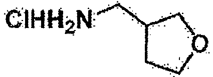

### The product was used in the next reaction without purification.

Next, formulation examples are shown. The term "part(s)" means part(s) by weight.

### Formulation Example 1

20 parts of any one of Compounds (1) to (138) of the present invention is dissolved in 65 parts of xylene, and 15 parts of SORPOL 3005X (registered trade name of TOHO Chemical Industry Co., Ltd.) is added thereto. The mixture is well stirred and mixed to obtain an emulsifiable concentrate.

### Formulation Example 2

40 parts of anyone of Compounds (1) to (138) of the present invention and 5 parts of SORPOL 3005X are well mixed, and then 32 parts of Carplex #80 (synthetic hydrous silicon oxide, registered trade name of Shionogi & Co., Ltd.), and 23 parts of 300-mesh diatomaceous earth are added thereto. The mixture is stirred and mixed with a juice mixer to obtain a wettable powder.

### Formulation Example 3

1.5 parts of any one of Compounds (1) to (138) of the present invention, 1 part of Tokuseal GUN (synthetic hydrous silicon oxide, manufactured by Tokuyama corporation), 2 parts of Reax 85A (sodium ligninsulfonate, manufactured by West vaco chemicals), 30 parts of Bentonite Fuji (bentonite, manufactured by HOJUN Co. , Ltd.), and 65. 5 parts of Shokozan A clay (kaolin clay, manufactured by SHOKOZAN MINING Co., Ltd.) are well pulverized and mixed, and water is added thereto. The mixture is then kneaded well, granulated with an extrusion granulator, and dried to obtain 1.5% granules.

### Formulation Example 4

10 parts of any one of Compounds (1) to (138) of the present invention, 10 parts of phenylxylylethane, and 0.5 part of Sumijule L-75 (tolylene diisocyanate, manufactured by Sumitomo Bayer Urethane Co. Ltd.) are mixed, and then the mixture is added to 20 parts of a 10% aqueous solution of arabic gum, followed by stirring the resulting mixture with a homomixer to obtain an emulsion having an average particle diameter of 20 µm. Thereto is added 2 parts of ethylene glycol, and the mixture is further stirred in a warm bath at 60°C for 24 hours to obtain a microcapsule slurry. Separately, 0.2 parts of xanthan gum and 1.0 part of Veegum R (aluminum magnesium silicate, manufactured by Sanyo Chemical Industries, Ltd.) are dispersed in 56.3 parts of ion-exchanged water to obtain a thickening agent solution. 42.5 parts of the microcapsule slurry and 57.5 parts of the thickening agent solution are mixed to obtain a microcapsule formulation.

### Formulation Example 5

10 parts of any one of Compounds (1) to (138) of the present invention and 10 parts of phenylxylylethane are mixed, and then the mixture is added to 20 parts of 10% aqueous solution of polyethylene glycol, followed by stirring the resulting mixture with a homomixer to obtain an emulsion having an average particle diameter of 3 µm. Separately, 0.2 parts of xanthan gum and 1.0 part of Veegum R (aluminum magnesium silicate, manufactured by Sanyo Chemical Industries, Ltd.) are dispersed in 58.8 parts of ion-exchanged water to obtain a thickening agent solution. 40 parts of the emulsion solution and 60 parts of the thickening agent solution are mixed to obtain a flowable. Formulation Example 6

5 parts of any one of Compounds (1) to (138) of the present invention, 3 parts of Carplex #80 (a fine powder of synthetic hydrous silicon oxide, registered trade name of Shionogi & Co. , Ltd.), 0.3 parts of PAP (a mixture of monoisopropyl phosphate and diisopropyl phosphate) and 91.7 parts of talc (300 mesh) are stirred and mixed with a juice mixer to obtain a dust formulation.

### Formulation Example 7

0.1 part of any one of Compounds (1) to (138) of the present invention is dissolved in 10 parts of isopropyl alcohol, and the mixture is mixed with 89.9 parts of deodorized kerosene to obtain an oil solution.

### Formulation Example 8

1 part of anyone of Compounds (1) to (138) of the present invention, 5 parts of dichloromethane and 34 parts of deodorized kerosene are mixed and dissolved. The resulting solution is filled into an aerosol container, and a valve part is attached to the container, then 60 parts of a propellant (liquefied petroleum gas) is filled under pressure into the container through the valve part to obtain an oil-based aerosol. Formulation Example 9

0.6 parts of any one of Compounds (1) to (138) of the present invention, 5 parts of xylene, 3.4 parts of deodorized kerosene and 1 part of ATMOS 300 (emulsifier, registered trade name of Atlas Chemical Company) are mixed and dissolved, and the resulting solution and 50 parts of water are filled into an aerosol container, and 40 parts of a propellant (liquefied petroleum gas) is filled under pressure into the container through the valve part to obtain an aqueous aerosol. Formulation Example 10

0.3 g of any one of Compounds (1) to (138) of the present invention is dissolved in 20 ml of acetone. The resulting solution is uniformly stirred and mixed with 99.7 g of a base material for an insecticidal coil (a mixture of Tabu powder, Pyrethrum marc and wood powder in a ratio of 4 : 3 : 3). Thereafter, 100 ml of water is added to the mixture, and the resulting mixture is sufficiently kneaded, and molded and dried to obtain an insecticidal coil.

### Formulation Example 11

0.8 g of any one of Compounds (1) to (138) of the present invention and 0.4 g of piperonyl butoxide are dissolved in acetone to have a total amount of 10 ml. A base material for an insecticidal mat for electric heating with a size of 2.5 cm × 1.5 cm, 0.3 cm in thickness (a plate of compacted fibrils of a mixture of cotton linter and pulp) is uniformly impregnated with 0.5 ml of this solution to obtain an insecticidal mat for electric heating.

### Formulation Example 12

3 parts of any one of Compounds (1) to (138) of the present invention is dissolved in 97 parts of deodorized kerosene to obtain a solution, and this solution is put in a container made of polyvinyl chloride. Into the container is inserted an

absorptive wick (which is prepared by solidifying powders of an inorganic powder with a binder followed by sintering them) whose upper portion can be heated by a heater, to obtain a part to be used for an absorptive wick type electric heating vaporizer.

### Formulation Example 13

100 mg of anyone of Compounds (1) to (138) of the present invention is dissolved in an appropriate amount of acetone, and a porous ceramic plate with a size of 4.0 cm × 4.0 cm, 1.2 cm in thickness is impregnated with the solution to obtain a fumigant for heating.

### Formulation Example 14

100 µg of any one of Compounds (1) to (138) of the present invention is dissolved in an appropriate amount of acetone, and the solution is uniformly applied on a filter paper of 2 cm × 2 cm, 0.3 mm in thickness. Then, the filter paper is air-dried to remove acetone to obtain a formulation vaporizable at room temperature.

### Formulation Example 15

10 parts of any one of Compounds (1) to (138) of the present invention, 35 parts of white carbon containing 50 parts of polyoxyethylene alkyl ether sulfate ammonium salt and 55 parts of water are mixed, and the mixture is finely pulverized by wet grinding method to obtain a formulation.

Next, the pest control effect of the compound of the present invention is shown as test examples.

### Test Example 1

The formulations of Compounds (1) to (4), (6) to (8), (10), (12) to (16) and (18) (13) of the present invention obtained in Formulation Example 7 were diluted with a mixture solution of isopropyl alcohol/deodorized kerosene of 1/9, so as to have a concentration of active ingredient of 0.5% w/v, to prepare a test drug solution.

Ten German cockroaches (Blattella germanica) (5 males and 5 females) were released in a test container (8.75 cm in diameter, 7.5 cm in height, and the bottom made of 16 mesh metallic wire) with the inner wall on which butter was applied, and the container was placed on the bottom of a test chamber (bottom: 46 cm × 46 cm, height: 70 cm). From a height of 60 cm above the upper surface of the container, 1.5 ml of the test drug solution was sprayed using a spray gun (a spraying pressure of 0.42 kg/cm²). Thirty seconds after spraying, the container was taken out from the test chamber, and after 5 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (1) to (4), (6) to (8), (10), (12) to (16) and (18) (13) of the present invention, the knockdown rate of the tested insect was 80% or more.

### Test Example 2

The formulations of Compounds (4), (6), (8), (10), (14) to (16) and (18) (10) of the present invention obtained in Formulation Example 7 were diluted with a mixture solution with a ratio of isopropyl alcohol/deodorized kerosene of 1/9, so as to have a concentration of active ingredient of 0.5% w/v, to prepare a test drug solution.

Ten adult Musca domestica (5 males and 5 females) were released in a polyethylene cup (a bottom diameter of 10.6 cm) which was then capped with 16-mesh nylon gauze. The polyethylene cup was placed on the bottom of a test chamber (bottom: 46 cm × 46 cm, height: 70 cm). From a height of 30 cm above the upper surface of the polyethylene cup, 0.5 ml of the test drug solution was sprayed using a spray gun (a spraying pressure of 0.9 kg/cm²). Immediately after spraying, the cup was taken out from the test container, and after 10 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (4), (6), (8), (10), (14) to (16) and (18) (10) of the present invention, the knockdown rate of the tested insect was 80% or more.

### Test Example 3

The formulations of Compounds (4), (6), (8) to (11) and (14) to (16) (8) to (11) of the present invention obtained in Formulation Example 7 were diluted with a mixture solution with a ratio of isopropyl alcohol/deodorized kerosene of 1/9, so as to have a concentration of active ingredient of 0.02% w/v, to prepare a test drug solution.

Ten adult common house mosquitos were released in a polyethylene cup (a bottom diameter of 10.6 cm) which was then capped with 16-mesh nylon gauze. The polyethylene cup was placed on the bottom of a test chamber (bottom: 46 cm × 46 cm, height: 70 cm) . From a height of 30 cm above the upper surface of the polyethylene cup, 0.5 ml of the test drug solution was sprayed using a spray gun (a spraying pressure of 0.4 kg/cm²). Immediately after spraying, the cup was taken out from the test container, and after 10 minutes, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (4), (6), (8) to (11) and (14) to (16) (8) to (11) of the present invention, the knockdown rate of the tested insect was 80% or more.

### Test Example 4

Each of the formulations of Compounds (4) to (6) of the present invention obtained in Formulation Example 15 was diluted with water, so as to have a concentration of active ingredient of 500 ppm, to prepare a test drug solution.

On the bottom of a polyethylene cup with a diameter of 5.5 cm, a filter paper of the same size was laid down, and 0.7 ml of the test drug solution was dropped onto the filter paper, then 30 mg of sucrose was uniformly put as bait. Ten adult houses (Musca domestica) were released in the polyethylene cup which was then capped. After 2 hours, the number of knockdown insects was counted to obtain a knockdown rate. The knockdown rate was calculated by the following equation.

Knockdown Rate (%) = (Number of Knockdown Insects/Number of Tested Insects) × 100

As a result, in treatments with Compounds (4) to (6) of the present invention, the knockdown rate of the tested insect was 100%.

### Test Example 5

Each of the formulations of Compounds (4), (5), (11) and (12) of the present invention obtained in Formulation Example 15 was diluted with water, so as to have a concentration of active ingredient of 500 ppm, to prepare a test drug solution.

0.7 ml of the test drug solution was added to 100 ml of ion-exchanged water (a concentration of active ingredient of 3.5 ppm). 20 last instar larvae of common house mosquitos (Culex pipiens pallens) were released in the solution, and after 8 days, the insects were observed for life or death to obtain the mortality.

As a result, in treatments with Compounds (4), (5), (11) and (12) of the present invention, the mortality showed 95% or more.

### INDUSTRIAL APPLICABILITY

The compound of the present invention has a control effect on pests, thus is useful as an active ingredient of a pest control agent.

## Claims

1. An amide compound represented by formula (I) 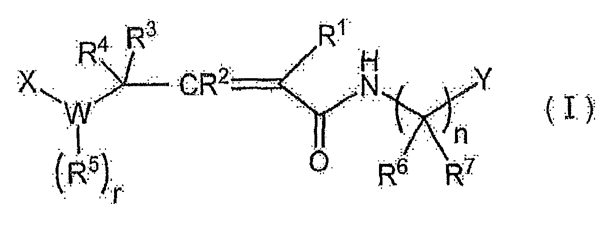 wherein
Y represents a 3 to 7-membered saturated heterocyclic ring which contains, as ring-forming component(s), one or more atoms or groups that are selected from the group consisting of an oxygen atom and -S(O)ₜ-, and the saturated heterocyclic ring may have one to three atoms or groups selected from group D,
t represents 0, 1 or 2,
X represents a C1 to C8 chain hydrocarbon group having one group that is selected from group A,
W represents -CR⁸-, an oxygen atom or -S(O)ᵤ-,
when W is -CR⁸-, r represents 1,
when W is an oxygen atom or -S(O)ᵤ-, r represents 0,
u represents 0, 1 or 2,
R⁸ represents a C1 to C4 alkyl group optionally having one or more halogen atoms, a halogen atom or a hydrogen atom,
R¹ and R² are the same or different and represent a C1 to C4 alkyl group optionally having one or more halogen atoms, a C1 to C4 alkoxy group optionally having one or more halogen atoms, a halogen atom or a hydrogen atom,
R⁴ represents a C1 to C4 alkyl group optionally having one or more halogen atoms, a halogen atom or a hydrogen atom,
R³ and R⁵ are the same or different and represent a C1 to C4 alkyl group optionally having one or more halogen atoms, a halogen atom or a hydrogen atom, or R³ and R⁵ are taken together to represent -(CR⁹R¹⁰)ᵥ-, -NR¹¹-, an oxygen atom or a sulfur atom,
R⁹ and R¹⁰ are the same or different and represent a C1 to C4 alkyl group optionally having one or more halogen atoms, a halogen atom or a hydrogen atom,
R¹¹ represents a C1 to C4 alkyl group optionally having one or more halogen atoms or a hydrogen atom,
v represents 1, 2, 3 or 4,
R⁶ and R⁷ are the same or different and represent a C1 to C4 alkyl group optionally having one or more halogen atoms or a hydrogen atom,
n represents 0, 1 or 2,
Group D: A group consisting of C1 to C4 alkyl groups optionally having one or more halogen atoms, C1 to C4 alkoxy groups optionally having one or more halogen atoms and halogen atoms. Group A: A group consisting of a phenyl group, a naphthyl group, a pyridyl group, a quinolyl group, a furyl group, a thienyl group, a benzofuranyl group and a benzothienyl group. Here, all the above-described groups in the group A may have one or more atoms or groups selected from group B.
Group B: A group consisting of C1 to C4 alkyl groups optionally having one or more halogen atoms, C1 to C4 alkoxy groups optionally having one or more halogen atoms, C1 to C4 alkylthio groups optionally having one or more halogen atoms, C1 to C4 alkylsulfinyl groups optionally having one or more halogen atoms, C1 to C4 alkylsulfonyl groups optionally having one or more halogen atoms, C1 to C4 alkoxycarbonyl groups optionally having one or more halogen atoms, vinyl groups optionally having one or more atoms or groups selected from group E, ethynyl groups optionally having an atom or group selected from group E, a cyano group, a nitro group, a carboxyl group, a hydroxyl group, -CONR¹²R¹³ group and halogen atoms.
R¹² and R¹³ are the same or different and represent a C1 to C4 alkyl group optionally having one or more halogen atoms or a hydrogen atom,
Group E: A group consisting of C1 to C4 alkyl groups optionally having one or more halogen atoms and halogen atoms.

2. The amide compound according to claim 1, wherein Y
is represented by the following formula (II-a) or (II-b) 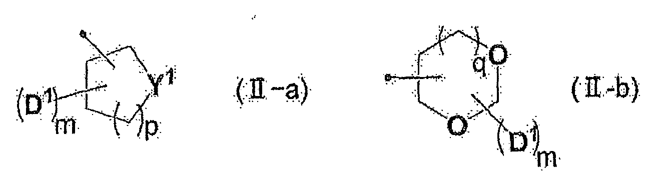 wherein Y¹ represents an oxygen atom or a sulfur atom, D¹ represents an atom or a group selected from group D, m represents 0 or 1, p represents 0, 1 or 2, and q is 0 or 1.

3. The amide compound according to claim 2, wherein p is 1, or q is 0.

4. The amide compound according to claim 2, wherein p is 2, or q is 1.

5. The amide compound according to any of claims 2 to 4, wherein Y¹ is an oxygen atom.

6. The amide compound according to claim 2, wherein Y is a group represented by the formula (II-a), and Y¹ is an oxygen atom.

7. The amide compound according to claim 2, wherein Y is a group represented by the formula (II-b).

8. The amide compound according to any of claims 1 to 7, wherein
W is -CR⁸-, r is 1, R¹ is a halogen atom or a hydrogen atom, R² is a C1 to C4 alkyl group optionally having one or more halogen atoms or a hydrogen atom,
R⁴ is a C1 to C4 alkyl group optionally having one or more halogen atoms, a halogen atom or a hydrogen atom,
R³ and R⁵ are the same or different and represent a C1 to C4 alkyl group optionally having one or more halogen atoms, a halogen atom or a hydrogen atom, or R³ and R⁵ are taken together to be -(CR⁹R¹⁰)ᵥ-, v is 1, R⁹ and R¹⁰ are the same or different and are a C1 to C4 alkyl group optionally having one or more halogen atoms, a halogen atom or a hydrogen atom,
R⁸ is a hydrogen atom, and
R⁶ and R⁷ are the same or different and are a C1 to C4 alkyl group optionally having one or more halogen atoms or a hydrogen atom.

9. The amide compound according to any of claims 1 to 8, wherein X is a C1 to C5 alkyl group having one group selected from group A.

10. The amide compound according to any of claims 1 to 8, wherein X is a C1 to C5 alkyl group having one group selected from group A, one group selected from group A is a phenyl group, a 2-naphthyl group, a 2-pyridyl group, a 3-pyridyl group, a 4-pyridyl group, a 2-quinolyl group, a 3-quinolyl group, a 2-thienyl group or a 3-thienyl group, and all the above-described groups in the group A may have one or more atoms or groups selected from group B.

11. A pest control agent containing the amide compound as defined in any of claims 1 to 10 and an inert carrier.

12. A method for controlling pests including the step of applying an effective amount of the amide compound as defined in any of claims 1 to 10 to a pest or a place where a pest inhabits.
